# FASCICULE DE BREVET EUROPEEN

(11) **EP 2 257 640 B1**
(45) Date de publication et mention de la délivrance du brevet: **20.05.2015**
(21) Numéro de dépôt: 09728943.3
(22) Date de dépôt: 10.03.2009
(51) Int. Cl.: C12Q 1/68, C12Q 1/70

(54) **PROCEDE POUR LE DIAGNOSTIC OU LE PRONOSTIC IN VITRO DU CANCER DU TESTICULE**
VERFAHREN ZUR IN-VITRO-DIAGNOSE ODER PROGNOSE VON HODENKREBS
METHOD FOR IN VITRO DIAGNOSIS OR PROGNOSIS OF TESTICULAR CANCER

(30) Priorité: 12.03.2008 FR 0851619
(43) Date de publication de la demande: 08.12.2010
(73) Titulaire: Biomérieux, 69280 Marcy L'etoile (FR)
(72) Inventeur: ARSAC, Maud, F-42400 Saint-Chamond (FR); BONNAUD, Bertrand, F-42410 Verin (FR); MALLET, François, F-69100 Villeurbanne (FR); PICHON, Jean-Philippe, F-69300 Clermont-ferrand (FR)
(86) Numéro de dépôt international: PCT/FR2009/050390
(87) Numéro de publication internationale: WO 2009/122053

(56) Documents cités:
- US-A- 5 858 723
- YI J-M ET AL: "Expression of the human endogenous retrovirus HERV-W family in various human tissues and cancer cells" JOURNAL OF GENERAL VIROLOGY, SOCIETY FOR GENERAL MICROBIOLOGY, SPENCERS WOOD, GB, vol. 85, no. Pt 5, 1 mai 2004 (2004-05-01), pages 1203-1210, XP002417878 ISSN: 0022-1317
- GOEDERT J J ET AL: "High prevalence of antibodies against HERV-K10 in patients with testicular cancer but not with AIDS" CANCER EPIDEMIOLOGY, BIOMARKERS AND PREVENTION, PHILADELPHIA, PA, vol. 8, no. 4, 1 avril 1999 (1999-04-01), pages 293-296, XP002983119 ISSN: 1055-9965
- YI JOO-MI ET AL: "Expression and identification of HERV-W family in Japanese monkeys (Macaca fuscata)." ZOOLOGICAL SCIENCE JUN 2004, vol. 21, no. 6, juin 2004 (2004-06), pages 649-659, XP002500823 ISSN: 0289-0003

## Description

Le cancer du testicule représente 1 à 2% des cancers chez l'homme, et 3,5% des tumeurs urologiques. C'est la tumeur la plus fréquente chez l'homme jeune, rare avant 15 ans et après 50 ans. Le risque est plus élevé chez les patients séropositifs pour le virus HIV. Le séminome est la forme la plus fréquente du cancer du testicule (40%), mais de nombreux autres types de cancer existent parmi lesquels le carcinome embryonnaire (20%), le tératocarcinome (30%) et le choriocarcinome (1%).

Le diagnostic du cancer du testicule est d'abord clinique : il se présente souvent sous la forme d'une tuméfaction dure et irrégulière du testicule. L'échographie confirme la tumeur intra-testiculaire et l'échographie Doppler met en évidence l'augmentation de la vascularisation dans la tumeur. Dans certains cas, un examen par résonance magnétique (IRM testiculaire) peut être utile. Un scanner thoracique, abdominal et pelvien permet de rechercher une extension ganglionnaire du cancer. Un prélèvement sanguin pour le dosage des marqueurs tumoraux est quasi-systématique. Il permet d'orienter le diagnostic du type de tumeur. Deux marqueurs tumoraux principaux sont utilisés et dosés dans le sang : la β-HCG et l'α-foetoprotéine. Mais ces marqueurs ne sont pas très spécifiques et de plus leur concentration à des niveaux physiologiques ne signifie pas une absence de tumeur. A ce jour, le diagnostic et le pronostic finaux sont posés après l'ablation du testicule atteint (orchidectomie) qui constitue le premier stade du traitement. Ensuite, en fonction du type de cancer et de son stade, un traitement complémentaire par radiothérapie ou chimiothérapie est appliqué. Il existe donc un réel besoin de pouvoir disposer de marqueurs spécifiques du cancer du testicule qui, de plus, permettent d'établir un diagnostic et un pronostic les plus précoces possible.

L'événement rare que représente l'infection d'une cellule de la lignée germinale par un provirus exogène conduit à l'intégration dans le génome de l'hôte d'un ADN proviral ou provirus, celui-ci devenant partie intégrante du patrimoine génétique de l'hôte. Ce provirus endogène (HERV) est donc transmissible à la génération suivante de façon mendélienne. On estime qu'il existe environ une centaine de familles HERV représentant environ 8% du génome humain. Chacune des familles compte de quelques dizaines à des milliers de loci, qui sont le résultat de rétrotranspositions intracellulaires de copies transcriptionnellement actives. Les loci des familles HERV contemporaines sont tous défectifs pour la réplication, ce qui signifie la perte des propriétés infectieuses et implique donc un mode de transmission exclusivement vertical (mendélien).

L'expression des HERV a été particulièrement étudiée dans trois contextes spécifiques, la placentation, l'auto-immunité et le cancer, qui sont associés à la différentiation cellulaire ou à la modulation de l'immunité. Il a ainsi été montré que la glycoprotéine d'enveloppe du locus ERVWE1 de la famille HERV-W est impliquée dans le processus de fusion aboutissant à la formation du syncytiotrophoblaste. Il a par ailleurs été suggéré que la protéine Rec, variant d'épissage du gène *env* de HERV-K, pourrait avoir une implication dans le processus de tumorigenèse testiculaire. Cependant, il n'a pas encore été répondu à la question suivante : les HERV sont-ils des acteurs ou des marqueurs dans des contextes pathologiques ?

Les présents inventeurs ont maintenant découvert et démontré que des séquences d'acides nucléiques, appartenant à des loci de la famille HERV-W, sont associées au cancer du testicule et que ces séquences sont des marqueurs moléculaires de la pathologie. Les séquences identifiées sont soit des provirus, c'est à dire des séquences contenant tout ou partie des gènes *gag, pol* et *env* flanqués en 5' et en 3' de longues terminaisons répétées (LTR ou « Long Terminal Repeat » selon la terminologie anglo-saxonne), soit des LTR isolées. Les séquences ADN identifiées sont respectivement référencées en SEQ ID Nos 1 à 6 dans le listage de séquences.

La présente invention a donc pour objet un procédé pour le diagnostic ou le pronostic, *in vitro,* du cancer du testicule dans un échantillon de testicule, issu d'un patient suspecté d'être atteint d'un cancer du testicule qui comprend une étape de détection de la présence ou de l'absence d'au moins un produit d'expression d'au moins une séquence d'acide nucléique choisie parmi les séquences identifiées en SEQ ID NOs : 1 à 6 ou parmi les séquences qui présentent au moins 99% d'identité avec une des séquences identifiées en SEQ ID NOs : 1 à 6, une étape de comparaison de la quantité du produit d'expression de l'échantillon à analyser par rapport à la quantité du produit d'expression d'un échantillon sain ; dans lequel une surexpression dans l'échantillon analysé par rapport à l'expression dans l'échantillon sain est corrélée au cancer du testicule.
Le pourcentage d'identité décrit ci-dessus a été déterminé en prenant en considération la diversité nucléotidique dans le génome. Il est connu que la diversité nucléotidique est plus élevée dans les régions du génome riches en séquences répétées que dans les régions ne contenant pas séquences répétées. A titre d'exemple, Nickerson D. A*. et al.,* ^{[1]} ont montré une diversité d'environ 0,3% (0,32%) dans des régions contenant des séquences répétées.

Le produit d'expression qui est détecté est de préférence au moins un transcrit ARNm d'au moins une des séquences SEQ ID NOs : 1 à 6, mais ce peut être également un polypeptide qui est le produit de la traduction d'au moins un desdits transcrits.

Lorsque le produit d'expression est un transcrit ARNm, il est détecté par toute méthode appropriée, telle que l'hybridation, le séquençage ou l'amplification. L'ARNm peut être détecté directement par mise en contact avec au moins une sonde et/ou au moins une amorce qui sont conçues pour s'hybrider dans des conditions de stringence prédéterminées aux transcrits ARNm, la mise en évidence de la présence ou de l'absence d'hybridation à l'ARNm et éventuellement la quantification de l'ARNm. Parmi les méthodes préférées ont peut citer l'amplification (par exemple la RT-PCR, la NASBA, etc...) ou encore le Northern blot. L'ARNm peut également être détecté indirectement à partir d'acides nucléiques dérivés desdits transcrits, comme les copies ADNc, etc...

Généralement le procédé de l'invention comprend une étape initiale d'extraction de l'ARNm de l'échantillon à analyser.

Ainsi, le procédé peut comprendre :
(i) une étape d'extraction de l'ARNm dans l'échantillon à analyser,
(ii) une étape de détection et de quantification de l'ARNm de l'échantillon à analyser,
(iii) une étape d'extraction de l'ARNm dans un échantillon sain,
(iv) une étape de détection et de quantification de l'ARNm de l'échantillon sain,
(iii) une étape de comparaison de la quantité d'ARNm exprimé dans l'échantillon à analyser et dans l'échantillon sain ; la détermination d'une quantité d'ARNm exprimé dans l'échantillon à analyser supérieure à la quantité d'ARNm exprimé dans l'échantillon sain pouvant être corrélée avec le diagnostic ou le pronostic d'un cancer du testicule ;
et en particulier :
(i) une extraction de l'ARN à analyser dans l'échantillon,
(ii) une détermination, dans l'ARN à analyser, d'un niveau d'expression d'au moins une séquence ARN dans l'échantillon, ladite séquence ARN étant le produit de transcription d'au moins une séquence d'acide nucléique choisie parmi les séquences identifiées en SEQ ID NOs : 1 à 6 ou parmi les séquences qui présentent au moins 99% d'identité, de préférence au moins 99,5% d'identité et avantageusement au moins 99,6% d'identité avec une des séquences identifiées en SEQ ID NOs : 1 à 6, et
(iii) une comparaison du niveau d'expression de la ou desdites séquence(s) ARN définie(s) en
(ii) avec le niveau d'expression de la ou desdites séquences ARN dans un échantillon biologique non cancéreux ; la détermination d'un niveau d'expression de l'ARN à analyser supérieur au niveau d'expression de l'ARN extrait de l'échantillon biologique non cancéreux pouvant être corrélé avec le diagnostic ou le pronostic d'un cancer du testicule.

Les transcrits sont sur-exprimés dans les tumeurs du testicule. Pour détecter une telle sur-expression, un point de référence peut être nécessaire, c'est à dire un contrôle. La quantité d'ARNm dans l'échantillon sain sert de standard de référence auquel peut être comparée la quantité d'ARNm dans l'échantillon à analyser, une sur-expression d'ARNm dans l'échantillon à analyser par rapport à l'expression d'ARNm dans l'échantillon sain pouvant être corrélée avec un diagnostic ou un pronostic d'un cancer du testicule. Cependant, comme la transcription est généralement négligeable voire inexistante dans l'échantillon sain, alors qu'elle est significativement plus importante dans l'échantillon cancéreux, un point de référence n'est pas indispensable, l'expression significative de transcrits étant un indicateur de la maladie.

Par séquence sur-exprimée on entend une séquence d'ARNm qui est trouvée en des quantités plus importantes ou à des niveaux supérieurs à ceux trouvés pour une même séquence d'ARNm issue du même type d'échantillon non cancéreux constituant la valeur seuil de référence.

Les séquences desdits transcrits sont respectivement identifiées en SEQ ID NOs 7 à 12 (données en référence avec l'ADN génomique) :
SEQ ID NO : 7 = transcrit du locus HW4TT,
SEQ ID NO : 8 = transcrit du locus HW2TT,
SEQ ID NO : 9 = transcrit du locus HW13TT,
SEQ ID NO : 10 = transcrit du locus HWXTT,
SEQ ID NO : 11 = transcrit du locus HW21TT,
SEQ ID NO : 12 = transcrit du locus ERVWE1.

Lorsque le produit d'expression est un polypeptide issu de la traduction d'au moins un des transcrits, il peut être détecté dans le procédé de l'invention en utilisant au moins un partenaire de liaison spécifique dudit polypeptide, en particulier un anticorps, par exemple un anticorps monoclonal. La méthode de production d'anticorps monoclonaux et le procédé de sélection sont bien connus de l'homme du métier.

A titre d'illustration, des séquences de polypeptides sont décrites et identifiées en SEQ ID NOs 14, 16, 18, 20, 22 et 24 :
SEQ ID NO : 14 = protéine Gag de HW4TT,
SEQ ID NO : 16 = protéase de HW4TT,
SEQ ID NO : 18 = protéine Gag de HW2TT,
SEQ ID NO : 20 = protéine de HW2TT,
SEQ ID NO : 22 = protéine Gag de HW13TT,
SEQ ID NO : 24 = protéine Gag de HW21TT
SEQ ID NO : 26 = protéine Env de ERVWE1 (Syncytine-1).

L'échantillon du patient comprendra généralement des cellules (telles que les cellules testiculaires). Elles peuvent être présentes dans un échantillon de tissu (tel que le tissu testiculaire) ou être trouvées dans la circulation. En général, l'échantillon est un extrait tissulaire de testicule ou un fluide biologique, tel que le sang, le sérum, le plasma, l'urine, ou encore le liquide séminal.

L'invention a aussi pour objet, une séquence d'acide nucléique, isolée, qui consiste en :
(i) au moins une séquence ADN choisie parmi les séquences SEQ ID NOs : 1 à 6, ou
(ii) au moins une séquence ADN complémentaire d'une séquence choisie parmi les séquences SEQ ID NOs : 1 à 6, ou
(iii) au moins une séquence ADN qui présente au moins 99% d'identité, de préférence au moins 99,5% d'identité et avantageusement au moins 99,6% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'une séquence choisie parmi les séquences qui présentent au moins 99% d'identité, de préférence au moins 99,5% d'identité et avantageusement au moins 99,6% d'identité avec une séquence telle que définie en (i), ou
(vi) au moins une séquence ARN choisie parmi les séquences SEQ ID NOs : 7 à 12 ; et
l'utilisation d'au moins une séquence d'acide nucléique, isolée, comme marqueur moléculaire pour le diagnostic ou le pronostic *in vitro* du cancer du testicule, dans laquelle la séquence d'acide nucléique consiste en :
(i) au moins une séquence ADN choisie parmi les séquences SEQ ID NOs : 1 à 6, ou
(ii) au moins une séquence ADN complémentaire d'une séquence choisie parmi les séquences SEQ ID NOs : 1 à 6, ou
(iii) au moins une séquence ADN qui présente au moins 99% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'une séquence choisie parmi les séquences qui présentent au moins 99% d'identité avec une séquence telle que définie en (i), ou
(vi) au moins une séquence ARN choisie parmi les séquences SEQ ID NOs : 7 à 12.

### Figures :

La figure 1 représente le principe de la méthode d'amplification WTA des ARNs.
La figure 2 représente un schéma synoptique de la nature et de l'enchaînement des différentes étapes de prétraitement des données de puces à ADN selon la méthode RMA.
La figure 3 illustre la nomenclature, la position et la structure des loci HERV-W surexprimés et présentant une perte de méthylation dans le testicule tumoral.
La figure 4 est un histogramme représentant l'accroissement d'expression des cinq loci (HW4TT, HW2TT, HW13TT, HWXTT et HW21TT) respectivement dans trois paires d'échantillons testiculaires (testicule 1, testicule 2 et testicule 3), basé sur une quantification comparative échantillon tumoral/échantillon sain. En abscisse sont représentés les loci et en ordonné sont représentés les facteurs d'accroissement de l'expression entre tissu tumoral et tissu sain.
Les figures 5 à 10 représentent le statut de méthylation de la région U3 de la LTR unique ou de la LTR 5' des différents loci, respectivement HW4TT, HW2TT, HW13TT, HWXTT, HW21TT et ERVWE1 dans le testicule sain (normal) et dans le testicule tumoral issus d'un même patient, après amplification et analyse des séquences obtenues.

### Exemples :

### Exemple 1: Identification de loci HERV-W exprimés dans des tissus cancéreux.

### Méthode :

L'identification de loci HERV-W exprimés repose sur la conception d'une puce à ADN haute densité au format GeneChip proposé par la société Affymetrix. Il s'agit d'une puce à façon, développée spécifiquement, dont les sondes correspondent à des loci HERV-W. Les séquences de la famille HERV-W ont été identifiées à partir de la banque de données nucléiques GenBank en utilisant l'algorithme Blast (Altschul et al. 1990) avec la séquence du locus ERVWE1, situé sur le chromosome 7 en 7q21.2 et codant pour la protéine dénommée syncytine. Les séquences homologues à HERV-W ont été comparées à une bibliothèque contenant des séquences de référence de la famille HERV-W (ERVWE1) découpées en régions fonctionnelles (LTR, *gag, pol* et *env*) en utilisant le logiciel RepeatMasker (A.F.A. Smit and P. Green). Ces éléments constituent la banque HERVgDB.

Les sondes composant la puce haute densité ont été définies sur un critère d'unicité de leurs séquences dans la banque HERVgDB. Les LTR provirales et solitaires HERV-W contenues dans la banque HERVgDB ont été extraites. Chacune de ces séquences, a été décomposée en un ensemble de séquences de 25 nucléotides (25-mers) la composant, soit autant de sondes potentielles. L'évaluation de l'unicité de chaque sonde a été réalisée par recherche de similarité avec l'ensemble des 25-mers générés pour toutes les LTRs de la famille considérée. Ceci a permis d'identifier l'ensemble des 25-mers d'occurrence unique pour chaque famille d'HERV. Ensuite, certains de ces 25-mers ont été retenus comme sondes. Pour chaque région cible U3 ou U5, un jeux de sondes a été constitué à partir des sondes identifiées comme uniques.

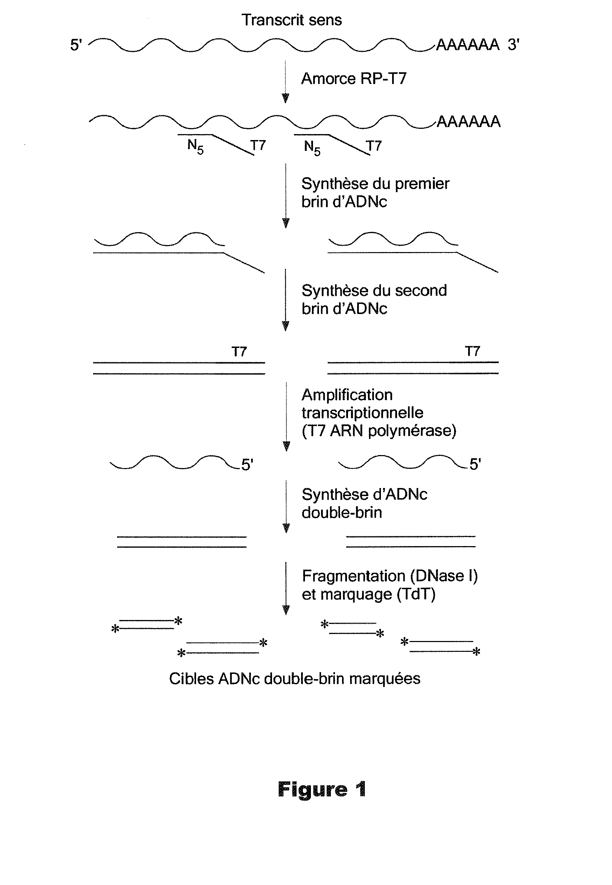

Les échantillons analysés à l'aide de la puce haute-densité HERV correspondent à des ARN extraits de tumeurs et aux ARN extraits des tissus sains adjacents à ces tumeurs. Les tissus analysés sont : l'utérus, le colon, le poumon, le sein, le testicule, la prostate et l'ovaire. Des ARN placentaires (tissu sain uniquement) ont également été analysés. Pour chaque échantillon, 400 ng d'ARN totaux ont été amplifiés à l'aide d'une méthode transcriptionnelle non biaisée connue sous le nom de WTA. Le principe de l'amplification WTA est le suivant: des amorces (RP-T7) comprenant une séquence aléatoire et une séquence promotrice T7 sont hybridées sur les transcrits ; des ADNc double-brin sont synthétisés et servent de matrice à une amplification transcriptionnelle par la T7 ARN polymérase ; les ARN anti-sens générés sont convertis en ADNc double brin qui sont ensuite fragmentés et marqués par introduction d'analogues nucléotidiques biotinylés aux niveaux des extrémités 3'OH par la *terminale transferase* (TdT) (cf. : Figure 1).

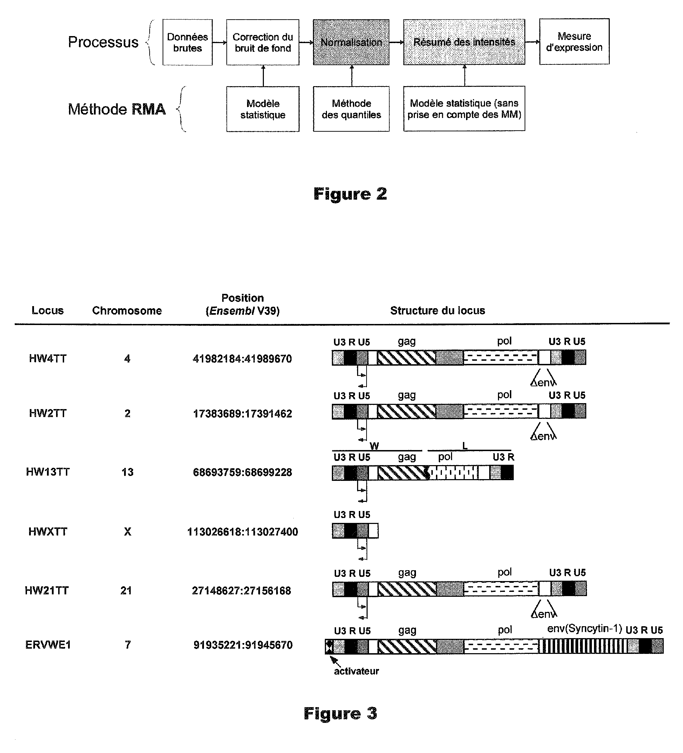

Pour chaque échantillon, 16 µg de produits d'amplification marqués par la biotine ont été hybridés sur puce à ADN selon le protocole recommandé par la société Affymetrix. Les puces ont ensuite été lavées et marquées, selon le protocole recommandé. Les puces ont enfin été lues par un scanner afin d'acquérir l'image de leur fluorescence. L'analyse d'image réalisée à l'aide du logiciel GCOS permet d'obtenir des valeurs numériques d'intensité de fluorescence qui sont prétraitées selon la méthode RMA (cf. : Figure 2) avant de pouvoir réaliser une analyse statistique pour identifier les loci HERV exprimés spécifiquement dans certains échantillons.

La comparaison des moyennes de plus de deux classes d'échantillons a été réalisée par la procédure SAM appliquée à un test de Fisher.

### Résultats :

Le traitement des données générées par l'analyse sur puce à ADN à l'aide de cette méthode a permis d'identifier six jeux de sondes correspondant à une surexpression dans un seul échantillon : le testicule tumoral. Ces cinq jeux de sondes sont spécifiques de six loci précis référencés HW4TT, HW2TT, HW13TT, HWXTT, HW21TT et ERVWE1 (cf. : Figure 3). Ces six loci représentent donc des marqueurs du cancer du testicule. Leurs séquences nucléotidiques sont respectivement identifiées en SEQ ID NOs 1 à 6 dans le listage de séquences et les séquences nucléotidiques de leurs transcrits respectifs sont identifiées en SEQ ID NOs 7 à 12 dans le listage de séquences.

Les informations relatives aux six loci précités sont synthétisées dans le tableau 1 ci-dessous.

**Tableau 1**

| **Locus** | **SEQ ID NO :** | **Chromosome** | **Position*** |
|---|---|---|---|
| HW4TT | 1 | 4 | 41982184:41989670 |
| HW2TT | 2 | 2 | 17383689:17391462 |
| HW13TT | 3 | 13 | 68693759:68699228 |
| HWXTT | 4 | X | 113026618:113027400 |
| HW21TT | 5 | 21 | 27148627:27156168 |
| ERVWE1 | 6 | 7 | 91935221:91945670 |

| | | | |
|---|---|---|---|
| * Position par rapport à la version ensembl n° 39 (juin 2006) (NCBI n° 36) http : //www.ensembl.org/Homo_sapiens/index.html | | | |

Le locus HW13TT est un provirus chimère de type HERV-W/L résultant de la recombinaison d'un provirus HERV-W et d'un provirus HERV-L. Cette chimère est telle que la région 5' constituée de la séquence partant du début de la LTR5' jusqu'à la fin du fragment *gag* déterminé est de type W et la région 3' constituée de la séquence partant du fragment subséquent *pol* jusqu'à la fin de la LTR3' (U3-R uniquement) est de type L. Il en résulte une fusion des régions 3' gag W-5' pol L.

Une recherche de trames de lecture (ORFs) d'au moins 150 bases, à l'aide du logiciel Mac Vector 9.5.2, basée sur l'identification d'un codon d'initiation et d'un codon stop a été effectuée et les polypeptides correspondants identifiés.

L'ORF1 de HW4TT identifiée en SEQ ID NO: 13 code pour une protéine Gag identifiée en SEQ ID NO: 14 et l'ORF 2 de HW4TT (SEQ ID NO : 15) code pour une protéase (SEQ ID NO : 16),
l'ORF1 de HW2TT identifiée en SEQ ID NO: 17 code pour une protéine Gag identifiée en SEQ ID NO : 18 et l'ORF 2 de HW2TT (SEQ ID NO : 19) code pour une protéine identifiée en SEQ ID NO : 20,
l'ORF de HW13TT identifiée en SEQ ID NO : 21 code pour une protéine Gag identifiée en SEQ ID NO : 22,
l'ORF de HW21TT identifiée en SEQ ID NO: 23 code pour une protéine Gag identifiée en SEQ ID NO : 24
l'ORF de ERVWE1 identifiée en QES ID NO: 25 code pour une protéine Env identifiée en SEQ ID NO : 26.

### Exemple 2: Validation des loci sur-exprimés dans le testicule tumoral et détermination du facteur d'induction associé.

### Principe :

Cinq des six loci identifiés comme sur-exprimés dans le testicule tumoral à l'aide de la puce HERV haute densité ont été validés par RT-PCR en temps réel sur trois paires d'échantillons testiculaires. La spécificité de cette sur-expression est évaluée par l'analyse d'échantillons provenant d'autres tissus. A cette fin, des systèmes d'amplification spécifiques ont été mis au point et utilisés pour les loci identifiés, comme décrit dans le tableau 2 ci-dessous.

**Tableau 2**

| **Locus** | **Amorce sens (SEQ ID NO :)** | **Amorce antisens (SEQ ID NO :)** |
|---|---|---|
| Gène G6PD | TGCAGATGCTGTGTCTGG (27) | CGTACTGGCCCAGGACC (28) |
| HW4TT | GGTTCGTGCTAATTGAGCTG (29) | ATGGTGGCAAGCTTCTTGTT (30) |
| HW2TT | TGAGCTTTCCCTCACTGTCC (31) | TGTTCGGCTTGATTAGGATG (32) |
| HW13TT | CATGGCCCAATATTCCATTC (33) | GGTCCTTGTTCACAGAACTCC (34) |
| HWXTT | CCGCTCCTGATTGGACTAAA (35) | CGTGGGTCAAGGAAGAGAAC (36) |
| HW21TT | ATGACCCGCAGCTTCTAACAG (37) | CTCCGCTCACAGAGCTCCTA (38) |

L'expression de ces loci est normalisée par celle d'un gène de ménage approprié : G6PD. Cette quantification d'expression a été réalisée à l'aide d'un appareil de RT-PCR en temps réel Mx3005P, commercialisé par la société Stratagene.

### Résultats :

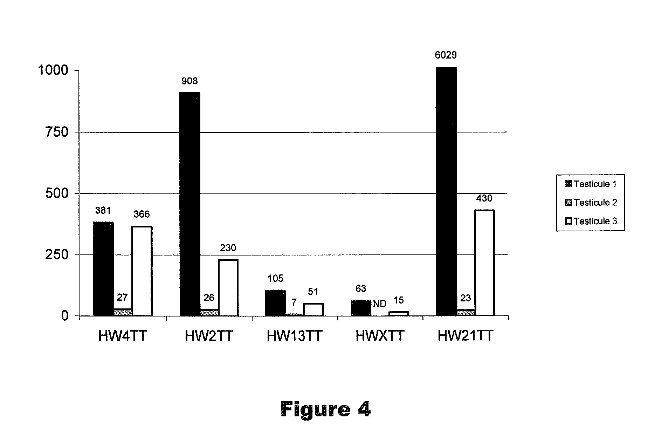

L'étude des trois paires d'échantillons testiculaires indique que les cinq loci identifiés, à l'exception de HWXTT, dont l'expression n'a pas pu être quantifiée dans le second couple d'ARN testiculaire, sont sur-exprimés dans le testicule tumoral par rapport au tissu sain (cf. : Figure 4). Le caractère très marqué de la sur-expression, c'est à dire une expression transcriptionnelle faible, voire absente, dans le testicule sain et une expression forte dans le testicule tumoral, révèle la possibilité d'un mode de régulation épigénique de la transcription de ces loci.

L'analyse de paires d'échantillons provenant d'autres tissus (colon, utérus, sein ovaire, poumon et prostate) montre que le phénomène de sur-expression est restreint au testicule tumoral. Par conséquent, l'expression des loci identifiés revêt le caractère de marqueur spécifique du cancer du testicule.

### Exemple 3 : Contrôle épigénétique de la transcription.

### Principe :

La méthylation de l'ADN est une modification épigénétique, qui s'opère, chez les eucaryotes, par l'addition d'un groupement méthyle sur les cytosines des di-nucléotides 5'-CpG, et se traduit par une répression transcriptionnelle quand cette modification a lieu au sein de la séquence nucléotidique d'un promoteur. Mises à part quelques exceptions, les séquences endogènes humaines d'origine rétrovirale sont contraintes, par ce processus de méthylation, à un état transcriptionnel silencieux dans les cellules de l'organisme en condition physiologique.

Afin d'analyser le statut de méthylation de la LTR unique ou de la LTR 5' des cinq loci, la méthode dite de « Bisulfite Sequencing PCR » a été utilisée. Cette méthode permet, à partir du séquençage d'un échantillon représentatif de la population, d'identifier l'état de méthylation de chaque dinucléotide CG sur chacune des séquences au sein du tissu étudié.

L'information de méthylation étant perdue lors des étapes d'amplification, il convient, par la méthode de traitement de l'ADN génomique par le bisulfite de sodium, de traduire l'information de méthylation au sein même de la séquence nucléotidique. L'action du bisulfite (sulfonation), suivie d'une désamination hydrolytique puis d'une désulfonation alcaline, permet en effet de modifier toutes les cytosines contenues dans l'ADN génomique en uracile. La vitesse de désamination des cytosines (C) sulfonées est cependant bien supérieure à celle des 5-méthyl-C sulfonées. On peut donc, en limitant le temps de réaction à 16 heures, transformer de manière stricte les cytosines non méthylées en uracile (U), tout en préservant les cytosines possédant un groupement méthyle. Après le traitement au bisulfite de sodium, la séquence d'intérêt est amplifiée à partir de l'ADN génomique issu de la section testiculaire tumorale et de celui issu de la section testiculaire saine adjacente par réaction de polymérisation en chaîne (PCR) en deux étapes. La première PCR permet une sélection spécifique de la séquence d'intérêt, la seconde PCR «nichée» permet d'amplifier cette séquence.

La séquence ADN ayant été modifiée par le bisulfite, la conception des amorces a tenu compte du changement de code (C en U), et les amorces ont été choisies de manière à s'hybrider sur une zone ne contenant aucun CpG (leur état de méthylation, donc de conversion, étant a priori inconnu).

Les séquences des amorces utilisées sont décrites dans les tableaux 3 à 8, ci-dessous.

**Tableau 3 - locus HW4TT**

| **Amplification** | **Amorce sens 5'→3'* (SEQ ID NO :)** | **Amorce antisens 5'→3' (SEQ ID NO :)** |
|---|---|---|
| Première PCR | CCAACATCACTAACACAACCT(39) | GGGAGTTAGTAAGGGGTTTG(40) |
| PCR nichée | CAACCTATTAAACAAAACTAAATT(41) | AGATTTAATAGAGTGAAAATAGAGTTT(42) |

**Tableau 4 - locus HW2TT**

| **Amplification** | **Amorce sens 5'→3' (SEQ ID NO :)** | **Amorce antisens 5'→3' (SEQ ID NO :)** |
|---|---|---|
| Première PCR | TTATTAGTTTAGGGGATAGTTG (43) | ACACAATAAACAACCTACTAAAT (44) |
| PCR nichée | GAGGGTAAGTGGTGATAAA (45) | AACCTACTAAATCCAAAAAAA (46) |

**Tableau 5 -locus HW13TT**

| **Amplification** | **Amorce sens 5'→3' (SEQ ID NO :)** | **Amorce antisens 5'→3' (SEQ ID NO :)** |
|---|---|---|
| Première PCR | TAGGATTTTAGGTTTATTGTTA (47) | AAAAATAAAATATTAAACC (48) |
| PCR nichée | ATATGTGGGAGTGAGAGATA (49) | CAACAACAAACAATAATAATAA (50) |

**Tableau 6 - locus HWXTT**

| **Amplification** | **Amorce sens 5'→3' (SEQ ID NO :)** | **Amorce antisens 5'→3' (SEQ ID NO :)** |
|---|---|---|
| Première PCR | TTGAGTTTTTTTATTGATAGTG (51) | TCTAAATCCTATTTTCCTACT (52) |
| PCR nichée | GTTTTTTTATTGATAGTGAGAGAT (53) | TAACAAACCTTTAATCCAAT (54) |

**Tableau 7 - locus HW21TT**

| **Amplification** | **Amorce sens 5'→3' (SEQ ID NO :)** | **Amorce antisens 5'→3' (SEQ ID NO :)** |
|---|---|---|
| Première PCR | TTTAGTGAGGATGATGTAATAT (55) | CAACTTAATAAAAATAAACCCA (56) |
| PCR nichée | ATAATGTTTTAGTAAGTGTTGGAT (57) | ACAATTACAAACCTTTAACC (58) |

**Tableau 8 - locus ERVWE1**

| **Amplification** | **Amorce sens 5'→3' (SEQ ID NO :)** | **Amorce antisens 5'→3' (SEQ ID NO :)** |
|---|---|---|
| Première PCR | AATTCATTCAACATCCATTC (59) | GGTTTAATATTATTTATTATTTTGGA (60) |
| PCR nichée | CTCTTACCTTCCTATACTCTCTAAA (61) | AGAGTGTAGTTGTAAGATTTAATAGAGT(62) |

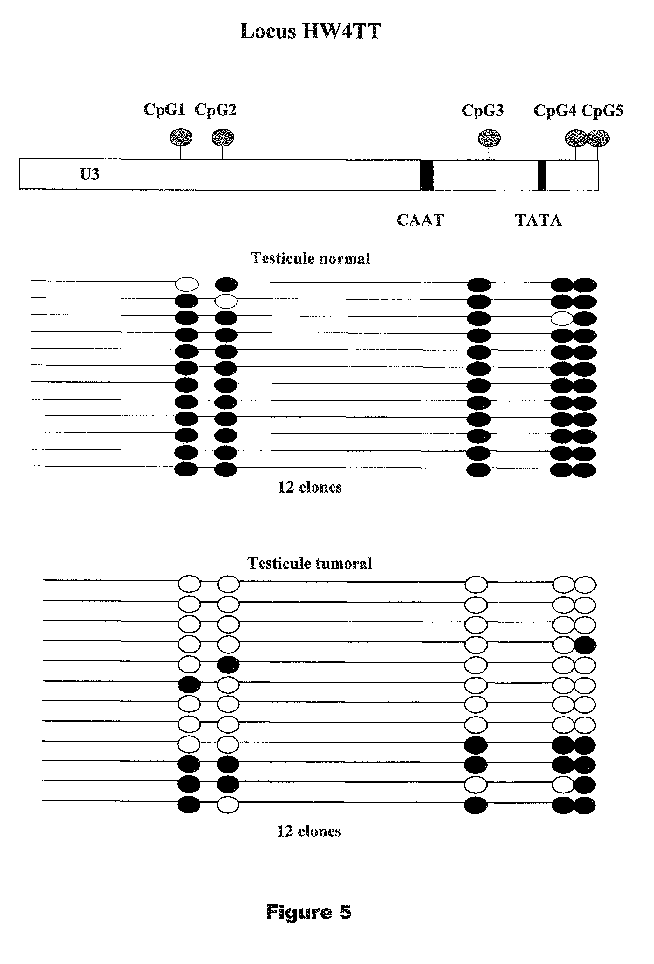

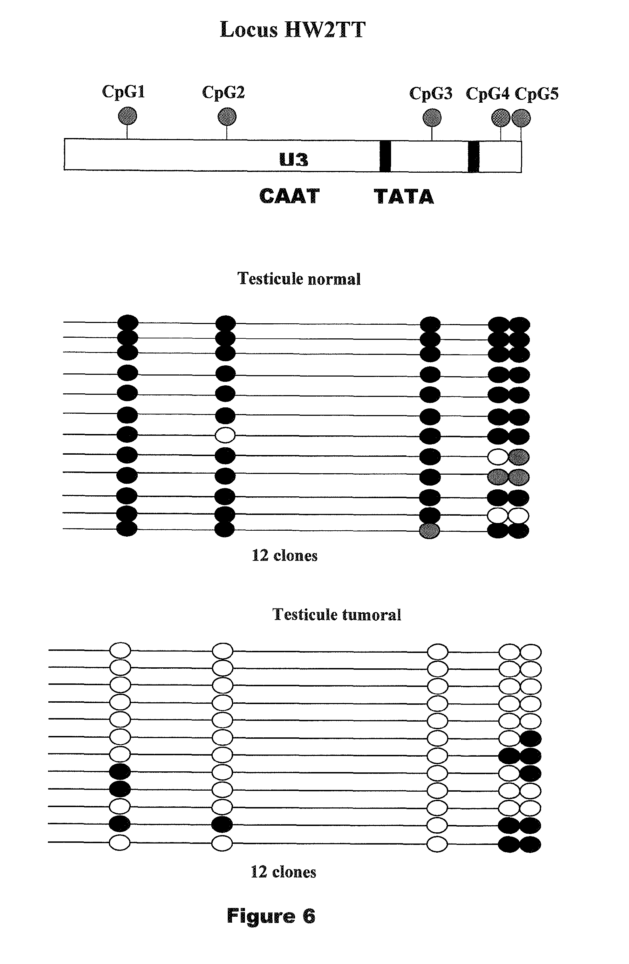

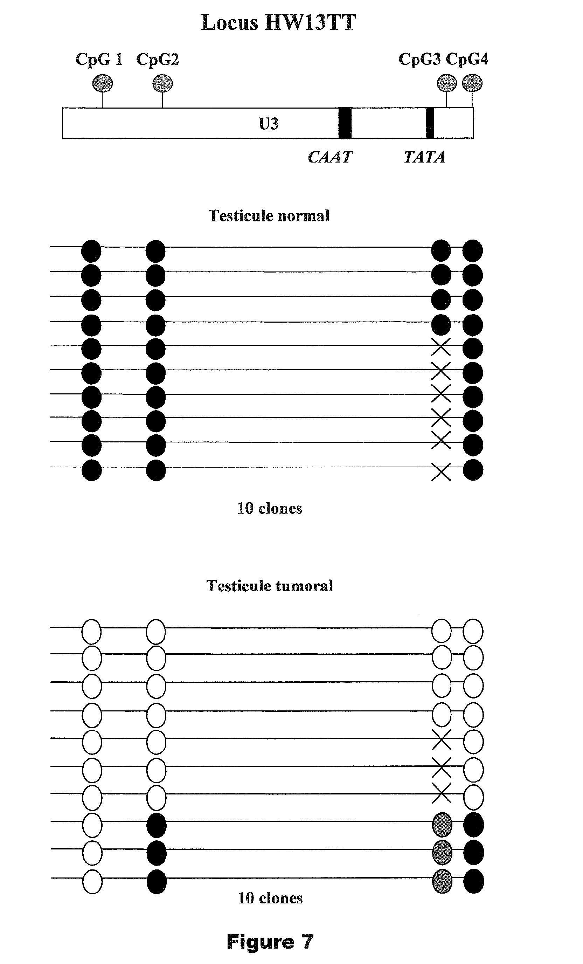

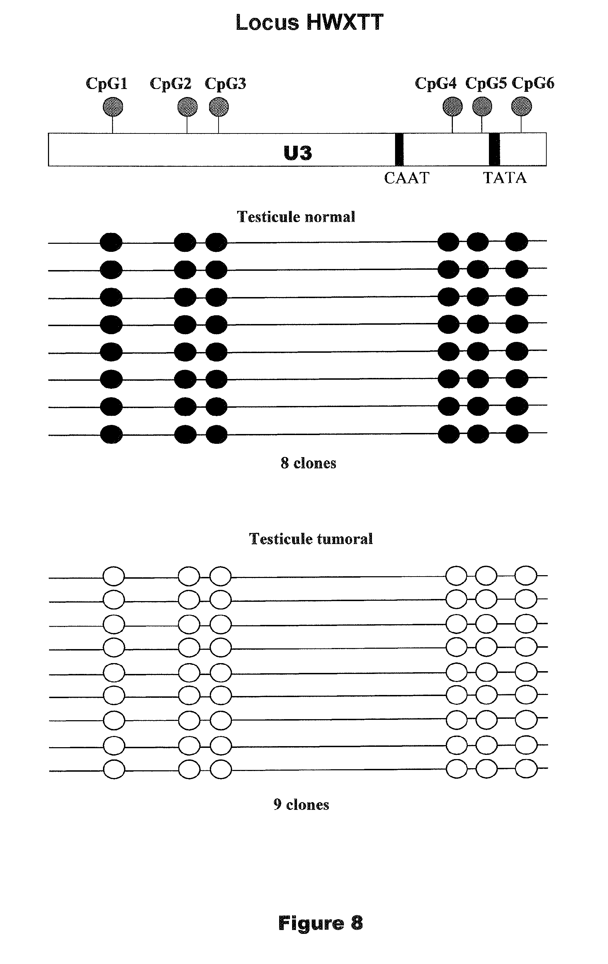

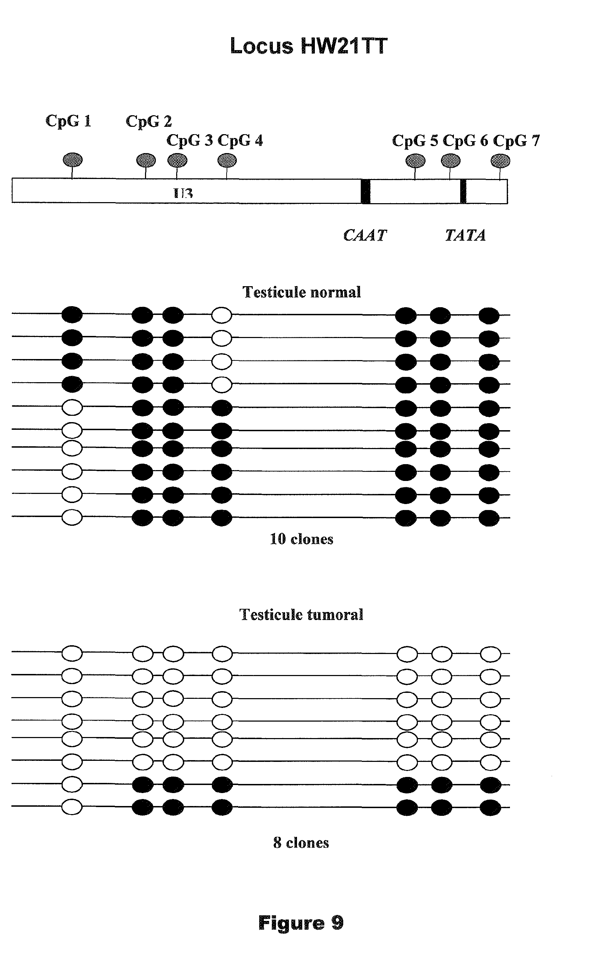

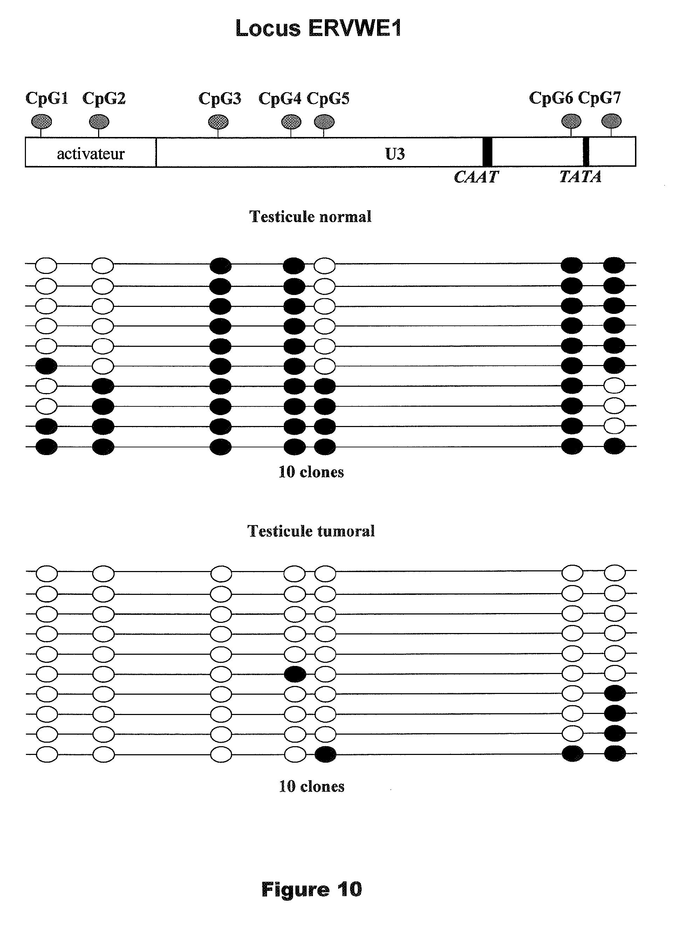

Après extraction sur gel, et purification, les amplicons sont clonés dans des plasmides, et ces derniers sont utilisés pour transformer des bactéries compétentes. Une douzaine de minipréparations d'ADN plasmidique sont réalisées à partir des bactéries transformées et les amplicons contenus dans les plasmides sont séquencés. On procède alors à l'analyse des séquences obtenues (cf. : Figures 5 à 10).

### Résultats :

L'analyse de la région 5' des transcrits des loci identifiés a été réalisée à l'aide de la technique 5' Race. Elle a notamment permis de montrer que la transcription est initiée au début de la région R de la LTR 5' provirale. Ceci traduit l'existence d'un rôle promoteur de la région U3 de la LTR 5' provirale.

### 1. Etat de méthylation des séquences U3 de la LTR5'du locus HW4TT :

La séquence U3 de la LTR5' du locus HW4TT de référence contient 5 sites CpGs.
a) dans l'échantillon de tissu testiculaire sain : sur 12 séquences analysées, 9 sont totalement méthylées. Les 3 autres présentent à chaque fois 1 CpG non méthylé sur les 5 que compte la région U3. Ceci représente donc une méthylation globale de la région U3 de la LTR5' du locus HW4TT s'élevant à 95% dans l'échantillon testiculaire sain ;
b) dans l'échantillon de tissu testiculaire tumoral : sur 12 séquences analysées, 5 (soit 41,66% des séquences) sont totalement déméthylées, 3 séquences ont 4 CpG sur 5 non méthylés, 2 séquences ont 2 CpG sur 5 non méthylés, 1 séquence a 1 CpG sur 5 non méthylé, et 1 séquence reste totalement méthylée. Ceci représente donc une méthylation globale de la région U3 de la LTR5' du locus HW4TT s'élevant à 30% dans l'échantillon testiculaire tumoral.

### 2. Etat de méthylation des séquences U3 de la LTR5'du locus HW2TT :

La séquence U3 de la LTR5' du locus HW2TT de référence contient 5 sites CpGs.
a) dans l'échantillon de tissu testiculaire sain : sur 12 séquences analysées, 9 sont totalement méthylées, 1 possède son 2^{ème} CpG non méthylé, 1 a le CpG en position 4 non méthylé, 1 a les CpG en position 4 et 5 non méthylés et 3 séquences présentent des mutations ponctuelles sur un ou deux CpG (une en position 3, une en position 5 et une en positions 4 et 5), reflétant très probablement des artefacts de PCR. Ceci représente donc une méthylation globale de la région U3 de la LTR5' du locus HW2TT s'élevant à 92,9% dans l'échantillon testiculaire sain ;
b) dans l'échantillon de tissu testiculaire tumoral : sur 12 séquences analysées, 6 sont totalement déméthylées. 5 séquences possèdent un ou deux CpG méthylé(s) (1 à la position 1, 1 autre à la position 5, 1 sur les positions 1 et 5, 2 aux positions 4 et 5 et 1 à la position 3). Enfin, une séquences possède 4 CpG méthylés sur 5 (positions 1, 2, 4 et 5). Ceci correspond à une méthylation globale de la région U3 de la LTR5' du locus HW2TT s'élevant à 20% dans l'échantillon testiculaire tumoral.

### 3. Etat de méthylation des séquences U3 de la LTR5'du locus HW13TT :

La séquence U3 de la LTR5' du locus HW13TT de référence contient 3 sites CpGs.
a) dans l'échantillon de tissu testiculaire sain : un CpG supplémentaire par rapport à la séquence de référence est retrouvé dans 4 des 10 clones étudiés pour ce locus. Il est situé entre les CpG 2 et 3 et est méthylé. Dans les 6 autres clones, ce site est muté par rapport à la séquence de référence. Les 3 autres CpG de la région U3 sont méthylés dans les 10 séquences analysées. Ceci représente donc une méthylation globale de la région U3 de la LTR5' du locus HW13TT s'élevant à 100% dans l'échantillon testiculaire sain ;
b) dans l'échantillon de tissu testiculaire tumoral : le CpG supplémentaire indiqué ci-dessus est également retrouvé. Il est déméthylé dans 4 des 10 séquences analysées, muté dans 3 autres séquences et son état de méthylation est indéterminé dans les 3 dernières séquences. 7 séquences sur 10 sont totalement déméthylées et les 3 autres sont méthylées sur le 2^{ème} et sur le 3^{ème} CpG. Ceci correspond à une méthylation globale de la région U3 de la LTR5' du locus HW13TT s'élevant à 20% dans l'échantillon testiculaire tumoral.

### 4. Etat de méthylation des séquences U3 de la LTR solitaire du locus HWXTT :

La séquence U3 de la LTR5' du locus HWXTT de référence contient 6 sites CpGs.
a) dans l'échantillon de tissu testiculaire sain : les 8 séquences analysées sont totalement méthylées, ce qui correspond à un pourcentage de méthylation de 100% dans l'échantillon testiculaire sain ;
b) dans l'échantillon de tissu testiculaire tumoral : les 9 séquences analysées 6 sont totalement déméthylées, ce qui correspond à un pourcentage de méthylation de 0%.

### 5. Etat de méthylation des séquences U3 de la LTR5'du locus HW21TT :

La séquence U3 de la LTR5' du locus HW21TT de référence contient 7 sites CpGs.
a) dans l'échantillon de tissu testiculaire sain : les 10 séquences analysées ont toutes 6 CpG méthylés sur 7, pour 6 des séquences le 1^{er} CpG est non méthylé et pour les 4 autres séquences le 4^{ème} CpG est non méthylé. Ceci représente donc une méthylation globale de la région U3 de la LTR5' du locus HW21TT s'élevant à 85,7% dans l'échantillon testiculaire sain ;
b) dans l'échantillon de tissu testiculaire tumoral : sur 8 séquences analysées, 6 sont totalement déméthylées, 2 autres présentent un profil identique à l'un de ceux trouvés dans le tissu testiculaire sain, à savoir 6 CpG méthylés et le 1^{er} CpG non méthylé. Ceci correspond à une méthylation globale de la région U3 de la LTR5' du locus HW21TT s'élevant à 21,4% dans l'échantillon testiculaire tumoral.

### 6. Etat de méthylation des séquences de l'activateur et de la U3 de la LTR 5' du locus ERVWE1:

Le locus ERVWE1 comprend, en plus de sa région U3 promotrice, un activateur connu localisé directement en amont de la LTR5', et qui contient deux sites CpG (CpG 1 et 2). La séquence U3 de la LTR5' du locus ERVWE1 de référence contient qant à elle 5 sites CpGs (CpG 3 à 7).
a) dans l'échantillon de tissu testiculaire sain : sur 10 séquences analysées, 5 séquences ont les CpG 1 et 2 (activateur) et 5 (U3) non méthylés, 1 séquence a les CpG 2 et 5 non ùéthylés, 2 séquences ont les CpG 1 (activateur) et 7 (U3) non méthylés, 1 séquence a le CpG 7 uniquement non méthylé, et 1 enfin est totalement méthylée pour les 7 CpG. Au total, ceci correspond à un pourcentage de méthylation de 68,57% dans l'échantillon testiculaire sain ;
b) dans l'échantillon de tissu testiculaire tumoral : sur les 10 séquences analysées, seules 3 séquences présentent pour chacune un unique CpG méthylé (CpG 4 ou CpG5 ou CpG6), les 7 autres séquences sont totalement déméthylées, ce qui correspond à un pourcentage de méthylation de 4,29%.

Le niveau de méthylation très élevé des promoteurs rétroviraux U3 des loci considérés dans le tissu sain est corrélé à l'expression transcriptionnelle faible, voire absente, des régions U5 qui correspondent aux loci considérés, indiquant une répression de l'expression transcriptionnelle par un mécanisme épigénétique. En revanche, le niveau faible de méthylation de ces mêmes promoteurs dans le tissu tumoral traduit une levée d'inhibition transcriptionnelle dont découle l'expression significativement plus élevée mise en évidence à l'aide de la puce ADN HERV haute densité et à l'aide de la RT-PCR en temps réel.

### Références bibliographiques

[1] Nickerson D. A. et al., DNA sequence diversity in a 9.7 -kb region of the human lipoprotein lipase gene, Nature Genetics, Vol. 19, pp 233-240 (1998).
[2] Cottrell S. E., Molecular diagnostic applications of DNA methylation technology, Clinical Biochemistry 37, pp 595-604 (2004).

## Revendications

1. Procédé pour le diagnostic ou le pronostic, *in vitro,* du cancer du testicule dans un échantillon de testicule, issu d'un patient suspecté d'être atteint d'un cancer du testicule, **caractérisé en ce qu'**il est comprend une étape de détection de la présence ou de l'absence d'au moins un produit d'expression d'au moins une séquence d'acide nucléique choisie parmi les séquences identifiées en SEQ ID NOs : 1 à 6 ou parmi les séquences qui présentent au moins 99% d'identité avec une des séquences identifiées en SEQ ID NOs : 1 à 6, une étape de comparaison de la quantité du produit d'expression de l'échantillon à analyser par rapport à la quantité du produit d'expression d'un échantillon sain ; dans lequel une surexpression dans l'échantillon analysé par rapport à l'expression dans l'échantillon sain est corrélée au cancer du testicule.

2. Procédé selon la revendication 1, **caractérisé en ce que** le produit d'expression détecté est au moins un transcrit ARNm ou au moins un polypeptide.

3. Procédé selon la revendication 2, **caractérisé en ce que** le transcrit ARNm est détecté par hybridation, par amplification ou par séquençage.

4. Procédé selon l'une quelconque des revendications précédentes, **caractérisé en ce que** le produit d'expression est au moins un transcrit ARNm dont la séquence est choisie parmi une des séquences identifiées en SEQ ID NOs : 7 à 12

5. Procédé selon l'une quelconque des revendications précédentes, **caractérisé en ce que** l'ARNm est mis en contact avec au moins une sonde et/ou au moins une amorce dans des conditions prédéterminées permettant l'hybridation et **en ce qu'**on détecte la présence ou l'absence d'hybridation à l'ARNm.

6. Procédé selon l'une quelconque des revendications précédentes, **caractérisé en ce qu'**on prépare des copies ADN de l'ARNm, on met en contact les copies ADN avec au moins une sonde et/ou au moins une amorce dans des conditions prédéterminées permettant l'hybridation et **en ce qu'**on détecte la présence ou l'absence d'hybridation aux dites copies ADN.

7. Procédé selon la revendication 2, **caractérisé en ce que** l'on détecte le polypeptide exprimé par mise en contact avec au moins un partenaire de liaison spécifique dudit polypeptide, en particulier un anticorps.

8. Utilisation d'au moins une séquence d'acide nucléique, isolée, comme marqueur moléculaire pour le diagnostic ou le pronostic *in vitro* du cancer du testicule, **caractérisée en ce que** ladite séquence d'acide nucléique consiste en :
(i) au moins une séquence ADN choisie parmi les séquences SEQ ID NOs : 1 à 6, ou
(ii) au moins une séquence ADN complémentaire d'une séquence choisie parmi les séquences SEQ ID NOs : 1 à 6, ou
(iii) au moins une séquence ADN qui présente au moins 99% d'identité avec une séquence telle que définie en (i) et (ii), ou
(iv) au moins une séquence ARN qui est le produit de transcription d'une séquence choisie parmi les séquences telles que définies en (i), ou
(v) au moins une séquence ARN qui est le produit de transcription d'une séquence choisie parmi les séquences qui présentent au moins 99% d'identité avec une séquence telle que définie en (i), ou
(vi) au moins une séquence ARN choisie parmi les séquences SEQ ID NOs : 7 à 12.

## Patentansprüche

1. Verfahren zum Diagnostizieren oder Prognostizieren in vitro von Hodenkrebs in einer Hodenprobe von einem Patienten, von dem vermutet wird, dass er an Hodenkrebs leidet, **dadurch gekennzeichnet, dass** es einen Schritt des Nachweisens des Vorliegens oder Fehlens von mindestens einem Expressionsprodukt von mindestens einer Nukleinsäuresequenz, ausgewählt aus den Sequenzen gemäß SEQ ID NO: 1 bis 6 oder aus den Sequenzen mit mindestens 99% Identität zu den Sequenzen SEQ ID NO: 1 bis 6, einem Schritt des Vergleichens der Menge des Expressionsprodukts der zu analysierenden Probe im Vergleich zu der Menge des Expressionsprodukts in einer gesunden Probe umfasst; wobei eine Überexpression in der analysierten Probe im Vergleich zu der Expression in der gesunden Probe mit Hodenkrebs korreliert.

2. Verfahren nach Anspruch 1, **dadurch gekennzeichnet, dass** das nachgewiesene Expressionsprodukt mindestens ein mRNA-Transkript oder mindestens ein Polypeptid ist.

3. Verfahren nach Anspruch 2, **dadurch gekennzeichnet, dass** das mRNA-Transkript mittels Hybridisierung, Amplifizierung oder Sequenzierung nachgewiesen wird.

4. Verfahren nach einem der vorhergehenden Ansprüche, **dadurch gekennzeichnet, dass** das Expressionsprodukt mindestens ein mRNA-Transkript ist, dessen Sequenz aus einer der Sequenzen gemäß SEQ ID NO: 7 bis 12 ausgewählt ist.

5. Verfahren nach einem der vorhergehenden Ansprüche, **dadurch gekennzeichnet, dass** die mRNA mit mindestens einer Sonde und/oder mindestens einem Primer unter vorbestimmten Bedingungen, die die Hybridisierung gestatten, in Kontakt gebracht wird und das Vorliegen oder Fehlen einer Hybridisierung mit der mRNA nachgewiesen wird.

6. Verfahren nach einem der vorhergehenden Ansprüche, **dadurch gekennzeichnet, dass** man DNA-Kopien der mRNA herstellt, die DNA-Kopien mit mindestens einer Sonde und/oder mindestens einem Primer unter vorbestimmten Bedingungen, die die Hybridisierung gestatten, in Kontakt bringt und das Vorliegen oder Fehlen von Hybridisierung der genannten DNA-Kopien nachgewiesen wird.

7. Verfahren nach Anspruch 2, **dadurch gekennzeichnet, dass** man das exprimierte Polypeptid dadurch nachweist, dass man es mit mindestens einem spezifischen Bindungspartner des Polypeptids, insbesondere einem Antikörper, in Kontakt bringt.

8. Verwendung von mindestens einer isolierten Nukleinsäuresequenz als molekularem Marker für das Diagnostizieren oder Prognostizieren in vitro von Hodenkrebs, **dadurch gekennzeichnet, dass** die Nukleinsäuresequenz aus Folgendem besteht:
(i) mindestens einer DNA-Sequenz, ausgewählt aus den Sequenzen SEQ ID NO: 1 bis 6, oder
(ii) mindestens einer DNA-Sequenz, die zu einer Sequenz, ausgewählt aus den Sequenzen SEQ ID NO: 1 bis 6, komplementär ist, oder
(iii) mindestens einer DNA-Sequenz, die mindestens 99% Identität zu einer Sequenz wie in (i) und (ii) definiert aufweist, oder
(iv) mindestens einer RNA-Sequenz, die das Produkt der Transkription einer aus den Sequenzen wie in (i) definiert ausgewählten Sequenz ist, oder
(v) mindestens einer RNA-Sequenz, die das Produkt der Transkription einer Sequenz, ausgewählt aus den Sequenzen mit mindestens 99% Identität zu einer in (i) definierten Sequenz, ist, oder
(vi) mindestens einer aus den Sequenzen SEQ ID NO: 7 bis 12 ausgewählten RNA-Sequenz.

## Claims

1. A method for in vitro diagnosis or prognosis of testicular cancer, in a testicular sample, from a patient suspected of suffering from testicular cancer, **characterized in that** it comprises a step of detecting the presence or absence of at least one expression product from at least one nucleic acid sequence selected from the sequences identified in SEQ ID Nos.: 1 to 6 or from the sequences which exhibit at least 99% identity with one of the sequences identified in SEQ ID Nos.: 1 to 6, a step of comparing the quantity of the expression product of the sample to be analyzed, compared with the quantity of the expression product of an healthy sample; in which an overexpression in the analyzed sample, compared with the expression in the healthy sample, is correlated to the testicular cancer.

2. The method according to claim 1, **characterized in that** the expression product detected is at least one mRNA transcript or at least one polypeptide.

3. The method according to claim 2, **characterized in that** the mRNA transcript is detected by hybridization, amplification or sequencing.

4. The method according to any one of the preceding claims, **characterized in that** the expression product is at least one mRNA transcript, the sequence of which is selected from one of the sequences identified in SEQ ID Nos.: 7 to 12.

5. The method according to any one of the preceding claims, **characterized in that** the mRNA is brought into contact with at least one probe and/or at least one primer under predetermined conditions which enable hybridization, and **in that** the presence or absence of hybridization to the mRNA is detected.

6. The method according to any one of the preceding claims, **characterized in that** DNA copies of the mRNA are prepared, the DNA copies are brought into contact with at least one probe and/or at least one primer under predetermined conditions which enable hybridization, and **in that** the presence or absence of hybridization to said DNA copies is detected.

7. The method according to claim 2, **characterized in that** the polypeptide expressed is detected by bringing it into contact with at least one binding partner specific for said polypeptide, in particular an antibody.

8. The use of at least one isolated nucleic acid sequence, as a molecular marker for *in vitro* diagnosis or prognosis of testicular cancer, **characterized in that** said nucleic acid sequence consists of:
(i) at least one DNA sequence selected from the sequences SEQ ID Nos.: 1 to 6, or
(ii) at least one DNA sequence complementary to a sequence selected from the sequences SEQ ID Nos.: 1 to 6, or
(iii) at least one DNA sequence which exhibits at least 99% identity with a sequence as defined in (i) and (ii), or
(iv) at least one RNA sequence which is the product of transcription of a sequence selected from the sequences as defined in (i), or
(v) at least one RNA sequence which is the product of transcription of a sequence selected from the sequences which exhibit at least 99% identity with a sequence as defined in (i), or
(vi) at least one RNA sequence chosen from the sequences SEQ ID Nos.: 7 to 12.


### SEQUENCE LISTING

<110> bioMérieux
<120> Procédé pour le diagnostic ou pronostic in vitro du cancer du testicule
<130> Cancerendo1
<150> FR08/51619
   <151> 2008-03-12
<160> 62
<170> PatentIn version 3.3
<210> 1
   <211> 7487
   <212> DNA
   <213> Homo sapiens
<400> 1
   tgagaaacag gactagttag atttcctagg ccaactaaga atccctaagc ctagctggga     60
   aggtgatcgc atccaccttt aaacacgggc ttgcaactta gctcacacct gaccaatcag    120
   gtagtaaaga gagctcacta aaatgctaat taggcaaaaa caggaggtaa agaaatagcc    180
   aatcatctat tgcctgacac cacacgggga gggacaatga ttgggatata aacccaggaa    240
   ttcgagctgg caacggcaac tccctttggg tctcctctca ttgtatggga gctctgtttt    300
   cactctatta aatcttgcaa ctgcacactc ttctggtctg tgtttgttat ggcttgagct    360
   gagcttttgc tggctgtcca ccactgctgt ttgctgccgt cgcagacccc ttgctgactc    420
   ccacccctgc ggatctggca gggtgtctgc tgcgctcctg atccagccag gcacccactg    480
   ctgctcccaa tcaggctaaa ggcttgccat tgttcctgca tggctaagtg cccgggttcg    540
   tgctaattga gctgaacact agtcgctggg ttccacagtt ctcttccgtg acccacagct    600
   tctaatagag ctataacact cactgcatgg cccaacattc cattccttgg aatctgtgag    660
   gccaagaacc cccggtcaga gaacaagaag cttgccacca tcttggaagc agcccgccac    720
   cattttggga gctctaagaa caaggacccc ccagtaacat tttggtgacc acgaagggac    780
   ctccaaagca gtgagtaata ttgaaccact tccgcttgct attctgtcct aaccttcctt    840
   agaattggag gaaaataccg ggcacctgtc ggccagttaa gaacgattag cgtggccgcc    900
   agacttaaga ctctggtgtg aggctgtctg ggaaagggct ttctaacaac ccccaaccct    960
   tccgggttgg gagctttggt ctgcctggaa ccagcttcca ctttcaattt tcctggggaa   1020
   tccaagggct gactagaggc agaaagctgt catcccgaac tcctggcatt agacagttga   1080
   gatcgtggcg cagccagaag tctctactca acagtcaccc atgcgtgcac ccctaccttt   1140
   ccttctaacc catacctccc gggtcccaac catgactttc ttgaaagtgt agcccctaaa   1200
   ttctctttac ctctaaatct acttcttctc atccctgctt cctaggtact aatggttcag   1260
   actttcattt cctctagcaa gttctatctc cagagggatc taaggaaggg atctatgctg   1320
   tgtccttagg cccctaggct atgaacccag agagtcttct ccctgttatc tctccccatt 1380
   taggcataca gctctcaaca tggacagtta tgtgggaccc attccctacc acccttgcca 1440
   gggccccaag ttttcaaagg gctagaagaa aaaagagaga aagagagaga gaggcagagg 1500
   ggagagaaag agagagagac aaagagggag tcaaagagag atagaaagag aaagatagaa 1560
   ctagtaaaga aaaaaagtat gccccattcc tttaaaagcc agggtaaatt taaaacctat 1620
   aattgataat tgaaggtctt ctccatgacc ctataacact ccaataccac cttgttttca 1680
   gtgtaaacaa gggtgtagcc cgaaaacact gagaccactg acaacccata gccttcctat 1740
   caaaaatcct taacccagga acccatggat ggcccaaatg cattcaatct gtagcagcaa 1800
   ctgctttgct aacagaagaa agtagaaaag taacttttag agaaaacctc attgtgagca 1860
   cacctcacca gttcagaatt attctaagtc aaaaaagcaa aaaggtagct tactaactca 1920
   aaaatcttaa agtatggggt tattctgtta gaaaaaggtg atttaacatt aaccactgaa 1980
   aattccctta acccagcagg tttcctaatg ggatttaaat cttcattacc atacaaaggt 2040
   ccgaccagac ccagcaggaa ctccctttag gacaggatga tagatggttc ctcctgggtg 2100
   attgaggggg tgaaaaacca caatgggtgt tcagtaattg atagggagac tcttgtggaa 2160
   ggagagttag gaaaattgcc taataattgg tctgctcaaa tgtgcgagct gtttgcactc 2220
   agccaagcct taaagtactt acagaatcaa aaagactcta tctcaatcct gactcaaaat 2280
   gttacctaca ccatctctga catgaatttg cataagaact gttgtttatg ggaatgcatc 2340
   ttgatggggc agctgggttg ttatgaaata ctcaggaacc cagcccaggt ctagaattca 2400
   cctctgagcg caaaggcaat gttggccatg ctggtaaagg accactagaa tccaggagcc 2460
   tggacccctt tctttgtggt caagaaaggc gggaaaacag gtgcaggact gctacatcag 2520
   agagcataac aaatccgata agcagagttc catgagtggt taagcaccct ggaaaggaac 2580
   tcacctctga gtgcaaaggc aatgttaggc acaccagtaa aggaccacta gaatccagca 2640
   gcccagaccc ctttctttgt gatcaagaaa ggcgggaaaa ggggtgcagg actgctacat 2700
   cagtgagcgt aactaatctg ataagcagaa gtccatgggt ggttacgcac cctggaaagg 2760
   aataagcatt aggaccacag aggacactct aagactaatg ctcattggaa aatgactagg 2820
   ggtgctggca tccctatgtt tttttttcag atgggaaaca ttccccccaa ggcaaaaacg 2880
   cccataagat atattctgga gaattcggcc cagagtgtat gtatcttttt tccctgtcag 2940
   acttgaagca aacctaggta aattatcaga tagccctgat ggctatattg atgctttaca 3000
   agggttagga caatcctttg atctaacatg gagagatata ctgttactgc tagatcagac 3060
   actaatccca aatgaaagaa gtgccaccat aactgcagcc agagagtttg atgatctctg 3120
   gtatctcagt caggtcaatg ataggatgac aacagaagaa agaaaacaat tccccacagg 3180
   ccagcaggca gttcccagcg tagaccttca ttgggacaca gaatcagaac atggagattg 3240
   gtgccgcaga catttactaa cttgcgcgct agaagcacta aggaaaacta ggaagaagcc 3300
   tatgaattat tcaatgatgt ccactataac acagggaaag gaagaaaatc ctactgcctt 3360
   tctggagaga ctaagggagg cattgagaaa gcatacctct ctgtcacctg actctattga 3420
   aggccaacta atcttaaagg ataagttttc cactcagtca gctgcagaca ttagaaaaaa 3480
   acttcaaaag tctgcgttag gccgggagca aaacttagaa accctattga acttggcaac 3540
   ctcagttttt tatgatagag atcaggagga tcaggtggaa tggacaaatg agattttaaa 3600
   aaaaggccac cactttagtc atggccctca ggcaagcaga ctttggacac tctggaaaag 3660
   ggaaaagctg ggcaaatcga atgcctaata agacttgctt ccagtgtggt ctacaaggac 3720
   actttaaaaa agattgtcca aatagaaata agccaccccc tcgtccatgc tccttatgtc 3780
   aagggaatca ctggaaggcc tactgcccca ggggatgaag gtcctctgag tcagaagcca 3840
   ctaaccagat gattcagccc caggactcag ggtgcccagg gcaagcgcca gcctatgcca 3900
   tcaccctcac agagccctgg gtatgcttga ccattgaggg tcaggaggtt aactatctcc 3960
   tggacactgg cgtggccttc tcagtcttac tctcctgtcc cggacaactg tcctccagat 4020
   ctgtcactat ccgagggttt ctacgacagc cagccactag atacttctcc cagccactaa 4080
   gttgtgactg gggaactcta ctcttttcac atgtttttct aattatgcct gaaagcccca 4140
   ctcctttgtt agggaaagac attctagcaa aagcaggggc cattatacac ctgaacatag 4200
   gagaaggaac acctgtttgt tgtcccctgc ttgaagaagg aattaatcct gaagtctgga 4260
   caacagaagg acaatacaga tgagcaacaa atgcctgtcc tgttcaagtt aaactaaagg 4320
   attatgcctc ctttccctac caaaggcagt acccccttag acccgaggcc caacaaggac 4380
   tccaaaagat tgttaaggac ctaaaagctc aaagcctagc aaaaccatgc agtagcccct 4440
   gcaatactcc aattttagga gtacagaaaa ccaacagaca gtggaggtta gtgcaagatc 4500
   tcaggattat caatgaggct gttgttccta acccttatac tctgctttcc caaataccag 4560
   aagaagcaga gtggtttaca gtcctggacc ttaaggatgg ctttttctgc atccctgtac 4620
   atcctgactc tcaattcttg tttgcctttg gagatccttc gaacccaatg tctcaactca 4680
   gcttgactgt tttaccccaa gggttcaggg atagccccca tctagttggc caagcattag 4740
   ccgagccagt tctcctacct ggacactctt gtcctctggt acatggatga tttattttta 4800
   gctgcccgtt cagaaacctt gtgccatcaa gccacccaag tgctcttaaa tttcctcgcc 4860
   acctgtggct acaaggtttc caaaccaaag gctcagctct gctcacagca ggttaaatac 4920
   ttagggctaa aattatccaa aggcaccagg gccctcagtg aggaatgtat ccagcctgta 4980
   ttggcttatc ctcatcccaa aaccctaaag caactaagag ggttccttgg cataacaggt 5040
   ttctgccaaa tgtggattcc caggtacggt gaaatagcca ggccattata taccctaatt 5100
   aaggaaactc agaaagccaa cacccattta ttaagatgga cacctgaagc agaagcagct 5160
   ttccaggccc taaagaaggc cctaacccaa gccccagtgt taagcttgcc aacggggaag 5220
   acttttcttt atatgtcaca gaaaaaacag gaatagctct aggagtcctt agacaggtcc   5280
   aagggatgag cttgcaacct gtggcatacc tgagtaagga aattgatgta gttgcaaagg   5340
   gttgacctca ttgtttacag gtagtggcgg cagtagcagt cttagtatct gaagcagtta   5400
   aaataataca gggaagagat cttactgtgt ggacatctca tgatgtaaac ggcgtactca   5460
   cttctaaagg agacttgtgg ctgtcagaca accgtttact taaatatcag gctctattac   5520
   ttgaagggcc agtgctgcga ctgcccactt gttcaactct taacccagcc acatttcttt   5580
   cagacaatga agaaaagata gaacataact gtcaacaggt gattgctcaa acctacggcg   5640
   ctcgagggga ccttctagag gttcccttga ctgatcccaa cctcaacttg tatactgatg   5700
   gaagctcctt tgtagaaaaa ggactttgaa aggtggggta tgcagtggtc agtgataatg   5760
   gaatacttga aagtaattcc ttcactccag gaactagtgc tcagctggca gaactaatag   5820
   ccctcactca ggcactagaa ttaggagaag gaaaaagggt aaatatatat gcagactcta   5880
   agtatgctta cccagtcctc cacgcccaca cagcaatatg gagagatagg aaattcctaa   5940
   cttctgaggg aacaccgatc aaacatcagg aagccattag gagattatta ttggctgtac   6000
   agaaacctaa agaggtggca gtcttacact gctggggtca tcagaaagga aaggaaaagg   6060
   aaatagaaag gaaccaccaa gtggatattg aagccaaaag agccacaagg caggccctcc   6120
   attagaaatg cttatagaag gatccctagt atggggtaat cccctccggg aaaccaagcc   6180
   ccagtactca gcaggagaaa tagacacgag gacatagttt cctcccctca ggatggctag   6240
   ccaccgaaaa agggaaaata cttttgcctg cagctaatca atggaaatta cttaaaaccc   6300
   ttcaccaaac ctttcacttg ggcatggata gcatctatca gatggccaat ttattattta   6360
   ctggaccagg ccttttcaaa actatcaagc agatagtcag ggcctgtgaa atgtgccaaa   6420
   gaaataatcc cctgcacttc aagccataca tttcaatccc tgtatcttta acctcctgtt   6480
   gtttgtctct tccagactca aagctgtaaa actgcaaatg gttcctcata tggagcccca   6540
   gatgcagtcc atgactaaga tctaccacag agccctagac cggcctgtta gcccatgctc   6600
   cgatgttgat gacatcaaag gcacaccttc cgaggaaatc tcaactgcac gacccctact   6660
   aagccccaat tcagcaggaa gcagttaaga gcagtcgttg gctaacatcc ccaatagtat   6720
   gtgggttttc ctgttgagag gggggactga gagacaggac tagctggatt tcctaggcca   6780
   actaagaatc cctaagccta gttgggaagg tgaccgcatc cacctttaaa cacggggctt   6840
   gcaacttagc tcacacccga ccaatcaggt agtaaagaga gctcactaaa atgctaatta   6900
   ggcaaaaaca agaggtaaag aaatagccaa tcatctatcg cctgagagca cagtggggag   6960
   ggacaatgat cgggatataa acccaggcat tcgggccggc aacggcaacc cccattgcgt   7020
   cccctcccat tgtatgggag ctctgttttc attctattaa atcttgcaac tgcacactct   7080
   tctggtctat gtttgttatg gctcgagctg agctttcgct cgctgtccac cactgctgtt   7140
   tgccgccatc gcagacccac cactgacttc cacctctgca gatctggcag ggtgtccgct   7200
   gtgctcctga cccagcgagc cacccattgc tgctcccaat caggctaaag gcttgccatt   7260
   gttcctgcat ggctaagagc ccagggttcg tcctaatcga gctgaacgct agtagctggg   7320
   ttccacagtt ctcttccgtg acccacggct cctaatagag ctataacact caccacatgg   7380
   cccaaggttc cattcattgg aatccgtgag gccaagaacc cccggtcaga gaacaagaag   7440
   cttgccacca tcttggaagc tctaaaaaca gagacacccc agtaaca                 7487
<210> 2
   <211> 7774
   <212> DNA
   <213> Homo sapiens
<400> 2
   tgagacacag gactagctgg atttcctagg ccgactaaga atccctaagc ctagctggga     60
   aggtgaccac atccaccttt aaacacgggg tttacaactt agctcacacc cagccaatca    120
   gagagctcac taaaatgcta attaggcaaa aacaggaggt aaagaaatag ccaatcatct    180
   attgcctgag agcacagcgg gagggacaag gattgggata taaacccagg cattcgagct    240
   ggcaacggca accccctttg ggtctcctcc ctttgcatag gagctctgtt ttcactctat    300
   taagtcttgc aactgcactc ttctggtccg tgtttcttac cgcttgagct gagctttccc    360
   tcactgtcca ccactgctgt tttgccaccg tcacaggccc accgctgact tccattcttc    420
   tggatctagc aggctgtcca ctgtgctcct gatccagcga ggcgcccatt gccgctcccg    480
   attgggctaa aggcttgcca ttgttcctgc atggctacgt gcctgggttc atcctaatca    540
   agccgaacac tagtcactgg gttccacggt tctcttccat gacccacgac ttctaataga    600
   actataacac tcacctcatg gcccaagatt ccattccttg gaatccatga ggccaagaac    660
   cccaggtcag agaacacgag gcttgccacc atcttggaag tggccccacc accatcttgg    720
   gagctctggg agcaaggacc cccggtaaca ttttggcgac cacaaaggga catccaaagt    780
   ggtgagtaat attggaccac tttcacttgc tattctgttc tatccttcct tagaactgga    840
   ggaaaatacc aggcacaggc acctgtcagc cagttaaaaa caattagcgt cgccgccaca    900
   cttaagactc aggtgtgagg ctatctgggg aaagactttc taacaacccc caacccatct    960
   agtggggatg ttggtctgcc tggagacagc ttccactttc aattttcttg gggaagccga   1020
   gggctcacta gaggcagaca gctgttgtcc caaactccgg gcagtagccg gttgagatca   1080
   tggtgcagcc aggagtctct actcagcagt cgccgatgca tgtgccccta ccttcccttc   1140
   tgacccatac atcctgagtc ccgactgtga ctttcttgaa agtgtagccc caaaattctc   1200
   cttacctctg aatctacttc ctctgatccc tgcctcctgg gtactaatga ttcagacttt   1260
   catttcctct agcaagttgt gtctccaaag ggatctaagg aggctctacg ctgcatcctt   1320
   aggcacctag gctataaccc aaggagtctt atccctggtg tccctcccga tttgggtata   1380
   caactctcaa catgggcagt tatgtaggac ccattcccca ccacacttgc cagggcccca 1440
   agtttgtaat ggctaagaga gagacacaga gagagagaga gagatggaga gagagacaag 1500
   gagggagtca aagagaaaaa gaaagaaaaa gaaatagtag aaaaaaaagt gtgccctatt 1560
   cctttaaaag ccagggtaaa tttaaaacct gtaattgata attgaaggtc ttctccgtga 1620
   ccctgtaaca ctccaatgcc attttgttgt cagtgtaaat aagggcatag cccaaaagca 1680
   ctgaggtcac tgacaacccg tagctttccc atcaaaaatc cttaacccag taatccgcgg 1740
   atgggccaaa tgcattcagt cggtagcagc aaccgctttg ctaaaagtag aaaagtaact 1800
   tttagaggaa acctcattgt gagcgcacac ctcaccagtt cagaattatt ctaagtcaaa 1860
   aaaaaaaaaa gcaaaaaggt aacttactaa ctcaaaaatc ttaaagtata ggtctatcat 1920
   attagaaaag ggtaatgtaa ctccaaccac tgataattcc cttaacccag cagatttcct 1980
   aacaggggat ttaaaactta attaccatac aaaggtccca ccagacctag gaggaactcc 2040
   cttcaggaca ggacgataaa cggttcctcc caggtgattg aggaaaaaaa ccacaatggg 2100
   tattcagtaa ttgatacaga gactcatgtg gaagcagtta gaaaaattgc ctaataattg 2160
   gtctcctcaa acgtgtaagc tgtttgcact cagccaagcc ttaaagtact tacagaatca 2220
   aaaagactct gaatcctgac tcaaaaggtt tgctacaccc tctgtgaaac aaatttgcat 2280
   aagaactgtt gtttatggga aggcatcttg atggggcagc tgggttgtta tgaaatactc 2340
   aggaccccag cccggctcta ggactcaccc ctgagcgcaa aaggcaatgt tgggcacgct 2400
   ggtaaaggac cactagaatc cagcagcccg gacccctttc tttgtggtca agagaggcgg 2460
   gaaaacaggt gcaggactgc tacatcagtg agcataacta atccagtaag cagaggtcca 2520
   tgggtggtta tgcaccctgg aaaagaatac gcattaggcc cttagaggat gctctaggac 2580
   taatgctcat cggaaaatga ctaggggtgc tgacatccct atgttctttt ttcagatggg 2640
   aaacgttcct cccaccccaa ggcaaaaaac acccctaaga tgtattttgg agaattagga 2700
   ccaatttgac cctcagacac taagaaagaa atgacttaca ttcttctgca gtaccatgat 2760
   atcctcttca agggggagaa acctggcctc ctgagagaag tataaattat aacaccatct 2820
   tacagtgaga cctcttctgt agaaaggagg gcaaatggag tgaagtgcaa actttccttt 2880
   cattaagaga caactcgcaa ttatgtaaaa agtgtgattt atgccctaca gaaagccctc 2940
   agtctacctc cctatcccag ggtccccccg attcctttcc caactaataa ggacccccct 3000
   tttacccaaa tggtccaaag gagatagatg aagggataaa caatgaacca aacagtgcca 3060
   atattccctg attatgcccc ctccaggcag tgggaggagg agaattcggc ccagccagag 3120
   tgcatgtacc tttttttttc tctcagactt aaagcaaatt aaaatagacc taggtaaatt 3180
   ctcagataac cctgatggct atattgatgt tttacaaggg ttaggacaat cctttgctct 3240
   gacatggaga gatataatgt tactgctaaa tcagacacta accccaaatg agagaagtgt 3300
   caccatagct gcagcccaag agtttggcaa tctctggtat ctcagtcagg tcaatgatag 3360
   gatgacaaca gaggaaaggg aatgattccc cacaggccag caggcagttc tcagtgtaga 3420
   ccctcactgg gacacagaat aagaacatgg agatcggtgc cgcagatatt tgctaacttg 3480
   cgtgctagga ctaaggaaaa ctaggaagaa gcctatgaat tattcagtga tgtccactat 3540
   aacacaggga aaggaagaaa atcatactgc ctttccggaa atactaaggg aggcattgag 3600
   gaagcatacc tctctgtcac ctgactgtat tgaagtccaa ctaatcttaa aggatatgtt 3660
   tatcactcag tcagctgcag acattagaaa aaacttcaaa agtccacctt aggcccagag 3720
   caaaacttag aaaccctatt gaacttgtta acctcagttt tttataatag agatcaggag 3780
   gagcaggcgg aacaggacaa acaggattaa aaaaagacca ccgctttagt catggccctc 3840
   aggcaagtgg actttggaag ctctggaaaa gggaaaagct gggcaaattg aatgcctaat 3900
   agggcttgct tccagtgtgg tctacaagga cacttaaaaa aagattgtcc aagtagaaat 3960
   aagctgcccc ttcgtccatg cctcttatgt caagggaatc actggaaggc ccattgcccc 4020
   aggggaggaa ggtcctctga gtcagaagcc actaaccaga tgatccagca gcaggactaa 4080
   gggtgcccag ggcaagcccc agcccatgcc atcaccctca cagagccccg ggtatgcttg 4140
   accattgagg gccaggaggt taactgtctc ctgaacactg gcacagcctt ctcagtctta 4200
   ctttcctgtc ccggacaact gtcctccaga tctgtcacta tctgagcggt cctaggacag 4260
   ccagtcacta gatatttctc ccagccacta agttgtgact ggggaacttt actcttttca 4320
   catgcttttc taattatgcc tgaaagcccc actcctttgt tagggagaga cattctagca 4380
   aaagcagggg ccattataca tctgaacata ggagaaggaa cacccgtttg ttgtcacctg 4440
   cttgaggaag gaattaatgc tgaagtctgg gcaacagaag gacaatatgg atgagcaaag 4500
   aatgcccatc ctgttcaagt taaattaaag gattccgcct cctttcccta ccaaaggcaa 4560
   taccccctta gacccgaggc ccaacaagga ctccaaaaga ttgttaagga cctaaaagcc 4620
   caaggcctag taaaaccatg caatagcccc tgccatactc caattttagg agtaaggaaa 4680
   cccaacggac agtggaggtt agtgcaagaa ctcaggatta tcaatgaggc tgttgttcct 4740
   ctatacccag ctgtacctaa cccttataca gtgctttccc aaataccaga ggaagcagag 4800
   tggtttacag tcctggacct taaggatgcc tttttctgca tccctgtacg tcctgactct 4860
   caattcttgt ttgcctttga agatcctttg aacccaacgt ctcaactcac ctggactgtt 4920
   ttaccccaag ggttcaagga tagcccccat ctatttggcc aggcattagc ccaagacttg 4980
   agccaattct catacctgga cactcttatc cttcggtatg gggatgattt aattttagct 5040
   acccattcag aaacgttgtg ccatcaagcc acccaagtgc tcttaaattt cctcgctacc 5100
   tgtggctaca ggtttccaaa cgaaaggctc agctctgctc acagcaggtt aaatacttag 5160
   ggctaaaatt atccaaaggc accagggccc tcagtgagga acgtatccag cctatactgg 5220
   cttattctca tcccaaaacc ctaaagcaac taagagcatt ccttggcata acaggctgct 5280
   gctgaatatg gattcccagg tacagtgaaa tagccaggcc attatacaca ctaattaagg 5340
   aaactcagaa agccaatacc catttagtaa gatggacacc ttaagcagaa gcggctttcc 5400
   aggccttaaa gaaggcccta acccaagccc cagtggtaag cttgccaaca gggcaagact 5460
   tttctttata tgtcacagaa gaaacaggaa tagctctagg agtccttaca caggtctgag 5520
   ggatgagctt gcaacccatg gcatacctga gtaaggaaac tgatgtagtg gcaaagggtt 5580
   ggcctcattg tttacgggta gtggcagcag tagcagtctt agtatctgaa gtagttaaaa 5640
   taatacaggg aagagatctt actgtgtgaa catctcatga tgtgaatggc atagtcactg 5700
   ctaaaggaga cttgtggctg tcagacaact gtttacttaa ataccaggct ctattacttg 5760
   aagggccagt gctgcgactg tgcacttgtg caactcttaa cccagacaca tttcttccag 5820
   acaatgaaga aaagatagaa cataactgcc aacaagtaat tgctcaaacc tatgccactc 5880
   gaggggacct tttagaggtt cccttgactg atcccaacct caacttgtat actgatggaa 5940
   gttcctctgt agaaaaagga ctttgaaaag tggggtatgc agtggtcagt gataatggaa 6000
   tacttgaaag taatcccctc actccaggaa ctagtgctca gctggcagaa ctaatagccc 6060
   tcactcgggc actagaatta ggagaagaga aaagggtaaa tatatacaga ctctaagtat 6120
   gcttacctag tcctccatgc ccatgcagca atatggagag aaagggaatt cctaatttcc 6180
   aagggaacac ctatccaaca tcaggaagcc attaggagat tactattggc tgtacagaaa 6240
   cataaagagg tggcaatctt acactgccgg tgtcaccaga aaggaaagga aagggaaata 6300
   gaaaggaacc accaagcgga tattgaagcc aaaagagccg caaggcagga ccctccatta 6360
   gaaatgctta tagaaggacc cctagtatgg ggtaatcccc tccaggaaac caagccccag 6420
   tactcagaag aagaaataga atgaggaacc tcacaagcac atagtttcct cccctcagga 6480
   tggctagcca ctgaagaagg aaaaatactt ttgcctgcag ctaaccaatg gaaattactt 6540
   aaaacccttc accaaacatt tcccttaggc attgatagca cccatcagat ggccaaatta 6600
   ttatttactg gaccaggcct tttcaaaact atcaagcaga tagtcagggc ctgtaaagtg 6660
   tgccaaacaa gtaatcccct gcactgcagg ccatacattt caatccctgt atctttaacc 6720
   tccttgttaa gtttgtctct tccagaatca aagctgtaaa actacaaata gttcttcaaa 6780
   tggagcccca gatgtagtcc atgactaaga tctaccgcgg acccctggac aagcctgcta 6840
   gcccatgctc tgatgttaat gacatggaag gcacccctcc cgaggaaatc gcaactgcac 6900
   aacccctatt acaccccaat tcagcaggaa gcagttagag cattcatcag ccaacctccc 6960
   caacagcact tgggttttcc tattgagagg gggtactgag agacaggact agctggatgt 7020
   cctaggctga ctaagaatcc ctaagcctag ctgggaaggt gaccacatcc acctttaaat 7080
   acggggcttg caacctagct cacacccaac agatcagaga gctcgttaaa atgctaatta 7140
   ggcaaaaaca ggaggtaaag aaatagccaa tcatctattg cctgagagca cagcaggagg 7200
   gacaaggatt gggatataat cccaggcatt cgagctggca acagcaaccc cctttgggtc 7260
   ccctcccttt gtatgggagc tgttttcact ctatttcact ctattaaatc ttgcaactgc   7320
   actcttctgg tgcatgtttg ttactgcttg agctgaactt tcactcgcca tctaccactg   7380
   ctgttttgcc gccgtcgcag acccactgct gacttccatt cttctggatc cagcagggtg   7440
   tccactgtgc tcctgatcca gtgaggcacc cattgccgct cccgatctgg ctaaaggctt   7500
   gccattgttc ctgcatcgct aagtgcctgg gttcgtccta atcaagctga acactagtca   7560
   ctgggttcca cagttctctt ccatgaccca cgacttctaa tagagctata acactcacct   7620
   tatggcccaa gattccattc cttggaatcc atgaggccaa aaaccccagg tcagagaaca   7680
   tgagacttgc caccatgttg aagtggcctg ctgccatttt ggaagtggcc caccaccatc   7740
   ttgggagctc tgggagcaag gacccctggt aaca                               7774
<210> 3
   <211> 5470
   <212> DNA
   <213> Homo sapiens
<400> 3
   tgagagacag ctggatttcc taggccgact aagaatccct aagcctagct gggaaggtga     60
   ccgcatccac ctttaaacac agggcttgca acttagctca cacccaacca atcagagagc    120
   tcactaaaat gctaattagg caaaaacagg aggtaaagaa atagcaagtc atctattgcc    180
   tgagagcaca gtgggaggga caaggaccag gatataaacc caggcatttg agccagcaac    240
   ggcaacctcc tttgagtccc ctccctttgt ataggagctc tgttttcact gtgtttcact    300
   ctattaaatc ttgcaattgc actcttctgg tccatatttg tcacggcttg agctgagctt    360
   tcacttgccg tccaccacta ctgtttgctg ctgtcacaga cccgccgctg actcccatcc    420
   cgctgctgac tcccatccct ccggatccgg cagggtgtcc gctgtgctcc tgatccagca    480
   agactcccat tgccactccc gatagtgcta aaggcttgcc attgttcctg catggctaag    540
   tgcctgggtt cgtcctaatc cagctgaaca ctagtcactg ggttccacgg ttctcttcca    600
   tgacccgcgg cttctaatag agctataaca ctcaccacat ggcccaatat tccattcctt    660
   ggaatccgtg aggccaagaa ccccaggtca gagaacacga ggcttgccac catcttggaa    720
   gcagcctgcc accatcttgg aagtggctca ccaccgtctt gggagttctg tgaacaagga    780
   cccctggtaa cattttggcg accacgaagg gacatccaaa gctgtgagta atattggacc    840
   actttcgctt gctattctgt tctatcctta gaactggagg aaaatactgg gcacctgtcg    900
   ccagttaaaa atgattagca tggccgccgg acttaagact caggtgtgag gctatctggg    960
   aaagggcttt ctaacaaccc ccaagccttc tgttgggaac tttggtctgc ctggagccag   1020
   cttccacttt caattttctt ggggaagcca agggctgact ggaggcagaa agctgttgtc   1080
   ccgaactccc ggcagtagcc ggttgagatc atggcgcagc cagaagtctc tactcggcag   1140
   tcgcccatgc gtgcgccctt acctttcctt ctgaattata cctccggggt cccgactccg   1200
   actttcttga gagtttagcc ccaaaattct ccttacctct gaatctactt cctttgatcc 1260
   ctgcctcctg cctcctaggt actaatagtt cagactttca tttcctctag caagttgtgt 1320
   ctccaaaggg atctaaggag gctctatgct gtgtccttag gcacctaggc tataacccag 1380
   ggagtcttat ccctggtatc cctcccgatt taggtataca gctcttgaca tgggcagtta 1440
   tgtgggacct gttccccacc acccttgtga gggccccaag tttgtaatgg ctaagaaaga 1500
   gagacggaga gagagagaga cggagaaaga gacaaagagg gagtcaaaga gaaaaagaaa 1560
   gaaaaagata gaaatagtta aaaaaaaaaa aaagtgtgcc ctattccttt aaaagccagg 1620
   gtaaatttaa aacctgtaat tgataattgc cactttgttg tcagtgtaaa taagggcgta 1680
   gcaaatcctt aacccagtaa cccgcggata ggccaaatgc attcagtcgg tagcggcaac 1740
   agctttgcta aaagtagaaa agtaactttt agaggaaacc tcattgtgag cacacctcac 1800
   cagttcagag ttattctaag taaaaaaaaa aaaaaaaaaa aaagcaaaaa ggtagcttac 1860
   taactcaata atcttaaagt atggggctac tatgctagaa aagggtaatg taactccaac 1920
   cactgataac tcccttaacc cagcagattt cctaacaggg gatttaaatc ttaattacca 1980
   cacgaaggtc cgaccagacc taggaggaac tcccttcagc acaggacgat agatggttcc 2040
   tcccaggtga ctgaggaaaa aactacaatg ggtattcagt aattggtatg gagactcttg 2100
   tggaagcaga gttaaaaatt tgcctaataa ttggtctcct caaatgtgcg agctgtttgc 2160
   actcagccaa gccttaaagt acttacagaa tcaaaagact atctcaatcc tgactcaaaa 2220
   ggttagctac acagtctctg aaatgaattt gcagaagaac tgttgtttat gggaatgcat 2280
   cttgatgggg cagctgggtt gttatgaaat actcaggaac ccagcccagc tctaggactc 2340
   accgctgagc gcaaaggcaa tgttgggcac gctggtaaag gaccactaga atccagcagc 2400
   ccaggcccct ttctttgtgg tcaagaaagg caggaaaagg agtgcagaac tgctacattg 2460
   gtgagcgtaa ctaatccaat aagcagaggt ccatgagtgg ttatgcacgc tggaaaagaa 2520
   taagcattag gcccttagag gatgctctag gactaatgct catcggaaaa tgactagggg 2580
   tgctggcatc cttatgttct ttcttcagat gggaaacgtt ccccccaagg caaaagcgcc 2640
   cctaagatgt attctggaga attagaacca atttgaccct cagatgtcaa gaaagaaacg 2700
   acttatattc ttctgcagta ctgcctggcc acgatatcct cttcaagggg gagaaacctg 2760
   gcctcctgag ggaagtacaa attataacac catcttacag ctagacctct tttgtagaaa 2820
   agaaggcaaa tggagtgaag tgccatatgt gcaaactttc ttttcattaa gagacaactc 2880
   acaattatgt aaaaagtgtg gtttatgtct tacaggaagc cctcagagtc tacctcccta 2940
   tcccagcatt cccccgactc cttccccaac taataagcac cacccttgaa cccaaacagt 3000
   ccaaaaggag atagacaaac aggtaaacaa tgaaccaaag agtgtcagta ttccccgatt 3060
   atgccccttc caagcagtgg gaggaggaga attcggccca gccagagtgc atgtaccttt 3120
   ttctctctca gacttaacgc aaattaaaat agacttaggt aaattctcag ataaccctga   3180
   tggctacatt gatgttttac aagggttagg gcaatccttt gatctgacat ggagagatat   3240
   aatgttactg ctaaatcaga cactaacccc aaatgagaga agtgccgccg taactgcagc   3300
   ccgagagttt ggtgatctct ggtatctcag tcaggtcaat gataggatga caacagagaa   3360
   aagagaacga ttccccacag gccagcaggc agtttccagt gtagaccctc attaggacac   3420
   agaatcagaa catggagatt ggtgccacag atatttgcta acttgagtgc tagaaggact   3480
   aaggaaaact aggaagaagc ctatgaatta ttcagtgatg tccactataa cacaaggaaa   3540
   ggaagaaaat cctactgcct ttctggagag agtaagggag gcattaagga agcatacctc   3600
   cctgtcacct gactctattg aaggccaact aatcttaaag gataagtttg tcactcagtt   3660
   agctgcagac attagaaaaa aacttcaaaa gtccgactta ggcctggagt acggctgagt   3720
   gcccaatttg gcagcaggca agaccaacac tgagcccttc atatggcacc atgctttgtg   3780
   gtgatcagcc aactacttga tggcaggttg attatattgg acatctttca tcagagaaat   3840
   ggcagtggtt tgtccttcct ggaatagaca cttattctcg atatgggttt gtctatcctg   3900
   caggcaatgc ttctgccagg agtaccatct gtggactcat ggaaagcctt atccaccatc   3960
   atggcattcc acacagcatt gcctctaaac aaggcactta ttttatagct aaggaagtgt   4020
   ggcagtgggc tcatgctcat ggaattcact gattgtatct tgttgcccat tatcttaaag   4080
   cagctggatt gatagaacag tggaaaggcc atttgaaatc acaattacac caccaactag   4140
   gtgacaatac tttgcagggc tcggcaaagt tctcttgaag gctgagtatg tcctgaatca   4200
   gcatccaata tatggtactg tttccctcat agccagcatt cacaggccta agaatcaagg   4260
   ggtagaagta gaagtggcac cactcaccat cactcctagt gacccactag caaaaatttt   4320
   acttccagtt cccccaacat tatgttctgc tggccttagt tccagaggga agaattctgc   4380
   caccagtcga cacaagaatg ataccattaa actgaaagtt aaaattgcca cctggccact   4440
   ttgggctcct cccacctcta agtcaacagg tcaagaaagg agttacagtg ttgacttggg   4500
   tgattgacct ggactatcaa gatgaaatca ggttactact ccacagtgga ggtaaggaag   4560
   aatatgtgtg gaatacagga gatcccttag gccgtctttt agtactacca tgccctgtga   4620
   ttaaggtcag tggaaaacta caacaatcca atctaggcag gactacaaat ggcccagact   4680
   cttcaggaat gaagggttgg gtgacttcac caggtaaaaa aataacagcc tgctgaggtg   4740
   ctagctgaag gcaaagggaa tacagaatgg ttagtagaaa aaggtagtca tcaataccag   4800
   ctatgaccac aagaccagtt gcagaaatga gacctgtaat tgtcatgtgg atttcctcct   4860
   tacatgtttg tgcatgtata cacttctact aagaaaatac ctttatttat ttcctttgct   4920
   tttcccttat caagtgacat tattaacttc atatcagcag ttaagtgtta ttaactttat   4980
   gtaatagcat ttcggttaat aattcacttc tggttgtatg aaggatagcc gtattaagtt   5040
   aggtgtaatt atgacatcat tattgtcttt atttgaagat tatgtgtaat ttcaggagat   5100
   gtgtatgggt tcaagttgac aagggatgga cttgtgatgg ctaatgttga gtgtcaactt 5160
   gactgaggat gcaaagtatt gttcctgggt gtgtctgtga gggtgttgcc aaaggagatt 5220
   aacatttgtg tcagtgaact gggagatgca gacccacccg caatctgggt gagcaccatg 5280
   taatcagctg ccagagcagc tagaataaag caagcagaag aaggtggaag gagctgactt 5340
   gctgagtctt ctagtattct tcgttcttct atgctggttg cttcctgccc ccaaacatca 5400
   gtctgcaagt tcttctgctt ttggactctt ggacttacac cagtggtttg ccagggactc 5460
   tcgggccttc 5470
<210> 4
   <211> 783
   <212> DNA
   <213> Homo sapiens
<400> 4
   tgagagacag gactaactgg atttcctagg ccgactaaga atccctaagc ctagctggga 60
   aggtgaccgc atccatcttt aaacacgggg cttgaaactt agctcacacc taaccagtca 120
   gagagctcac taaaatgcta attaggcaaa aaacaggagg taaagaaata gccaatcatc 180
   tattgcctga gagcacagcg ggagggacaa ggatcgggat ataaacccag gcattcgagc 240
   cagcaatggc aacccccttt gggtcccctt cccttgtatg ggagctctgt tttcactcta 300
   tttcactcta ttaaatcttg caactgcact cttctggtcc atgtttgtta cggctcgagc 360
   tgagctttgg ctcgccatcc accactgctg tttgccgccg tcgcacacct gctgctgact 420
   cccatccctc cggatccagc agggtgtgtc cgctgtgctc ctgatccagc gaggtgccca 480
   ttgccgctcc tgattggact aaaggcttgc cattgttcct gcacggctaa gtgcccgggt 540
   tcgtcctaat ccagctgaac actagtcact gggttccacg gttctcttcc ttgacccacg 600
   gcttctaata gagctataac actcaccgca tggcccaaga ttccattcct tggaatctgt 660
   gaggccaaga accccaggtc agagaacacg aggcttgcca ccatcttgga agcggcctgc 720
   caacatcttg gaagtggctc gccaccatct tgggagctct gtgagcaagg acccctggta 780
   aca 783
<210> 5
   <211> 7542
   <212> DNA
   <213> Homo sapiens
<400> 5
   tgagagacag gactagctgg atttcctagg ccgactaaga atccctaagc ctagctggga     60
   aggtgaccgc ttccaccttt aaacacgggg cttgcaactt agctcacacc cgaccaatca    120
   gatagtaaag agagcacact aaaatgctaa ttaggcaaaa acaggaggta aagaaatagc    180
   caatcatcta ttgcctgaga gcaaagcggg agggacaatg atcgggatag aaacccaggc    240
   attcaagccg gaatggctac cctctttggg tcccctccct ttgtatggga gctctgtttt    300
   cactctattc aatcttgcaa ctgcactctt ctggtccgtg tttgttacag cttgagctga    360
   gctttcgctc gccttccacc actgctgttt gccgccatcg cagacctgcc gtgctgactt    420
   ccatccctct agatctggca gggtgtccgc tgtgctcttg atccagcgag gcgcccattg    480
   ccgctcccga ttgggctaaa ggcttgcaat tgttcctgca cgctaagtgc ctgggttcat    540
   cctcatcaag ctgggttcca cggttctctt catgacccgc agcttctaac agagctataa    600
   aactctgtgc atggcccaag attccattcc ttggaatctg tgaggccaag aaccccaggt    660
   cagagaacag gaggcttgcc accatcttgg aagtggctcg ccaccatctt aggagctctg    720
   tgagcggaga cccccacccc ccggtaacat tttggcgacc acgaagggac ctccaaagcg    780
   gtgagtaata ttggatcact ttcgcttgct attctgtcct atccttcttt agaattggag    840
   gaaaatactg ggcacctgtc ggccagttaa aaacaattag cgtggctgcc cgacttaaga    900
   ctcaggtgtg aggctatctg gggaagggct ttctaacaac ccccaaccct tctgggttgg    960
   ggacgttggt ctgccccttc cactttcaat tttcttgggg aagccaaggg tcgactagag   1020
   gcagaaagct gtcgtccgga actcctggca gtagccggtt gagatcatgg cgcagccaga   1080
   agtctctact caacagtcgc ccatgcgtgc gctcctacct ttcctcctga cccatacctc   1140
   ctgggtcccg acgatgactt tcttgaaagt gtagccccaa aattctgctt acctctgaat   1200
   ctacttcccc tgatccctgg ctcctaggta ctaatggttc agtttcattt cctctagcaa   1260
   gttgtatctc caaagggatc taaggaagct ctacgctgcg tccttaggca tctaggctat   1320
   aaacccagga agtcttgtcc ctggtgtccc tcccgattta ggcatacagc tctcgacatg   1380
   ggcagttatg tgggacccgt tccccatcac ccttgtcaag gccccaagtt tgtaatggct   1440
   aagaggagag agagagaaag agagagagac ggaggggaga gagagagaga gagatggagg   1500
   ggagagagag agagagagac ggaggggaga gagagagaga gagagagacg gaggggagag   1560
   agagagagac ggaggggaga gagagagaga tggaggagag aaagacaaag ggagtcaaag   1620
   agaaaaagaa agagaaagac agaaatggta aaacaaacaa aaaacagcgt gccctattcc   1680
   tttaaaagcc ggggtaaatt taaaacctat aattgataat tgaaggtctt ctccatgacc   1740
   ctataatact ccaatactac cttgttgtca gtgtaaacaa gggcgtagcc tgaaaacact   1800
   gagaccactg acaacctgca gctttcctat caaaaaatcc ttaacccagt aaccggcaga   1860
   tgcattcaat ctgtagcagc aactgttttg ctaacagaag aaagtagaaa agtaactttt   1920
   agaggaaacc tcattgtgag cacaccttac cagttcagaa ttattctaag tcaaaaaagc   1980
   aaaaaggtag cttactaact caaaaatctt aaagtatggg gctattgtgt ttaaaaaaaa   2040
   aaaaaggtaa tttaacacca accactgata attctcttaa cccagcaggt ttcctaacag   2100
   gggatttaaa tcttaattac catacaaagg tctgaccaca cctaggagga actcccttca   2160
   ggacaggact atagagggtt cctcccaggt gattgaggaa aaaaccacag tgggtattca   2220
   gtaattgata gggagactct tgtggaagca gagttagaaa aattgcctaa taaatggtgt 2280
   cctcaaaagt gtgagctgtt tgcactcagc caagccttaa agtacttaca gaatcgtaaa 2340
   aactatctca atcctgactc aaaagtttac ttacaccctc tctgaaatga atttacataa 2400
   gaactgcttt tttgggaatg catcttgatg gggcagctgg gtggttatga aatactcagg 2460
   aaaccagccc agctctagga cacatccctg agcacaaagg caatgttggg cacgctggta 2520
   aaggaccact agaatccagc agcctggact cctttctttg tggtcaagaa aggcaggaaa 2580
   acaggtgcag gactgctaca tcagtgagca taactaatct gataagcaga gggccttggg 2640
   tggttacaca ccctggaaag gaattcaact ctgagcgcaa aggcaatgtt gggcacattg 2700
   gtaaaggacc actagaatcc agcagcccag gcccctttct ttatggtcaa gaaaggcggg 2760
   aaaaggggtg caggactgtt acctcggtga gcgtaactaa tccgataagc agaggtccat 2820
   gggtgattac gcaccctgaa aagaataagc attaggccct taaaggatgc tctaggacta 2880
   atgctcattg gaaaatgact aggggtgctg gcatccctat gttcttttct cagacgggaa 2940
   atgttctcca ccctccccaa ggcaaaaaca cccctaagat gtattctgga gaattgggac 3000
   caatttgacc cccagacgct aagaaagaga tgacttatgt tcttctgcag taccacctgg 3060
   ccacgatatc ctcttcaagg gggagaaacc tggcctcctg agggaagtat aaattataac 3120
   accatcttac agctagacct cttctgtaga aaggagggca aatggagtga agtgccatat 3180
   gtgcaaactt tcttttcatt aagagacaac ttgcaattat gtaagaagtg tgatttatgc 3240
   cctacaggaa gccctcagag tctacctccc taccccagca tccccctgac tccttctcca 3300
   actaataagg aacccccttc aacccaaacg gtccaaaagg agatagacaa aggggtaaac 3360
   aatgaaccaa agcgtgccaa tgttccctga ttatgccccc tctaagcagt gggaggagga 3420
   gaatttggcc cagccagtgt gcatgtgcct ttttctctct cagacttaaa gcaaattaaa 3480
   atagacctag gtaaattctc agataaccct gatggctata ttgatgtttt ataagggtta 3540
   ggataatcct ttgatctgac atggagagat ataatgttac tgctagatca gacactaacc 3600
   ccaaatgaga caagtgccgc cataactgca gcctgagagt ttggcgatct ctggtatctc 3660
   actcgggtca atgataggag gacaacagag gaaagagaat gattccccac agaccagcag 3720
   gcagttccca gtgtagaccc tcactgggac acagaatcag aacatggaca ttggtgctgc 3780
   agacatttgc taacttacat gctagaagga ctaaggaaaa ctaggaagaa gcctacgaat 3840
   tattcaatga tgtccactat aacacaggga aaggaagaaa atcctactgc ctttctggag 3900
   cgactaaggg aggcattgag gaagcatact tccctgtcac ctgactctat tgaaggccaa 3960
   ctaatcttaa aggataagtt tatcactcag tcagctgaag acattaggaa aaaacttcaa 4020
   aagtctgcct taggcccaga gcaaaactta gaaaccccat tgaacttggc aacctcggtt 4080
   ttttataata gagatcagga ggagcaggcg gaacaggaca aacggggtaa aaaaaaggcc 4140
   accgctttag ttatggccct caggcaagtg gactttggag gctctggaaa agggaaaagc   4200
   tgggcaaatc gaatgcctac tagggcttgc ttccagagtg gtctacaagg acactttgaa   4260
   aaagattgtc caagtagaaa taagtcgccc cttcgtccat gccccttata tcaagggaat   4320
   cactggaagg cccactatcc caggggacaa atgtcctctg agtcagaagc cactaaccag   4380
   atgatccagc agcaggactg agggtgccca gggcaagcac tagcccatgc cgtcaccctc   4440
   acagagcccc aggtatgctt gaccattgag ggccaggagg ttaactgtct cctggacact   4500
   agcacggcct tctcagtctt actctccttt cccggacaac tgtcctccag atctgtcact   4560
   atccgagggt tcctaggaca gtcagtcact agatacttat cccagtcact aagttgtgac   4620
   tggtgaactt tactcttttc acatgctttt ctaattatcc ctgaaagcac cactcccttg   4680
   ttagggcgag acattctagc aaaagcaggg gccattatac acctgaacat aggagaagga   4740
   acacctgttt gttgtcccct gcttgaggaa ggaattaatc ccgaagtctg ggcaacagaa   4800
   ggacaatacg gacgagcaaa gaatgcctgt gctgttcaag ttaaactaaa ggattccgcc   4860
   tcctttccct accaaaggca gtaccccctt agacctgagg cccaacaagg actccaaaag   4920
   attgttaagg acctaaaagc ccatggccta gtaaaaccat gcaatagccc ctgcaatact   4980
   ccaattttag gagtacagaa acccaacaga cagtggaggt tagtgcaaga tctcaggatt   5040
   atcattgagg ctgttgttcc tgtatagcca gctgtaccta acccttatac tctgctttcc   5100
   caaataccac aggaagcaga ggggtttaca gtccggggcc ttaaggacac ctttttctgc   5160
   atccctgtat atcctgactc tcaattcttg tttgcctttg aagatccttc aaactcaacg   5220
   tctcaactca cctggaatgt tttaccccaa gggttcaggg atagccccca ttagcccaag   5280
   acttgagcca gttcttatac ctggacactc ttgtcctttg gtacgtggat gatttacttt   5340
   tagccacctg ttcagaaacc ttgtgccatc aagccaccca agcactcttt aatttcctcg   5400
   ccacctgtgg ctacaggttt ccaaaccaaa ggctcagctc tgctcacagc aatttaaatg   5460
   cttagggcta aaattatcca aaggcaccag ggccctcagt gaggaaagta tccggcctat   5520
   actggcttat cctcatccca aaaccctaaa gcaactaaga gtgttccttg gcataacggg   5580
   tttctgccga atatggattc ccaggtacag cgaaatagcc agaccattat atacactaat   5640
   taaggaaact cagaaagcca atacccattt ggtaagatgg acacctgaag cagaagcaga   5700
   tttccaggcc ctaaagaagg ccctgaccca agccccagtg ttaagcttgc caatggggca   5760
   agacttttct ttatatgtca cagaaaaaac aggaatagct ccaggagtcc ttacgcagat   5820
   ccaagggacg agcctgcaac ccatggcata cctgagtaag gaaattagtg gcaaagggtt   5880
   ggcctcattg tttatgggta gtggcagcag tcacagtctt agtaactgaa gcagttaaaa   5940
   tgatacaagg aagagatctt actgtgtgga catctcatga tgtgaatggc atactcactg   6000
   ctaaaggaga cttgtgactg tcagacaact gtttacttaa atatcaggct ctattacttg   6060
   aagggccagt gttgcgactg tgcacttgtg caactcttaa cccagccaca ttgcttccag   6120
   acaatgaaga aaagatagaa cataactgtc aacaaataat tgctcaaacc tacactgctc 6180
   gaggggacct tttagaagtt cccttgactg atcccgatct caacttgtat actgatggaa 6240
   gttcctttgc agaaaaagga cttcaaaagg cggtgtatgc agtagtcctt caaaatcgaa 6300
   gagctttaga attgctaatc actgagagag ggggaacgtt tttattttta ggggaagaat 6360
   gctgttatta tgttaatcaa ttcggaatca tcaccaagaa agttaaagaa attcaagatc 6420
   gaatacaacg tagaacagag gagcttaaaa aacactggac cctggggcct cctcagccaa 6480
   tggatgccct ggattctccc cttcttagga cctctagcag ctatatttct actcctcttt 6540
   ggaccctgta tctttaacct ccgtgttaag tttgtctctt ccagaatcga agatgtaaaa 6600
   ctacaaatcg ttcttcaaat ggacccccag atgcagtcca tgactaagat ctactgagga 6660
   cccctggacc agcctgctag cccatgctcc aatgttaatg acattgaagg cacccctccc 6720
   aaggaaatct caactgcaca acccctacta tgctccaatt cagcaggaag cagttacagt 6780
   ggtcctcggc caacctcccc aacagcattt gtattttcct gttgggaggg ggcactgaga 6840
   gacaggacta gctggatttc ctaggctgac tgagaatccc taagcctagc tgggaaggtg 6900
   accacttcca cctttaaaca cagggcttgc aacttagctc acaccctacc aattggatag 6960
   taaagagagg tcactaaaat gctaattagg caaaaacagg aggtaaagaa atagccaatc 7020
   atccattgcc tgagagcaca gcgggaggga caatgaccag gatataaacc caggcattcc 7080
   agcctgcaac ggcaaccccc tttgggtccc ctctctttgt atgggagctc tgttttcact 7140
   ctattcaatc ttgcaactgc actcttctgg tccgtgtttg ttacggctca agctgagctt 7200
   ttgctcacca tccaccactg ctgtttgccg ccgttgcaga cccgtcgctg acttccatcc 7260
   ctccagatct ggcagggtgt ccactgtgct cctgatccag cgaggcaccc attgccactc 7320
   ccgatcaggc taaaggcttg ccattgttcc tgcacagcta agtgcctggg ttcgtcctaa 7380
   tcaagctgaa cactagtcac tgggttccat ggttctcttc catgacccat ggcttctaat 7440
   agagctataa cactcaccgc atggcccaag attccattcc ttggaatccg tgaggccaag 7500
   aaccccaggt cagagaacac gaggctgccg ccatcttgga ag 7542
<210> 6
   <211> 10288
   <212> DNA
   <213> Homo sapiens
<400> 6
   cctggggcgg gcttcctttc tgggatgagg gcaaaacgcc tggagataca gcaattatct      60
   tgcaactgag agacaggact agctggattt cctaggccga ctaagaatcc ctaagcctag     120
   ctgggaaggt gaccacgtcc acctttaaac acggggcttg caacttagct cacacctgac     180
   caatcagaga gctcactaaa atgctaatta ggcaaagaca ggaggtaaag aaatagccaa     240
   tcatctattg cctgagagca cagcaggagg gacaacaatc gggatataaa cccaggcatt     300
   cgagctggca acagcagccc ccctttgggt cccttccctt tgtatgggag ctgttttcat 360
   gctatttcac tctattaaat cttgcaactg cactcttctg gtccatgttt cttacggctc 420
   gagctgagct tttgctcacc gtccaccact gctgtttgcc accaccgcag acctgccgct 480
   gactcccatc cctctggatc ctgcagggtg tccgctgtgc tcctgatcca gcgaggcgcc 540
   cattgccgct cccaattggg ctaaaggctt gccattgttc ctgcacggct aagtgcctgg 600
   gtttgttcta attgagctga acactagtca ctgggttcca tggttctctt ctgtgaccca 660
   cggcttctaa tagaactata acacttacca catggcccaa gattccattc cttggaatcc 720
   gtgaggccaa gaactccagg tcagagaata cgaggcttgc caccatcttg gaagcggcct 780
   gctaccatct tggaagtggt tcaccaccat cttgggagct ctgtgagcaa ggaccccccg 840
   gtaacatttt ggcaaccacg aacggacatc caaagtggtg agtaatattg gaccactttc 900
   acttgctatt ctgtcctatc cttccttaga attggaggaa aataccgggc acttgtcggc 960
   cagttaaaaa cgattagtgt ggccaccgga cttaagactc aggtgtgagg ctatctgggg 1020
   aagggctttc taacaacccc caacccttct gggttgggga cttggtttgc ctcaagccag 1080
   cttccacttt cagttttctt ggggaagccg agggccgact agaggcagaa agctgtcgtc 1140
   ctgaactccc ggcagtagcc ggttgagatc atggtgtagc cagaagtctc aacagtcgcc 1200
   catgcatgca cccctatctt tccttctgac ccatacctcc tgggtcccaa ccacaacttt 1260
   cttcaaagtg tagccccaaa attctcctta cctctgaata tacttcctct gatccctgcc 1320
   tcctaggtac tattggttca gacttccatt tcctctagca agttgtatct ccaaagggat 1380
   ctaaggaagc tctgcgctgc gtccttaggc acctaggcta taacccaggg agtcttatcc 1440
   ctggtgtccc tcccaattta ggcatacagc tcttgacatg ggcagttatg taggacccac 1500
   tccccaccac ccttgccagg gccccaagtt tgtaaatggc tgagggaaaa gagagacaga 1560
   ggagagagag agaaatggag gagaaagaga gagagacaga gaggagagag agacagtgag 1620
   agagacagaa gagagagaga gacaaagagg agagagagag agtcaaagag agaaagaaag 1680
   agaaagaaat agtaaaaaac agtgtgccct attcctttaa aagccagggt aaatttaaaa 1740
   cctgtacttg ataattgaag gtcttctctg tgaccctata gcactccaat ccactttgtg 1800
   gtcagtgtaa ataagagcat aggccgaaag cactgaggcc attgacaacc cgtagcttcc 1860
   ctatcaaaaa tccttaaccc agtaacccgc agatggacca aatgcattca gtcggtagcg 1920
   caactgcttt gctaaaagta gaaaagtaac ttttagagga aacctcattg tgagcacacc 1980
   tcacctgttc agaattattc taataaaaaa agcaaaaagg tagcttacta actcaaaaat 2040
   cttaaagtat ggggctattc tgttagaaaa aggtaatgta actccaacca ctgataattc 2100
   ccttaaccca gcagatttcc taacgggatt taaatcttaa ttaccataca aaggtccgac 2160
   cagacctagg cggaactccc ttcaggacag gacgatagat ggttcctccc aggtgattga 2220
   ggaaaaaaac cacaatgggt attcagtaat tgatacgggg actcttgtgg aagcagagtt 2280
   agaaaaattg cctaataact ggtctcctca aacgtgtgag ctgtttgcac tcagccaagc 2340
   cttaaagtac ttacagaatc aaaagactat ctcaatcctg attcaaaagg ttagctacac 2400
   cctctctgta atgcatttgc ataagaactt gtttatggga atgcatcttg atggggcagc 2460
   tgggttgtta taaaatagga acccagccca gctctaggac tcacccctga gcgcaaaggc 2520
   aatgttgggc atgctggtaa aggaccacta gaatccagca gcccagaccc ctttctttgt 2580
   ggtcaagaaa ggcgggaaaa ggggtgcagg actgctacat cggtaagcat aactaatccg 2640
   ataaacagag gtccatgggt ggttacgcac cctggaaagg aactcacccc tgagcacaaa 2700
   ggcaatgttg ggcacgctgg taaaggacca ctagaatcca gcagcctgga cccctttctt 2760
   tgtggtcaag agaggcagga aaacaggtgc aggactgcaa catcagtgag cataactaat 2820
   tcgataagca gaggtccatg ggtggtgatg caccctggaa agaataagca ttaggaccat 2880
   agaggacact ccaggactaa agctcatcgg aaaatgacta gggttgctgg catccctatg 2940
   ttcttttttc agatgggaaa cgttccccgc aagacaaaaa cgcccctaag acgtattctg 3000
   gagaattggg accaatttga ccctcagaca ctaagaaaga aacgacttat attcttctgc 3060
   agtgccgcct ggcactcctg agggaagtat aaattataac accatcttac agctagacct 3120
   cttttgtaga aaaggcaaat ggagtgaagt gccataagta caaactttct tttcattaag 3180
   agacaactca caattatgta aaaagtgtga tttatgccct acaggaagcc ttcagagtct 3240
   acctccctat cccagcatcc ccgactcctt ccccaactaa taaggacccc ccttcaaccc 3300
   aaatggtcca aaaggagata gacaaaaggg taaacagtga accaaagagt gccaatattc 3360
   cccaattatg acccctccaa gcagtgggag gaagagaatt cggcccagcc agagtgcatg 3420
   tgcctttttc tctcccagac ttaaagcaaa taaaaacaga cttaggtaaa ttctcagata 3480
   accctgatgg ctatattgat gttttacaag ggttaggaca attctttgat ctgacatgga 3540
   gagatataat gtcactgcta aatcagacac taaccccaaa tgagagaagt gccaccataa 3600
   ctgcagcctg agagtttggc gatctctggt atctcagtca ggtcaatgat aggatgacaa 3660
   cagaggaaag agaatgattc cccacaggcc agcaggcagt tcccagtcta gaccctcatt 3720
   gggacacaga atcagaacat ggagattggt gctgcagaca tttgctaact tgtgtgctag 3780
   aaggactaag gaaaactagg aagaagtcta tgaattactc aatgatgtcc accataacac 3840
   agggaaggga agaaaatcct actgcctttc tggagagact aagggaggca ttgaggaagc 3900
   gtgcctctct gtcacctgac tcttctgaag gccaactaat cttaaagcgt aagtttatca 3960
   ctcagtcagc tgcagacatt agaaaaaaac ttcaaaagtc tgccgtaggc ccggagcaaa 4020
   acttagaaac cctattgaac ttggcaacct cggtttttta taatagagat caggaggagc 4080
   aggcggaaca ggacaaacgg gattaaaaaa aaggccaccg ctttagtcat gaccctcagg 4140
   caagtggact ttggaggctc tggaaaaggg aaaagctggg caaattgaat gcctaatagg 4200
   gcttgcttcc agtgcggtct acaaggacac tttaaaaaag attgtccaag tagaagtaag 4260
   ccgccccctc gtccatgccc cttatttcaa gggaatcact ggaaggccca ctgccccagg 4320
   ggacaaaggt cctctgagtc agaagccact aaccagatga tccagcagca ggactgaggg 4380
   tgcctggggc aagcgccatc ccatgccatc accctcacag agccctgggt atgcttgacc 4440
   attgagggcc aggaggttgt ctcctggaca ctggtgcggt cttcttagtc ttactcttct 4500
   gtcccggaca actgtcctcc agatctgtca ctatctgagg gggtcctaag acgggcagtc 4560
   actagatact tctcccagcc actaagttat gactggggag ctttattctt ttcacatgct 4620
   tttctaatta tgcttgaaag ccccactacc ttgttaggga gagacattct agcaaaagca 4680
   ggggccatta tacacctgaa cataggagaa ggaacacccg tttgttgtcc cctgcttgag 4740
   gaaggaatta atcctgaagt ctgggcaaca gaaggacaat atggacgagc aaagaatgcc 4800
   cgtcctgttc aagttaaact aaaggattcc acctcctttc cctaccaaag gcagtacccc 4860
   ctcagaccca aggcccaaca aggactccaa aagattgtta aggacctaaa agcccaaggc 4920
   ctagtaaaac catgcagtaa cccctgcagt actccaattt taggagtaca gaaacccaac 4980
   agacagtgga ggttagtgca agatctcagg attatcaatg aggctgttgt tcctctatag 5040
   ccagctgtac ctagccctta tactctgctt tcccaaatac cagaggaagc agagtggttt 5100
   acagtcctgg accttcagga tgccttcttc tgcatccctg tacatcctga ctctcaattc 5160
   ttgtttgcct ttgaagatac ttcaaaccca acatctcaac tcacctggac tattttaccc 5220
   caagggttca gggatagtcc ccatctattt ggccaggcat tagcccaaga cttgagccaa 5280
   tcctcatacc tggacacttg tccttcggta ggtggatgat ttacttttgg ccgcccattc 5340
   agaaaccttg tgccatcaag ccacccaagc gctcttcaat ttcctcgcta cctgtggcta 5400
   catggtttcc aaaccaaagg ctcaactctg ctcacagcag gttacttagg gctaaaatta 5460
   tccaaaggca ccagggccct cagtgaggaa cacatccagc ctatactggc ttatcctcat 5520
   cccaaaaccc taaagcaact aaggggattc cttggcgtaa taggtttctg ccgaaaatgg 5580
   attcccaggt atggcgaaat agccaggtca ttaaatacac taattaagga aactcagaaa 5640
   gccaataccc atttagtaag atggacaact gaagtagaag tggctttcca ggccctaacc 5700
   caagccccag tgttaagttt gccaacaggg caagactttt cttcatatgt cacagaaaaa 5760
   acaggaatag ctctaggagt ccttacacag atccgaggga tgagcttgca acctgtggca 5820
   tacctgacta aggaaattga tgtagtggca aagggttgac ctcattgttt acgggtagtg 5880
   gtggcagtag cagtcttagt atctgaagca gttaaaataa tacagggaag agatcttact 5940
   gtgtggacat ctcatgatgt gaatggcata ctcactgcta aaggagactt gtggctgtca 6000
   gacaactgtt tacttaaatg tcaggctcta ttacttgaag ggccagtgct gcgactgtgc 6060
   acttgtgcaa ctcttaaccc agccacattt cttccagaca atgaagaaaa gataaaacat 6120
   aactgtcaac aagtaatttc tcaaacctat gccactcgag gggacctttt agaggttcct 6180
   ttgactgatc ccgacctcaa cttgtatact gatggaagtt cctttgtaga aaaaggactt 6240
   cgaaaagtgg ggtatgcagt ggtcagtgat aatggaatac ttgaaagtaa tcccctcact 6300
   ccaggaacta gtgctcagct agcagaacta atagccctca cttgggcact agaattagga 6360
   gaagaaaaaa gggcaaatat atatacagac tctaaatatg cttacctagt cctccatgcc 6420
   catgcagcaa tatggaaaga aagggaattc ctaacttctg agagaacacc tatcaaacat 6480
   caggaagcca ttaggaaatt attattggct gtacagaaac ctaaagaggt ggcagtctta 6540
   cactgccggg gtcatcagaa aggaaaggaa agggaaatag aagagaactg ccaagcagat 6600
   attgaagcca aaagagctgc aaggcaggac cctccattag aaatgcttat aaaacaaccc 6660
   ctagtatagg gtaatcccct ccgggaaacc aagccccagt actcagcagg agaaacagaa 6720
   tggggaacct cacgaggaca gttttctccc ctcgggacgg ctagccactg aagaagggaa 6780
   aatacttttg cctgcaacta tccaatggaa attacttaaa acccttcatc aaacctttca 6840
   cttaggcatc gatagcaccc atcagatggc caaatcatta tttactggac caggcctttt 6900
   caaaactatc aagcagatag tcagggcctg tgaagtgtgc cagagaaata atcccctgcc 6960
   ttatcgccaa gctccttcag gagaacaaag aacaggccat taccctggag aagactggca 7020
   actgatttta cccacaagcc caaacctcag ggatttcagt atctactagt ctgggtagat 7080
   actttcacgg gttgggcaga ggccttcccc tgtaggacag aaaaggccca agaggtaata 7140
   aaggcactag ttcatgaaat aattcccaga ttcggacttc cccgaggctt acagagtgac 7200
   aatagccctg ctttccaggc cacagtaacc cagggagtat cccaggcgtt aggtatacga 7260
   tatcacttac actgcgcctg aaggccacag tcctcaggga aggtcgagaa aatgaatgaa 7320
   acactcaaag gacatctaaa aaagcaaacc caggaaaccc acctcacatg gcctgctctg 7380
   ttgcctatag ccttaaaaag aatctgcaac tttccccaaa aagcaggact tagcccatac 7440
   gaaatgctgt atggaaggcc cttcataacc aatgaccttg tgcttgaccc aagacagcca 7500
   acttagttgc agacatcacc tccttagcca aatatcaaca agttcttaaa acattacaag 7560
   gaacctatcc ctgagaagag ggaaaagaac tattccaccc ttgtgacatg gtattagtca 7620
   agtcccttcc ctctaattcc ccatccctag atacatcctg ggaaggaccc tacccagtca 7680
   ttttatctac cccaactgcg gttaaagtgg ctggagtgga gtcttggata catcacactt 7740
   gagtcaaatc ctggatactg ccaaaggaac ctgaaaatcc aggagacaac gctagctatt 7800
   cctgtgaacc tctagaggat ttgcgcctgc tcttcaaaca acaaccagga ggaaagtaac 7860
   taaaatcata aatccccatg gccctccctt atcatatttt tctctttact gttcttttac 7920
   cctctttcac tctcactgca ccccctccat gccgctgtat gaccagtagc tccccttacc 7980
   aagagtttct atggagaatg cagcgtcccg gaaatattga tgccccatcg tataggagtc 8040
   tttctaaggg aacccccacc ttcactgccc acacccatat gccccgcaac tgctatcact 8100
   ctgccactct ttgcatgcat gcaaatactc attattggac aggaaaaatg attaatccta 8160
   gttgtcctgg aggacttgga gtcactgtct gttggactta cttcacccaa actggtatgt 8220
   ctgatggggg tggagttcaa gatcaggcaa gagaaaaaca tgtaaaagaa gtaatctccc 8280
   aactcacccg ggtacatggc acctctagcc cctacaaagg actagatctc tcaaaactac 8340
   atgaaaccct ccgtacccat actcgcctgg taagcctatt taataccacc ctcactgggc 8400
   tccatgaggt ctcggcccaa aaccctacta actgttggat atgcctcccc ctgaacttca 8460
   ggccatatgt ttcaatccct gtacctgaac aatggaacaa cttcagcaca gaaataaaca 8520
   ccacttccgt tttagtagga cctcttgttt ccaatctgga aataacccat acctcaaacc 8580
   tcacctgtgt aaaatttagc aatactacat acacaaccaa ctcccaatgc atcaggtggg 8640
   taactcctcc cacacaaata gtctgcctac cctcaggaat attttttgtc tgtggtacct 8700
   cagcctatcg ttgtttgaat ggctcttcag aatctatgtg cttcctctca ttcttagtgc 8760
   cccctatgac catctacact gaacaagatt tatacagtta tgtcatatct aagccccgca 8820
   acaaaagagt acccattctt ccttttgtta taggagcagg agtgctaggt gcactaggta 8880
   ctggcattgg cggtatcaca acctctactc agttctacta caaactatct caagaactaa 8940
   atggggacat ggaacgggtc gccgactccc tggtcacctt gcaagatcaa cttaactccc 9000
   tagcagcagt agtccttcaa aatcgaagag ctttagactt gctaaccgct gaaagagggg 9060
   gaacctgttt atttttaggg gaagaatgct gttattatgt taatcaatcc ggaatcgtca 9120
   ctgagaaagt taaagaaatt cgagatcgaa tacaacgtag agcagaggag cttcgaaaca 9180
   ctggaccctg gggcctcctc agccaatgga tgccctggat tctccccttc ttaggacctc 9240
   tagcagctat aatattgcta ctcctctttg gaccctgtat ctttaacctc cttgttaact 9300
   ttgtctcttc cagaatcgaa gctgtaaaac tacaaatgga gcccaagatg cagtccaaga 9360
   ctaagatcta ccgcagaccc ctggaccggc ctgctagccc acgatctgat gttaatgaca 9420
   tcaaaggcac ccctcctgag gaaatctcag ctgcacaacc tctactacgc cccaattcag 9480
   caggaagcag ttagagcggt cgtcggccaa cctccccaac agcacttagg ttttcctgtt 9540
   gagatggggg actgagagac aggactagct ggatttccta ggctgactaa gaatccctaa 9600
   gcctagctgg gaaggtgacc acatccacct ttaaacacgg ggcttgcaac ttagctcaca 9660
   cctgaccaat cagagagctc actaaaatgc taattaggca aagacaggag gtaaagaaat 9720
   agccaatcat ctattgcctg agagcacagc aggagggaca atgatcggga tataaaccca 9780
   agtcttcgag ccggcaacgg caaccccctt tgggtcccct ccctttgtat gggagctctg 9840
   ttttcatgct atttcactct attaaatctt gcaactgcac tcttctggtc catgtttctt 9900
   acggcttgag ctgagctttc gctcgccatc caccactgct gtttgccgcc accgcagacc 9960
   cgccgctgac tcccatccct ctggatcatg cagggtgtcc gctgtgctcc tgatccagcg 10020
   aggcacccat tgccgctccc aatcgggcta aaggcttgcc attgttcctg catggctaag  10080
   tgcctgggtt catcctaatt gagctgaaca ctagtcactg ggttccatgg ttctcttctg  10140
   tgacccacag cttctaatag agctataaca ctcaccgcat ggcccaaggt tccattcctt  10200
   gaatccataa ggccaagaac cccaggtcag agaacacgag gcttgccacc atcttgggag  10260
   ctctgtgagc aaggaccccc aagtaaca                                    10288
<210> 7
   <211> 6818
   <212> DNA
   <213> Homo sapiens
<400> 7
   gcaactccct ttgggtctcc tctcattgta tgggagctct gttttcactc tattaaatct     60
   tgcaactgca cactcttctg gtctgtgttt gttatggctt gagctgagct tttgctggct    120
   gtccaccact gctgtttgct gccgtcgcag accccttgct gactcccacc cctgcggatc    180
   tggcagggtg tctgctgcgc tcctgatcca gccaggcacc cactgctgct cccaatcagg    240
   ctaaaggctt gccattgttc ctgcatggct aagtgcccgg gttcgtgcta attgagctga    300
   acactagtcg ctgggttcca cagttctctt ccgtgaccca cagcttctaa tagagctata    360
   acactcactg catggcccaa cattccattc cttggaatct gtgaggccaa gaacccccgg    420
   tcagagaaca agaagcttgc caccatcttg gaagcagccc gccaccattt tgggagctct    480
   aagaacaagg accccccagt aacattttgg tgaccacgaa gggacctcca aagcagtgag    540
   taatattgaa ccacttccgc ttgctattct gtcctaacct tccttagaat tggaggaaaa    600
   taccgggcac ctgtcggcca gttaagaacg attagcgtgg ccgccagact taagactctg    660
   gtgtgaggct gtctgggaaa gggctttcta acaaccccca acccttccgg gttgggagct    720
   ttggtctgcc tggaaccagc ttccactttc aattttcctg gggaatccaa gggctgacta    780
   gaggcagaaa gctgtcatcc cgaactcctg gcattagaca gttgagatcg tggcgcagcc    840
   agaagtctct actcaacagt cacccatgcg tgcaccccta cctttccttc taacccatac    900
   ctcccgggtc ccaaccatga ctttcttgaa agtgtagccc ctaaattctc tttacctcta    960
   aatctacttc ttctcatccc tgcttcctag gtactaatgg ttcagacttt catttcctct   1020
   agcaagttct atctccagag ggatctaagg aagggatcta tgctgtgtcc ttaggcccct   1080
   aggctatgaa cccagagagt cttctccctg ttatctctcc ccatttaggc atacagctct   1140
   caacatggac agttatgtgg gacccattcc ctaccaccct tgccagggcc ccaagttttc   1200
   aaagggctag aagaaaaaag agagaaagag agagagaggc agaggggaga gaaagagaga   1260
   gagacaaaga gggagtcaaa gagagataga aagagaaaga tagaactagt aaagaaaaaa   1320
   agtatgcccc attcctttaa aagccagggt aaatttaaaa cctataattg ataattgaag   1380
   gtcttctcca tgaccctata acactccaat accaccttgt tttcagtgta aacaagggtg   1440
   tagcccgaaa acactgagac cactgacaac ccatagcctt cctatcaaaa atccttaacc 1500
   caggaaccca tggatggccc aaatgcattc aatctgtagc agcaactgct ttgctaacag 1560
   aagaaagtag aaaagtaact tttagagaaa acctcattgt gagcacacct caccagttca 1620
   gaattattct aagtcaaaaa agcaaaaagg tagcttacta actcaaaaat cttaaagtat 1680
   ggggttattc tgttagaaaa aggtgattta acattaacca ctgaaaattc ccttaaccca 1740
   gcaggtttcc taatgggatt taaatcttca ttaccataca aaggtccgac cagacccagc 1800
   aggaactccc tttaggacag gatgatagat ggttcctcct gggtgattga gggggtgaaa 1860
   aaccacaatg ggtgttcagt aattgatagg gagactcttg tggaaggaga gttaggaaaa 1920
   ttgcctaata attggtctgc tcaaatgtgc gagctgtttg cactcagcca agccttaaag 1980
   tacttacaga atcaaaaaga ctctatctca atcctgactc aaaatgttac ctacaccatc 2040
   tctgacatga atttgcataa gaactgttgt ttatgggaat gcatcttgat ggggcagctg 2100
   ggttgttatg aaatactcag gaacccagcc caggtctaga attcacctct gagcgcaaag 2160
   gcaatgttgg ccatgctggt aaaggaccac tagaatccag gagcctggac ccctttcttt 2220
   gtggtcaaga aaggcgggaa aacaggtgca ggactgctac atcagagagc ataacaaatc 2280
   cgataagcag agttccatga gtggttaagc accctggaaa ggaactcacc tctgagtgca 2340
   aaggcaatgt taggcacacc agtaaaggac cactagaatc cagcagccca gacccctttc 2400
   tttgtgatca agaaaggcgg gaaaaggggt gcaggactgc tacatcagtg agcgtaacta 2460
   atctgataag cagaagtcca tgggtggtta cgcaccctgg aaaggaataa gcattaggac 2520
   cacagaggac actctaagac taatgctcat tggaaaatga ctaggggtgc tggcatccct 2580
   atgttttttt ttcagatggg aaacattccc cccaaggcaa aaacgcccat aagatatatt 2640
   ctggagaatt cggcccagag tgtatgtatc ttttttccct gtcagacttg aagcaaacct 2700
   aggtaaatta tcagatagcc ctgatggcta tattgatgct ttacaagggt taggacaatc 2760
   ctttgatcta acatggagag atatactgtt actgctagat cagacactaa tcccaaatga 2820
   aagaagtgcc accataactg cagccagaga gtttgatgat ctctggtatc tcagtcaggt 2880
   caatgatagg atgacaacag aagaaagaaa acaattcccc acaggccagc aggcagttcc 2940
   cagcgtagac cttcattggg acacagaatc agaacatgga gattggtgcc gcagacattt 3000
   actaacttgc gcgctagaag cactaaggaa aactaggaag aagcctatga attattcaat 3060
   gatgtccact ataacacagg gaaaggaaga aaatcctact gcctttctgg agagactaag 3120
   ggaggcattg agaaagcata cctctctgtc acctgactct attgaaggcc aactaatctt 3180
   aaaggataag ttttccactc agtcagctgc agacattaga aaaaaacttc aaaagtctgc 3240
   gttaggccgg gagcaaaact tagaaaccct attgaacttg gcaacctcag ttttttatga 3300
   tagagatcag gaggatcagg tggaatggac aaatgagatt ttaaaaaaag gccaccactt 3360
   tagtcatggc cctcaggcaa gcagactttg gacactctgg aaaagggaaa agctgggcaa 3420
   atcgaatgcc taataagact tgcttccagt gtggtctaca aggacacttt aaaaaagatt 3480
   gtccaaatag aaataagcca ccccctcgtc catgctcctt atgtcaaggg aatcactgga 3540
   aggcctactg ccccagggga tgaaggtcct ctgagtcaga agccactaac cagatgattc 3600
   agccccagga ctcagggtgc ccagggcaag cgccagccta tgccatcacc ctcacagagc 3660
   cctgggtatg cttgaccatt gagggtcagg aggttaacta tctcctggac actggcgtgg 3720
   ccttctcagt cttactctcc tgtcccggac aactgtcctc cagatctgtc actatccgag 3780
   ggtttctacg acagccagcc actagatact tctcccagcc actaagttgt gactggggaa 3840
   ctctactctt ttcacatgtt tttctaatta tgcctgaaag ccccactcct ttgttaggga 3900
   aagacattct agcaaaagca ggggccatta tacacctgaa cataggagaa ggaacacctg 3960
   tttgttgtcc cctgcttgaa gaaggaatta atcctgaagt ctggacaaca gaaggacaat 4020
   acagatgagc aacaaatgcc tgtcctgttc aagttaaact aaaggattat gcctcctttc 4080
   cctaccaaag gcagtacccc cttagacccg aggcccaaca aggactccaa aagattgtta 4140
   aggacctaaa agctcaaagc ctagcaaaac catgcagtag cccctgcaat actccaattt 4200
   taggagtaca gaaaaccaac agacagtgga ggttagtgca agatctcagg attatcaatg 4260
   aggctgttgt tcctaaccct tatactctgc tttcccaaat accagaagaa gcagagtggt 4320
   ttacagtcct ggaccttaag gatggctttt tctgcatccc tgtacatcct gactctcaat 4380
   tcttgtttgc ctttggagat ccttcgaacc caatgtctca actcagcttg actgttttac 4440
   cccaagggtt cagggatagc ccccatctag ttggccaagc attagccgag ccagttctcc 4500
   tacctggaca ctcttgtcct ctggtacatg gatgatttat ttttagctgc ccgttcagaa 4560
   accttgtgcc atcaagccac ccaagtgctc ttaaatttcc tcgccacctg tggctacaag 4620
   gtttccaaac caaaggctca gctctgctca cagcaggtta aatacttagg gctaaaatta 4680
   tccaaaggca ccagggccct cagtgaggaa tgtatccagc ctgtattggc ttatcctcat 4740
   cccaaaaccc taaagcaact aagagggttc cttggcataa caggtttctg ccaaatgtgg 4800
   attcccaggt acggtgaaat agccaggcca ttatataccc taattaagga aactcagaaa 4860
   gccaacaccc atttattaag atggacacct gaagcagaag cagctttcca ggccctaaag 4920
   aaggccctaa cccaagcccc agtgttaagc ttgccaacgg ggaagacttt tctttatatg 4980
   tcacagaaaa aacaggaata gctctaggag tccttagaca ggtccaaggg atgagcttgc 5040
   aacctgtggc atacctgagt aaggaaattg atgtagttgc aaagggttga cctcattgtt 5100
   tacaggtagt ggcggcagta gcagtcttag tatctgaagc agttaaaata atacagggaa 5160
   gagatcttac tgtgtggaca tctcatgatg taaacggcgt actcacttct aaaggagact 5220
   tgtggctgtc agacaaccgt ttacttaaat atcaggctct attacttgaa gggccagtgc 5280
   tgcgactgcc cacttgttca actcttaacc cagccacatt tctttcagac aatgaagaaa 5340
   agatagaaca taactgtcaa caggtgattg ctcaaaccta cggcgctcga ggggaccttc 5400
   tagaggttcc cttgactgat cccaacctca acttgtatac tgatggaagc tcctttgtag 5460
   aaaaaggact ttgaaaggtg gggtatgcag tggtcagtga taatggaata cttgaaagta 5520
   attccttcac tccaggaact agtgctcagc tggcagaact aatagccctc actcaggcac 5580
   tagaattagg agaaggaaaa agggtaaata tatatgcaga ctctaagtat gcttacccag 5640
   tcctccacgc ccacacagca atatggagag ataggaaatt cctaacttct gagggaacac 5700
   cgatcaaaca tcaggaagcc attaggagat tattattggc tgtacagaaa cctaaagagg 5760
   tggcagtctt acactgctgg ggtcatcaga aaggaaagga aaaggaaata gaaaggaacc 5820
   accaagtgga tattgaagcc aaaagagcca caaggcaggc cctccattag aaatgcttat 5880
   agaaggatcc ctagtatggg gtaatcccct ccgggaaacc aagccccagt actcagcagg 5940
   agaaatagac acgaggacat agtttcctcc cctcaggatg gctagccacc gaaaaaggga 6000
   aaatactttt gcctgcagct aatcaatgga aattacttaa aacccttcac caaacctttc 6060
   acttgggcat ggatagcatc tatcagatgg ccaatttatt atttactgga ccaggccttt 6120
   tcaaaactat caagcagata gtcagggcct gtgaaatgtg ccaaagaaat aatcccctgc 6180
   acttcaagcc atacatttca atccctgtat ctttaacctc ctgttgtttg tctcttccag 6240
   actcaaagct gtaaaactgc aaatggttcc tcatatggag ccccagatgc agtccatgac 6300
   taagatctac cacagagccc tagaccggcc tgttagccca tgctccgatg ttgatgacat 6360
   caaaggcaca ccttccgagg aaatctcaac tgcacgaccc ctactaagcc ccaattcagc 6420
   aggaagcagt taagagcagt cgttggctaa catccccaat agtatgtggg ttttcctgtt 6480
   gagagggggg actgagagac aggactagct ggatttccta ggccaactaa gaatccctaa 6540
   gcctagttgg gaaggtgacc gcatccacct ttaaacacgg ggcttgcaac ttagctcaca 6600
   cccgaccaat caggtagtaa agagagctca ctaaaatgct aattaggcaa aaacaagagg 6660
   taaagaaata gccaatcatc tatcgcctga gagcacagtg gggagggaca atgatcggga 6720
   tataaaccca ggcattcggg ccggcaacgg caacccccat tgcgtcccct cccattgtat 6780
   gggagctctg ttttcattct attaaatctt gcaactgc 6818
<210> 8
   <211> 7073
   <212> DNA
   <213> Homo sapiens
<400> 8
   gcaaccccct ttgggtctcc tccctttgca taggagctct gttttcactc tattaagtct 60
   tgcaactgca ctcttctggt ccgtgtttct taccgcttga gctgagcttt ccctcactgt 120
   ccaccactgc tgttttgcca ccgtcacagg cccaccgctg acttccattc ttctggatct 180
   agcaggctgt ccactgtgct cctgatccag cgaggcgccc attgccgctc ccgattgggc 240
   taaaggcttg ccattgttcc tgcatggcta cgtgcctggg ttcatcctaa tcaagccgaa     300
   cactagtcac tgggttccac ggttctcttc catgacccac gacttctaat agaactataa     360
   cactcacctc atggcccaag attccattcc ttggaatcca tgaggccaag aaccccaggt     420
   cagagaacac gaggcttgcc accatcttgg aagtggcccc accaccatct tgggagctct     480
   gggagcaagg acccccggta acattttggc gaccacaaag ggacatccaa agtggtgagt     540
   aatattggac cactttcact tgctattctg ttctatcctt ccttagaact ggaggaaaat     600
   accaggcaca ggcacctgtc agccagttaa aaacaattag cgtcgccgcc acacttaaga     660
   ctcaggtgtg aggctatctg gggaaagact ttctaacaac ccccaaccca tctagtgggg     720
   atgttggtct gcctggagac agcttccact ttcaattttc ttggggaagc cgagggctca     780
   ctagaggcag acagctgttg tcccaaactc cgggcagtag ccggttgaga tcatggtgca     840
   gccaggagtc tctactcagc agtcgccgat gcatgtgccc ctaccttccc ttctgaccca     900
   tacatcctga gtcccgactg tgactttctt gaaagtgtag ccccaaaatt ctccttacct     960
   ctgaatctac ttcctctgat ccctgcctcc tgggtactaa tgattcagac tttcatttcc    1020
   tctagcaagt tgtgtctcca aagggatcta aggaggctct acgctgcatc cttaggcacc    1080
   taggctataa cccaaggagt cttatccctg gtgtccctcc cgatttgggt atacaactct    1140
   caacatgggc agttatgtag gacccattcc ccaccacact tgccagggcc ccaagtttgt    1200
   aatggctaag agagagacac agagagagag agagagatgg agagagagac aaggagggag    1260
   tcaaagagaa aaagaaagaa aaagaaatag tagaaaaaaa agtgtgccct attcctttaa    1320
   aagccagggt aaatttaaaa cctgtaattg ataattgaag gtcttctccg tgaccctgta    1380
   acactccaat gccattttgt tgtcagtgta aataagggca tagcccaaaa gcactgaggt    1440
   cactgacaac ccgtagcttt cccatcaaaa atccttaacc cagtaatccg cggatgggcc    1500
   aaatgcattc agtcggtagc agcaaccgct ttgctaaaag tagaaaagta acttttagag    1560
   gaaacctcat tgtgagcgca cacctcacca gttcagaatt attctaagtc aaaaaaaaaa    1620
   aaagcaaaaa ggtaacttac taactcaaaa atcttaaagt ataggtctat catattagaa    1680
   aagggtaatg taactccaac cactgataat tcccttaacc cagcagattt cctaacaggg    1740
   gatttaaaac ttaattacca tacaaaggtc ccaccagacc taggaggaac tcccttcagg    1800
   acaggacgat aaacggttcc tcccaggtga ttgaggaaaa aaaccacaat gggtattcag    1860
   taattgatac agagactcat gtggaagcag ttagaaaaat tgcctaataa ttggtctcct    1920
   caaacgtgta agctgtttgc actcagccaa gccttaaagt acttacagaa tcaaaaagac    1980
   tctgaatcct gactcaaaag gtttgctaca ccctctgtga aacaaatttg cataagaact    2040
   gttgtttatg ggaaggcatc ttgatggggc agctgggttg ttatgaaata ctcaggaccc    2100
   cagcccggct ctaggactca cccctgagcg caaaaggcaa tgttgggcac gctggtaaag    2160
   gaccactaga atccagcagc ccggacccct ttctttgtgg tcaagagagg cgggaaaaca    2220
   ggtgcaggac tgctacatca gtgagcataa ctaatccagt aagcagaggt ccatgggtgg   2280
   ttatgcaccc tggaaaagaa tacgcattag gcccttagag gatgctctag gactaatgct   2340
   catcggaaaa tgactagggg tgctgacatc cctatgttct tttttcagat gggaaacgtt   2400
   cctcccaccc caaggcaaaa aacaccccta agatgtattt tggagaatta ggaccaattt   2460
   gaccctcaga cactaagaaa gaaatgactt acattcttct gcagtaccat gatatcctct   2520
   tcaaggggga gaaacctggc ctcctgagag aagtataaat tataacacca tcttacagtg   2580
   agacctcttc tgtagaaagg agggcaaatg gagtgaagtg caaactttcc tttcattaag   2640
   agacaactcg caattatgta aaaagtgtga tttatgccct acagaaagcc ctcagtctac   2700
   ctccctatcc cagggtcccc ccgattcctt tcccaactaa taaggacccc ccttttaccc   2760
   aaatggtcca aaggagatag atgaagggat aaacaatgaa ccaaacagtg ccaatattcc   2820
   ctgattatgc cccctccagg cagtgggagg aggagaattc ggcccagcca gagtgcatgt   2880
   accttttttt ttctctcaga cttaaagcaa attaaaatag acctaggtaa attctcagat   2940
   aaccctgatg gctatattga tgttttacaa gggttaggac aatcctttgc tctgacatgg   3000
   agagatataa tgttactgct aaatcagaca ctaaccccaa atgagagaag tgtcaccata   3060
   gctgcagccc aagagtttgg caatctctgg tatctcagtc aggtcaatga taggatgaca   3120
   acagaggaaa gggaatgatt ccccacaggc cagcaggcag ttctcagtgt agaccctcac   3180
   tgggacacag aataagaaca tggagatcgg tgccgcagat atttgctaac ttgcgtgcta   3240
   ggactaagga aaactaggaa gaagcctatg aattattcag tgatgtccac tataacacag   3300
   ggaaaggaag aaaatcatac tgcctttccg gaaatactaa gggaggcatt gaggaagcat   3360
   acctctctgt cacctgactg tattgaagtc caactaatct taaaggatat gtttatcact   3420
   cagtcagctg cagacattag aaaaaacttc aaaagtccac cttaggccca gagcaaaact   3480
   tagaaaccct attgaacttg ttaacctcag ttttttataa tagagatcag gaggagcagg   3540
   cggaacagga caaacaggat taaaaaaaga ccaccgcttt agtcatggcc ctcaggcaag   3600
   tggactttgg aagctctgga aaagggaaaa gctgggcaaa ttgaatgcct aatagggctt   3660
   gcttccagtg tggtctacaa ggacacttaa aaaaagattg tccaagtaga aataagctgc   3720
   cccttcgtcc atgcctctta tgtcaaggga atcactggaa ggcccattgc cccaggggag   3780
   gaaggtcctc tgagtcagaa gccactaacc agatgatcca gcagcaggac taagggtgcc   3840
   cagggcaagc cccagcccat gccatcaccc tcacagagcc ccgggtatgc ttgaccattg   3900
   agggccagga ggttaactgt ctcctgaaca ctggcacagc cttctcagtc ttactttcct   3960
   gtcccggaca actgtcctcc agatctgtca ctatctgagc ggtcctagga cagccagtca   4020
   ctagatattt ctcccagcca ctaagttgtg actggggaac tttactcttt tcacatgctt   4080
   ttctaattat gcctgaaagc cccactcctt tgttagggag agacattcta gcaaaagcag   4140
   gggccattat acatctgaac ataggagaag gaacacccgt ttgttgtcac ctgcttgagg   4200
   aaggaattaa tgctgaagtc tgggcaacag aaggacaata tggatgagca aagaatgccc   4260
   atcctgttca agttaaatta aaggattccg cctcctttcc ctaccaaagg caataccccc   4320
   ttagacccga ggcccaacaa ggactccaaa agattgttaa ggacctaaaa gcccaaggcc   4380
   tagtaaaacc atgcaatagc ccctgccata ctccaatttt aggagtaagg aaacccaacg   4440
   gacagtggag gttagtgcaa gaactcagga ttatcaatga ggctgttgtt cctctatacc   4500
   cagctgtacc taacccttat acagtgcttt cccaaatacc agaggaagca gagtggttta   4560
   cagtcctgga ccttaaggat gcctttttct gcatccctgt acgtcctgac tctcaattct   4620
   tgtttgcctt tgaagatcct ttgaacccaa cgtctcaact cacctggact gttttacccc   4680
   aagggttcaa ggatagcccc catctatttg gccaggcatt agcccaagac ttgagccaat   4740
   tctcatacct ggacactctt atccttcggt atggggatga tttaatttta gctacccatt   4800
   cagaaacgtt gtgccatcaa gccacccaag tgctcttaaa tttcctcgct acctgtggct   4860
   acaggtttcc aaacgaaagg ctcagctctg ctcacagcag gttaaatact tagggctaaa   4920
   attatccaaa ggcaccaggg ccctcagtga ggaacgtatc cagcctatac tggcttattc   4980
   tcatcccaaa accctaaagc aactaagagc attccttggc ataacaggct gctgctgaat   5040
   atggattccc aggtacagtg aaatagccag gccattatac acactaatta aggaaactca   5100
   gaaagccaat acccatttag taagatggac accttaagca gaagcggctt tccaggcctt   5160
   aaagaaggcc ctaacccaag ccccagtggt aagcttgcca acagggcaag acttttcttt   5220
   atatgtcaca gaagaaacag gaatagctct aggagtcctt acacaggtct gagggatgag   5280
   cttgcaaccc atggcatacc tgagtaagga aactgatgta gtggcaaagg gttggcctca   5340
   ttgtttacgg gtagtggcag cagtagcagt cttagtatct gaagtagtta aaataataca   5400
   gggaagagat cttactgtgt gaacatctca tgatgtgaat ggcatagtca ctgctaaagg   5460
   agacttgtgg ctgtcagaca actgtttact taaataccag gctctattac ttgaagggcc   5520
   agtgctgcga ctgtgcactt gtgcaactct taacccagac acatttcttc cagacaatga   5580
   agaaaagata gaacataact gccaacaagt aattgctcaa acctatgcca ctcgagggga   5640
   ccttttagag gttcccttga ctgatcccaa cctcaacttg tatactgatg gaagttcctc   5700
   tgtagaaaaa ggactttgaa aagtggggta tgcagtggtc agtgataatg gaatacttga   5760
   aagtaatccc ctcactccag gaactagtgc tcagctggca gaactaatag ccctcactcg   5820
   ggcactagaa ttaggagaag agaaaagggt aaatatatac agactctaag tatgcttacc   5880
   tagtcctcca tgcccatgca gcaatatgga gagaaaggga attcctaatt tccaagggaa   5940
   cacctatcca acatcaggaa gccattagga gattactatt ggctgtacag aaacataaag   6000
   aggtggcaat cttacactgc cggtgtcacc agaaaggaaa ggaaagggaa atagaaagga   6060
   accaccaagc ggatattgaa gccaaaagag ccgcaaggca ggaccctcca ttagaaatgc   6120
   ttatagaagg acccctagta tggggtaatc ccctccagga aaccaagccc cagtactcag 6180
   aagaagaaat agaatgagga acctcacaag cacatagttt cctcccctca ggatggctag 6240
   ccactgaaga aggaaaaata cttttgcctg cagctaacca atggaaatta cttaaaaccc 6300
   ttcaccaaac atttccctta ggcattgata gcacccatca gatggccaaa ttattattta 6360
   ctggaccagg ccttttcaaa actatcaagc agatagtcag ggcctgtaaa gtgtgccaaa 6420
   caagtaatcc cctgcactgc aggccataca tttcaatccc tgtatcttta acctccttgt 6480
   taagtttgtc tcttccagaa tcaaagctgt aaaactacaa atagttcttc aaatggagcc 6540
   ccagatgtag tccatgacta agatctaccg cggacccctg gacaagcctg ctagcccatg 6600
   ctctgatgtt aatgacatgg aaggcacccc tcccgaggaa atcgcaactg cacaacccct 6660
   attacacccc aattcagcag gaagcagtta gagcattcat cagccaacct ccccaacagc 6720
   acttgggttt tcctattgag agggggtact gagagacagg actagctgga tgtcctaggc 6780
   tgactaagaa tccctaagcc tagctgggaa ggtgaccaca tccaccttta aatacggggc 6840
   ttgcaaccta gctcacaccc aacagatcag agagctcgtt aaaatgctaa ttaggcaaaa 6900
   acaggaggta aagaaatagc caatcatcta ttgcctgaga gcacagcagg agggacaagg 6960
   attgggatat aatcccaggc attcgagctg gcaacagcaa ccccctttgg gtcccctccc 7020
   tttgtatggg agctgttttc actctatttc actctattaa atcttgcaac tgc 7073
<210> 9
   <211> 5068
   <212> DNA
   <213> Homo sapiens
<400> 9
   gcaacctcct ttgagtcccc tccctttgta taggagctct gttttcactg tgtttcactc      60
   tattaaatct tgcaattgca ctcttctggt ccatatttgt cacggcttga gctgagcttt     120
   cacttgccgt ccaccactac tgtttgctgc tgtcacagac ccgccgctga ctcccatccc     180
   gctgctgact cccatccctc cggatccggc agggtgtccg ctgtgctcct gatccagcaa     240
   gactcccatt gccactcccg atagtgctaa aggcttgcca ttgttcctgc atggctaagt     300
   gcctgggttc gtcctaatcc agctgaacac tagtcactgg gttccacggt tctcttccat     360
   gacccgcggc ttctaataga gctataacac tcaccacatg gcccaatatt ccattccttg     420
   gaatccgtga ggccaagaac cccaggtcag agaacacgag gcttgccacc atcttggaag     480
   cagcctgcca ccatcttgga agtggctcac caccgtcttg ggagttctgt gaacaaggac     540
   ccctggtaac attttggcga ccacgaaggg acatccaaag ctgtgagtaa tattggacca     600
   ctttcgcttg ctattctgtt ctatccttag aactggagga aaatactggg cacctgtcgc     660
   cagttaaaaa tgattagcat ggccgccgga cttaagactc aggtgtgagg ctatctggga     720
   aagggctttc taacaacccc caagccttct gttgggaact ttggtctgcc tggagccagc     780
   ttccactttc aattttcttg gggaagccaa gggctgactg gaggcagaaa gctgttgtcc     840
   cgaactcccg gcagtagccg gttgagatca tggcgcagcc agaagtctct actcggcagt     900
   cgcccatgcg tgcgccctta cctttccttc tgaattatac ctccggggtc ccgactccga     960
   ctttcttgag agtttagccc caaaattctc cttacctctg aatctacttc ctttgatccc    1020
   tgcctcctgc ctcctaggta ctaatagttc agactttcat ttcctctagc aagttgtgtc    1080
   tccaaaggga tctaaggagg ctctatgctg tgtccttagg cacctaggct ataacccagg    1140
   gagtcttatc cctggtatcc ctcccgattt aggtatacag ctcttgacat gggcagttat    1200
   gtgggacctg ttccccacca cccttgtgag ggccccaagt ttgtaatggc taagaaagag    1260
   agacggagag agagagagac ggagaaagag acaaagaggg agtcaaagag aaaaagaaag    1320
   aaaaagatag aaatagttaa aaaaaaaaaa aagtgtgccc tattccttta aaagccaggg    1380
   taaatttaaa acctgtaatt gataattgcc actttgttgt cagtgtaaat aagggcgtag    1440
   caaatcctta acccagtaac ccgcggatag gccaaatgca ttcagtcggt agcggcaaca    1500
   gctttgctaa aagtagaaaa gtaactttta gaggaaacct cattgtgagc acacctcacc    1560
   agttcagagt tattctaagt aaaaaaaaaa aaaaaaaaaa aagcaaaaag gtagcttact    1620
   aactcaataa tcttaaagta tggggctact atgctagaaa agggtaatgt aactccaacc    1680
   actgataact cccttaaccc agcagatttc ctaacagggg atttaaatct taattaccac    1740
   acgaaggtcc gaccagacct aggaggaact cccttcagca caggacgata gatggttcct    1800
   cccaggtgac tgaggaaaaa actacaatgg gtattcagta attggtatgg agactcttgt    1860
   ggaagcagag ttaaaaattt gcctaataat tggtctcctc aaatgtgcga gctgtttgca    1920
   ctcagccaag ccttaaagta cttacagaat caaaagacta tctcaatcct gactcaaaag    1980
   gttagctaca cagtctctga aatgaatttg cagaagaact gttgtttatg ggaatgcatc    2040
   ttgatggggc agctgggttg ttatgaaata ctcaggaacc cagcccagct ctaggactca    2100
   ccgctgagcg caaaggcaat gttgggcacg ctggtaaagg accactagaa tccagcagcc    2160
   caggcccctt tctttgtggt caagaaaggc aggaaaagga gtgcagaact gctacattgg    2220
   tgagcgtaac taatccaata agcagaggtc catgagtggt tatgcacgct ggaaaagaat    2280
   aagcattagg cccttagagg atgctctagg actaatgctc atcggaaaat gactaggggt    2340
   gctggcatcc ttatgttctt tcttcagatg ggaaacgttc cccccaaggc aaaagcgccc    2400
   ctaagatgta ttctggagaa ttagaaccaa tttgaccctc agatgtcaag aaagaaacga    2460
   cttatattct tctgcagtac tgcctggcca cgatatcctc ttcaaggggg agaaacctgg    2520
   cctcctgagg gaagtacaaa ttataacacc atcttacagc tagacctctt ttgtagaaaa    2580
   gaaggcaaat ggagtgaagt gccatatgtg caaactttct tttcattaag agacaactca    2640
   caattatgta aaaagtgtgg tttatgtctt acaggaagcc ctcagagtct acctccctat    2700
   cccagcattc ccccgactcc ttccccaact aataagcacc acccttgaac ccaaacagtc   2760
   caaaaggaga tagacaaaca ggtaaacaat gaaccaaaga gtgtcagtat tccccgatta   2820
   tgccccttcc aagcagtggg aggaggagaa ttcggcccag ccagagtgca tgtacctttt   2880
   tctctctcag acttaacgca aattaaaata gacttaggta aattctcaga taaccctgat   2940
   ggctacattg atgttttaca agggttaggg caatcctttg atctgacatg gagagatata   3000
   atgttactgc taaatcagac actaacccca aatgagagaa gtgccgccgt aactgcagcc   3060
   cgagagtttg gtgatctctg gtatctcagt caggtcaatg ataggatgac aacagagaaa   3120
   agagaacgat tccccacagg ccagcaggca gtttccagtg tagaccctca ttaggacaca   3180
   gaatcagaac atggagattg gtgccacaga tatttgctaa cttgagtgct agaaggacta   3240
   aggaaaacta ggaagaagcc tatgaattat tcagtgatgt ccactataac acaaggaaag   3300
   gaagaaaatc ctactgcctt tctggagaga gtaagggagg cattaaggaa gcatacctcc   3360
   ctgtcacctg actctattga aggccaacta atcttaaagg ataagtttgt cactcagtta   3420
   gctgcagaca ttagaaaaaa acttcaaaag tccgacttag gcctggagta cggctgagtg   3480
   cccaatttgg cagcaggcaa gaccaacact gagcccttca tatggcacca tgctttgtgg   3540
   tgatcagcca actacttgat ggcaggttga ttatattgga catctttcat cagagaaatg   3600
   gcagtggttt gtccttcctg gaatagacac ttattctcga tatgggtttg tctatcctgc   3660
   aggcaatgct tctgccagga gtaccatctg tggactcatg gaaagcctta tccaccatca   3720
   tggcattcca cacagcattg cctctaaaca aggcacttat tttatagcta aggaagtgtg   3780
   gcagtgggct catgctcatg gaattcactg attgtatctt gttgcccatt atcttaaagc   3840
   agctggattg atagaacagt ggaaaggcca tttgaaatca caattacacc accaactagg   3900
   tgacaatact ttgcagggct cggcaaagtt ctcttgaagg ctgagtatgt cctgaatcag   3960
   catccaatat atggtactgt ttccctcata gccagcattc acaggcctaa gaatcaaggg   4020
   gtagaagtag aagtggcacc actcaccatc actcctagtg acccactagc aaaaatttta   4080
   cttccagttc ccccaacatt atgttctgct ggccttagtt ccagagggaa gaattctgcc   4140
   accagtcgac acaagaatga taccattaaa ctgaaagtta aaattgccac ctggccactt   4200
   tgggctcctc ccacctctaa gtcaacaggt caagaaagga gttacagtgt tgacttgggt   4260
   gattgacctg gactatcaag atgaaatcag gttactactc cacagtggag gtaaggaaga   4320
   atatgtgtgg aatacaggag atcccttagg ccgtctttta gtactaccat gccctgtgat   4380
   taaggtcagt ggaaaactac aacaatccaa tctaggcagg actacaaatg gcccagactc   4440
   ttcaggaatg aagggttggg tgacttcacc aggtaaaaaa ataacagcct gctgaggtgc   4500
   tagctgaagg caaagggaat acagaatggt tagtagaaaa aggtagtcat caataccagc   4560
   tatgaccaca agaccagttg cagaaatgag acctgtaatt gtcatgtgga tttcctcctt   4620
   acatgtttgt gcatgtatac acttctacta agaaaatacc tttatttatt tcctttgctt   4680
   ttcccttatc aagtgacatt attaacttca tatcagcagt taagtgttat taactttatg   4740
   taatagcatt tcggttaata attcacttct ggttgtatga aggatagccg tattaagtta   4800
   ggtgtaatta tgacatcatt attgtcttta tttgaagatt atgtgtaatt tcaggagatg   4860
   tgtatgggtt caagttgaca agggatggac ttgtgatggc taatgttgag tgtcaacttg   4920
   actgaggatg caaagtattg ttcctgggtg tgtctgtgag ggtgttgcca aaggagatta   4980
   acatttgtgt cagtgaactg ggagatgcag acccacccgc aatctgggtg agcaccatgt   5040
   aatcagctgc cagagcagct agaataaa                                       5068
<210> 10
   <211> 535
   <212> DNA
   <213> Homo sapiens
<400> 10
   gcaaccccct ttgggtcccc ttcccttgta tgggagctct gttttcactc tatttcactc     60
   tattaaatct tgcaactgca ctcttctggt ccatgtttgt tacggctcga gctgagcttt    120
   ggctcgccat ccaccactgc tgtttgccgc cgtcgcacac ctgctgctga ctcccatccc    180
   tccggatcca gcagggtgtg tccgctgtgc tcctgatcca gcgaggtgcc cattgccgct    240
   cctgattgga ctaaaggctt gccattgttc ctgcacggct aagtgcccgg gttcgtccta    300
   atccagctga acactagtca ctgggttcca cggttctctt ccttgaccca cggcttctaa    360
   tagagctata acactcaccg catggcccaa gattccattc cttggaatct gtgaggccaa    420
   gaaccccagg tcagagaaca cgaggcttgc caccatcttg gaagcggcct gccaacatct    480
   tggaagtggc tcgccaccat cttgggagct ctgtgagcaa ggacccctgg taaca         535
<210> 11
   <211> 6905
   <212> DNA
   <213> Homo sapiens
<400> 11
   gctaccctct ttgggtcccc tccctttgta tgggagctct gttttcactc tattcaatct     60
   tgcaactgca ctcttctggt ccgtgtttgt tacagcttga gctgagcttt cgctcgcctt    120
   ccaccactgc tgtttgccgc catcgcagac ctgccgtgct gacttccatc cctctagatc    180
   tggcagggtg tccgctgtgc tcttgatcca gcgaggcgcc cattgccgct cccgattggg    240
   ctaaaggctt gcaattgttc ctgcacgcta agtgcctggg ttcatcctca tcaagctggg    300
   ttccacggtt ctcttcatga cccgcagctt ctaacagagc tataaaactc tgtgcatggc    360
   ccaagattcc attccttgga atctgtgagg ccaagaaccc caggtcagag aacaggaggc    420
   ttgccaccat cttggaagtg gctcgccacc atcttaggag ctctgtgagc ggagaccccc    480
   accccccggt aacattttgg cgaccacgaa gggacctcca aagcggtgag taatattgga    540
   tcactttcgc ttgctattct gtcctatcct tctttagaat tggaggaaaa tactgggcac    600
   ctgtcggcca gttaaaaaca attagcgtgg ctgcccgact taagactcag gtgtgaggct    660
   atctggggaa gggctttcta acaaccccca acccttctgg gttggggacg ttggtctgcc    720
   ccttccactt tcaattttct tggggaagcc aagggtcgac tagaggcaga aagctgtcgt    780
   ccggaactcc tggcagtagc cggttgagat catggcgcag ccagaagtct ctactcaaca    840
   gtcgcccatg cgtgcgctcc tacctttcct cctgacccat acctcctggg tcccgacgat    900
   gactttcttg aaagtgtagc cccaaaattc tgcttacctc tgaatctact tcccctgatc    960
   cctggctcct aggtactaat ggttcagttt catttcctct agcaagttgt atctccaaag   1020
   ggatctaagg aagctctacg ctgcgtcctt aggcatctag gctataaacc caggaagtct   1080
   tgtccctggt gtccctcccg atttaggcat acagctctcg acatgggcag ttatgtggga   1140
   cccgttcccc atcacccttg tcaaggcccc aagtttgtaa tggctaagag gagagagaga   1200
   gaaagagaga gagacggagg ggagagagag agagagagat ggaggggaga gagagagaga   1260
   gagacggagg ggagagagag agagagagag agacggaggg gagagagaga gagacggagg   1320
   ggagagagag agagatggag gagagaaaga caaagggagt caaagagaaa aagaaagaga   1380
   aagacagaaa tggtaaaaca aacaaaaaac agcgtgccct attcctttaa aagccggggt   1440
   aaatttaaaa cctataattg ataattgaag gtcttctcca tgaccctata atactccaat   1500
   actaccttgt tgtcagtgta aacaagggcg tagcctgaaa acactgagac cactgacaac   1560
   ctgcagcttt cctatcaaaa aatccttaac ccagtaaccg gcagatgcat tcaatctgta   1620
   gcagcaactg ttttgctaac agaagaaagt agaaaagtaa cttttagagg aaacctcatt   1680
   gtgagcacac cttaccagtt cagaattatt ctaagtcaaa aaagcaaaaa ggtagcttac   1740
   taactcaaaa atcttaaagt atggggctat tgtgtttaaa aaaaaaaaaa ggtaatttaa   1800
   caccaaccac tgataattct cttaacccag caggtttcct aacaggggat ttaaatctta   1860
   attaccatac aaaggtctga ccacacctag gaggaactcc cttcaggaca ggactataga   1920
   gggttcctcc caggtgattg aggaaaaaac cacagtgggt attcagtaat tgatagggag   1980
   actcttgtgg aagcagagtt agaaaaattg cctaataaat ggtgtcctca aaagtgtgag   2040
   ctgtttgcac tcagccaagc cttaaagtac ttacagaatc gtaaaaacta tctcaatcct   2100
   gactcaaaag tttacttaca ccctctctga aatgaattta cataagaact gcttttttgg   2160
   gaatgcatct tgatggggca gctgggtggt tatgaaatac tcaggaaacc agcccagctc   2220
   taggacacat ccctgagcac aaaggcaatg ttgggcacgc tggtaaagga ccactagaat   2280
   ccagcagcct ggactccttt ctttgtggtc aagaaaggca ggaaaacagg tgcaggactg   2340
   ctacatcagt gagcataact aatctgataa gcagagggcc ttgggtggtt acacaccctg   2400
   gaaaggaatt caactctgag cgcaaaggca atgttgggca cattggtaaa ggaccactag   2460
   aatccagcag cccaggcccc tttctttatg gtcaagaaag gcgggaaaag gggtgcagga   2520
   ctgttacctc ggtgagcgta actaatccga taagcagagg tccatgggtg attacgcacc 2580
   ctgaaaagaa taagcattag gcccttaaag gatgctctag gactaatgct cattggaaaa 2640
   tgactagggg tgctggcatc cctatgttct tttctcagac gggaaatgtt ctccaccctc 2700
   cccaaggcaa aaacacccct aagatgtatt ctggagaatt gggaccaatt tgacccccag 2760
   acgctaagaa agagatgact tatgttcttc tgcagtacca cctggccacg atatcctctt 2820
   caagggggag aaacctggcc tcctgaggga agtataaatt ataacaccat cttacagcta 2880
   gacctcttct gtagaaagga gggcaaatgg agtgaagtgc catatgtgca aactttcttt 2940
   tcattaagag acaacttgca attatgtaag aagtgtgatt tatgccctac aggaagccct 3000
   cagagtctac ctccctaccc cagcatcccc ctgactcctt ctccaactaa taaggaaccc 3060
   ccttcaaccc aaacggtcca aaaggagata gacaaagggg taaacaatga accaaagcgt 3120
   gccaatgttc cctgattatg ccccctctaa gcagtgggag gaggagaatt tggcccagcc 3180
   agtgtgcatg tgcctttttc tctctcagac ttaaagcaaa ttaaaataga cctaggtaaa 3240
   ttctcagata accctgatgg ctatattgat gttttataag ggttaggata atcctttgat 3300
   ctgacatgga gagatataat gttactgcta gatcagacac taaccccaaa tgagacaagt 3360
   gccgccataa ctgcagcctg agagtttggc gatctctggt atctcactcg ggtcaatgat 3420
   aggaggacaa cagaggaaag agaatgattc cccacagacc agcaggcagt tcccagtgta 3480
   gaccctcact gggacacaga atcagaacat ggacattggt gctgcagaca tttgctaact 3540
   tacatgctag aaggactaag gaaaactagg aagaagccta cgaattattc aatgatgtcc 3600
   actataacac agggaaagga agaaaatcct actgcctttc tggagcgact aagggaggca 3660
   ttgaggaagc atacttccct gtcacctgac tctattgaag gccaactaat cttaaaggat 3720
   aagtttatca ctcagtcagc tgaagacatt aggaaaaaac ttcaaaagtc tgccttaggc 3780
   ccagagcaaa acttagaaac cccattgaac ttggcaacct cggtttttta taatagagat 3840
   caggaggagc aggcggaaca ggacaaacgg ggtaaaaaaa aggccaccgc tttagttatg 3900
   gccctcaggc aagtggactt tggaggctct ggaaaaggga aaagctgggc aaatcgaatg 3960
   cctactaggg cttgcttcca gagtggtcta caaggacact ttgaaaaaga ttgtccaagt 4020
   agaaataagt cgccccttcg tccatgcccc ttatatcaag ggaatcactg gaaggcccac 4080
   tatcccaggg gacaaatgtc ctctgagtca gaagccacta accagatgat ccagcagcag 4140
   gactgagggt gcccagggca agcactagcc catgccgtca ccctcacaga gccccaggta 4200
   tgcttgacca ttgagggcca ggaggttaac tgtctcctgg acactagcac ggccttctca 4260
   gtcttactct cctttcccgg acaactgtcc tccagatctg tcactatccg agggttccta 4320
   ggacagtcag tcactagata cttatcccag tcactaagtt gtgactggtg aactttactc 4380
   ttttcacatg cttttctaat tatccctgaa agcaccactc ccttgttagg gcgagacatt 4440
   ctagcaaaag caggggccat tatacacctg aacataggag aaggaacacc tgtttgttgt 4500
   cccctgcttg aggaaggaat taatcccgaa gtctgggcaa cagaaggaca atacggacga 4560
   gcaaagaatg cctgtgctgt tcaagttaaa ctaaaggatt ccgcctcctt tccctaccaa 4620
   aggcagtacc cccttagacc tgaggcccaa caaggactcc aaaagattgt taaggaccta 4680
   aaagcccatg gcctagtaaa accatgcaat agcccctgca atactccaat tttaggagta 4740
   cagaaaccca acagacagtg gaggttagtg caagatctca ggattatcat tgaggctgtt 4800
   gttcctgtat agccagctgt acctaaccct tatactctgc tttcccaaat accacaggaa 4860
   gcagaggggt ttacagtccg gggccttaag gacacctttt tctgcatccc tgtatatcct 4920
   gactctcaat tcttgtttgc ctttgaagat ccttcaaact caacgtctca actcacctgg 4980
   aatgttttac cccaagggtt cagggatagc ccccattagc ccaagacttg agccagttct 5040
   tatacctgga cactcttgtc ctttggtacg tggatgattt acttttagcc acctgttcag 5100
   aaaccttgtg ccatcaagcc acccaagcac tctttaattt cctcgccacc tgtggctaca 5160
   ggtttccaaa ccaaaggctc agctctgctc acagcaattt aaatgcttag ggctaaaatt 5220
   atccaaaggc accagggccc tcagtgagga aagtatccgg cctatactgg cttatcctca 5280
   tcccaaaacc ctaaagcaac taagagtgtt ccttggcata acgggtttct gccgaatatg 5340
   gattcccagg tacagcgaaa tagccagacc attatataca ctaattaagg aaactcagaa 5400
   agccaatacc catttggtaa gatggacacc tgaagcagaa gcagatttcc aggccctaaa 5460
   gaaggccctg acccaagccc cagtgttaag cttgccaatg gggcaagact tttctttata 5520
   tgtcacagaa aaaacaggaa tagctccagg agtccttacg cagatccaag ggacgagcct 5580
   gcaacccatg gcatacctga gtaaggaaat tagtggcaaa gggttggcct cattgtttat 5640
   gggtagtggc agcagtcaca gtcttagtaa ctgaagcagt taaaatgata caaggaagag 5700
   atcttactgt gtggacatct catgatgtga atggcatact cactgctaaa ggagacttgt 5760
   gactgtcaga caactgttta cttaaatatc aggctctatt acttgaaggg ccagtgttgc 5820
   gactgtgcac ttgtgcaact cttaacccag ccacattgct tccagacaat gaagaaaaga 5880
   tagaacataa ctgtcaacaa ataattgctc aaacctacac tgctcgaggg gaccttttag 5940
   aagttccctt gactgatccc gatctcaact tgtatactga tggaagttcc tttgcagaaa 6000
   aaggacttca aaaggcggtg tatgcagtag tccttcaaaa tcgaagagct ttagaattgc 6060
   taatcactga gagaggggga acgtttttat ttttagggga agaatgctgt tattatgtta 6120
   atcaattcgg aatcatcacc aagaaagtta aagaaattca agatcgaata caacgtagaa 6180
   cagaggagct taaaaaacac tggaccctgg ggcctcctca gccaatggat gccctggatt 6240
   ctccccttct taggacctct agcagctata tttctactcc tctttggacc ctgtatcttt 6300
   aacctccgtg ttaagtttgt ctcttccaga atcgaagatg taaaactaca aatcgttctt 6360
   caaatggacc cccagatgca gtccatgact aagatctact gaggacccct ggaccagcct 6420
   gctagcccat gctccaatgt taatgacatt gaaggcaccc ctcccaagga aatctcaact 6480
   gcacaacccc tactatgctc caattcagca ggaagcagtt acagtggtcc tcggccaacc 6540
   tccccaacag catttgtatt ttcctgttgg gagggggcac tgagagacag gactagctgg 6600
   atttcctagg ctgactgaga atccctaagc ctagctggga aggtgaccac ttccaccttt 6660
   aaacacaggg cttgcaactt agctcacacc ctaccaattg gatagtaaag agaggtcact 6720
   aaaatgctaa ttaggcaaaa acaggaggta aagaaatagc caatcatcca ttgcctgaga 6780
   gcacagcggg agggacaatg accaggatat aaacccaggc attccagcct gcaacggcaa 6840
   ccccctttgg gtcccctctc tttgtatggg agctctgttt tcactctatt caatcttgca 6900
   actgc 6905
<210> 12
   <211> 9565
   <212> DNA
   <213> Homo sapiens
<400> 12
   gcagcccccc tttgggtccc ttccctttgt atgggagctg ttttcatgct atttcactct      60
   attaaatctt gcaactgcac tcttctggtc catgtttctt acggctcgag ctgagctttt     120
   gctcaccgtc caccactgct gtttgccacc accgcagacc tgccgctgac tcccatccct     180
   ctggatcctg cagggtgtcc gctgtgctcc tgatccagcg aggcgcccat tgccgctccc     240
   aattgggcta aaggcttgcc attgttcctg cacggctaag tgcctgggtt tgttctaatt     300
   gagctgaaca ctagtcactg ggttccatgg ttctcttctg tgacccacgg cttctaatag     360
   aactataaca cttaccacat ggcccaagat tccattcctt ggaatccgtg aggccaagaa     420
   ctccaggtca gagaatacga ggcttgccac catcttggaa gcggcctgct accatcttgg     480
   aagtggttca ccaccatctt gggagctctg tgagcaagga ccccccggta acattttggc     540
   aaccacgaac ggacatccaa agtggtgagt aatattggac cactttcact tgctattctg     600
   tcctatcctt ccttagaatt ggaggaaaat accgggcact tgtcggccag ttaaaaacga     660
   ttagtgtggc caccggactt aagactcagg tgtgaggcta tctggggaag ggctttctaa     720
   caacccccaa cccttctggg ttggggactt ggtttgcctc aagccagctt ccactttcag      780
   ttttcttggg gaagccgagg gccgactaga ggcagaaagc tgtcgtcctg aactcccggc     840
   agtagccggt tgagatcatg gtgtagccag aagtctcaac agtcgcccat gcatgcaccc     900
   ctatctttcc ttctgaccca tacctcctgg gtcccaacca caactttctt caaagtgtag     960
   ccccaaaatt ctccttacct ctgaatatac ttcctctgat ccctgcctcc taggtactat    1020
   tggttcagac ttccatttcc tctagcaagt tgtatctcca aagggatcta aggaagctct    1080
   gcgctgcgtc cttaggcacc taggctataa cccagggagt cttatccctg gtgtccctcc    1140
   caatttaggc atacagctct tgacatgggc agttatgtag gacccactcc ccaccaccct    1200
   tgccagggcc ccaagtttgt aaatggctga gggaaaagag agacagagga gagagagaga   1260
   aatggaggag aaagagagag agacagagag gagagagaga cagtgagaga gacagaagag   1320
   agagagagac aaagaggaga gagagagagt caaagagaga aagaaagaga aagaaatagt   1380
   aaaaaacagt gtgccctatt cctttaaaag ccagggtaaa tttaaaacct gtacttgata   1440
   attgaaggtc ttctctgtga ccctatagca ctccaatcca ctttgtggtc agtgtaaata   1500
   agagcatagg ccgaaagcac tgaggccatt gacaacccgt agcttcccta tcaaaaatcc   1560
   ttaacccagt aacccgcaga tggaccaaat gcattcagtc ggtagcgcaa ctgctttgct   1620
   aaaagtagaa aagtaacttt tagaggaaac ctcattgtga gcacacctca cctgttcaga   1680
   attattctaa taaaaaaagc aaaaaggtag cttactaact caaaaatctt aaagtatggg   1740
   gctattctgt tagaaaaagg taatgtaact ccaaccactg ataattccct taacccagca   1800
   gatttcctaa cgggatttaa atcttaatta ccatacaaag gtccgaccag acctaggcgg   1860
   aactcccttc aggacaggac gatagatggt tcctcccagg tgattgagga aaaaaaccac   1920
   aatgggtatt cagtaattga tacggggact cttgtggaag cagagttaga aaaattgcct   1980
   aataactggt ctcctcaaac gtgtgagctg tttgcactca gccaagcctt aaagtactta   2040
   cagaatcaaa agactatctc aatcctgatt caaaaggtta gctacaccct ctctgtaatg   2100
   catttgcata agaacttgtt tatgggaatg catcttgatg gggcagctgg gttgttataa   2160
   aataggaacc cagcccagct ctaggactca cccctgagcg caaaggcaat gttgggcatg   2220
   ctggtaaagg accactagaa tccagcagcc cagacccctt tctttgtggt caagaaaggc   2280
   gggaaaaggg gtgcaggact gctacatcgg taagcataac taatccgata aacagaggtc   2340
   catgggtggt tacgcaccct ggaaaggaac tcacccctga gcacaaaggc aatgttgggc   2400
   acgctggtaa aggaccacta gaatccagca gcctggaccc ctttctttgt ggtcaagaga   2460
   ggcaggaaaa caggtgcagg actgcaacat cagtgagcat aactaattcg ataagcagag   2520
   gtccatgggt ggtgatgcac cctggaaaga ataagcatta ggaccataga ggacactcca   2580
   ggactaaagc tcatcggaaa atgactaggg ttgctggcat ccctatgttc ttttttcaga   2640
   tgggaaacgt tccccgcaag acaaaaacgc ccctaagacg tattctggag aattgggacc   2700
   aatttgaccc tcagacacta agaaagaaac gacttatatt cttctgcagt gccgcctggc   2760
   actcctgagg gaagtataaa ttataacacc atcttacagc tagacctctt ttgtagaaaa   2820
   ggcaaatgga gtgaagtgcc ataagtacaa actttctttt cattaagaga caactcacaa   2880
   ttatgtaaaa agtgtgattt atgccctaca ggaagccttc agagtctacc tccctatccc   2940
   agcatccccg actccttccc caactaataa ggacccccct tcaacccaaa tggtccaaaa   3000
   ggagatagac aaaagggtaa acagtgaacc aaagagtgcc aatattcccc aattatgacc   3060
   cctccaagca gtgggaggaa gagaattcgg cccagccaga gtgcatgtgc ctttttctct   3120
   cccagactta aagcaaataa aaacagactt aggtaaattc tcagataacc ctgatggcta 3180
   tattgatgtt ttacaagggt taggacaatt ctttgatctg acatggagag atataatgtc 3240
   actgctaaat cagacactaa ccccaaatga gagaagtgcc accataactg cagcctgaga 3300
   gtttggcgat ctctggtatc tcagtcaggt caatgatagg atgacaacag aggaaagaga 3360
   atgattcccc acaggccagc aggcagttcc cagtctagac cctcattggg acacagaatc 3420
   agaacatgga gattggtgct gcagacattt gctaacttgt gtgctagaag gactaaggaa 3480
   aactaggaag aagtctatga attactcaat gatgtccacc ataacacagg gaagggaaga 3540
   aaatcctact gcctttctgg agagactaag ggaggcattg aggaagcgtg cctctctgtc 3600
   acctgactct tctgaaggcc aactaatctt aaagcgtaag tttatcactc agtcagctgc 3660
   agacattaga aaaaaacttc aaaagtctgc cgtaggcccg gagcaaaact tagaaaccct 3720
   attgaacttg gcaacctcgg ttttttataa tagagatcag gaggagcagg cggaacagga 3780
   caaacgggat taaaaaaaag gccaccgctt tagtcatgac cctcaggcaa gtggactttg 3840
   gaggctctgg aaaagggaaa agctgggcaa attgaatgcc taatagggct tgcttccagt 3900
   gcggtctaca aggacacttt aaaaaagatt gtccaagtag aagtaagccg ccccctcgtc 3960
   catgcccctt atttcaaggg aatcactgga aggcccactg ccccagggga caaaggtcct 4020
   ctgagtcaga agccactaac cagatgatcc agcagcagga ctgagggtgc ctggggcaag 4080
   cgccatccca tgccatcacc ctcacagagc cctgggtatg cttgaccatt gagggccagg 4140
   aggttgtctc ctggacactg gtgcggtctt cttagtctta ctcttctgtc ccggacaact 4200
   gtcctccaga tctgtcacta tctgaggggg tcctaagacg ggcagtcact agatacttct 4260
   cccagccact aagttatgac tggggagctt tattcttttc acatgctttt ctaattatgc 4320
   ttgaaagccc cactaccttg ttagggagag acattctagc aaaagcaggg gccattatac 4380
   acctgaacat aggagaagga acacccgttt gttgtcccct gcttgaggaa ggaattaatc 4440
   ctgaagtctg ggcaacagaa ggacaatatg gacgagcaaa gaatgcccgt cctgttcaag 4500
   ttaaactaaa ggattccacc tcctttccct accaaaggca gtaccccctc agacccaagg 4560
   cccaacaagg actccaaaag attgttaagg acctaaaagc ccaaggccta gtaaaaccat 4620
   gcagtaaccc ctgcagtact ccaattttag gagtacagaa acccaacaga cagtggaggt 4680
   tagtgcaaga tctcaggatt atcaatgagg ctgttgttcc tctatagcca gctgtaccta 4740
   gcccttatac tctgctttcc caaataccag aggaagcaga gtggtttaca gtcctggacc 4800
   ttcaggatgc cttcttctgc atccctgtac atcctgactc tcaattcttg tttgcctttg 4860
   aagatacttc aaacccaaca tctcaactca cctggactat tttaccccaa gggttcaggg 4920
   atagtcccca tctatttggc caggcattag cccaagactt gagccaatcc tcatacctgg 4980
   acacttgtcc ttcggtaggt ggatgattta cttttggccg cccattcaga aaccttgtgc 5040
   catcaagcca cccaagcgct cttcaatttc ctcgctacct gtggctacat ggtttccaaa 5100
   ccaaaggctc aactctgctc acagcaggtt acttagggct aaaattatcc aaaggcacca 5160
   gggccctcag tgaggaacac atccagccta tactggctta tcctcatccc aaaaccctaa 5220
   agcaactaag gggattcctt ggcgtaatag gtttctgccg aaaatggatt cccaggtatg 5280
   gcgaaatagc caggtcatta aatacactaa ttaaggaaac tcagaaagcc aatacccatt 5340
   tagtaagatg gacaactgaa gtagaagtgg ctttccaggc cctaacccaa gccccagtgt 5400
   taagtttgcc aacagggcaa gacttttctt catatgtcac agaaaaaaca ggaatagctc 5460
   taggagtcct tacacagatc cgagggatga gcttgcaacc tgtggcatac ctgactaagg 5520
   aaattgatgt agtggcaaag ggttgacctc attgtttacg ggtagtggtg gcagtagcag 5580
   tcttagtatc tgaagcagtt aaaataatac agggaagaga tcttactgtg tggacatctc 5640
   atgatgtgaa tggcatactc actgctaaag gagacttgtg gctgtcagac aactgtttac 5700
   ttaaatgtca ggctctatta cttgaagggc cagtgctgcg actgtgcact tgtgcaactc 5760
   ttaacccagc cacatttctt ccagacaatg aagaaaagat aaaacataac tgtcaacaag 5820
   taatttctca aacctatgcc actcgagggg accttttaga ggttcctttg actgatcccg 5880
   acctcaactt gtatactgat ggaagttcct ttgtagaaaa aggacttcga aaagtggggt 5940
   atgcagtggt cagtgataat ggaatacttg aaagtaatcc cctcactcca ggaactagtg 6000
   ctcagctagc agaactaata gccctcactt gggcactaga attaggagaa gaaaaaaggg 6060
   caaatatata tacagactct aaatatgctt acctagtcct ccatgcccat gcagcaatat 6120
   ggaaagaaag ggaattccta acttctgaga gaacacctat caaacatcag gaagccatta 6180
   ggaaattatt attggctgta cagaaaccta aagaggtggc agtcttacac tgccggggtc 6240
   atcagaaagg aaaggaaagg gaaatagaag agaactgcca agcagatatt gaagccaaaa 6300
   gagctgcaag gcaggaccct ccattagaaa tgcttataaa acaaccccta gtatagggta 6360
   atcccctccg ggaaaccaag ccccagtact cagcaggaga aacagaatgg ggaacctcac 6420
   gaggacagtt ttctcccctc gggacggcta gccactgaag aagggaaaat acttttgcct 6480
   gcaactatcc aatggaaatt acttaaaacc cttcatcaaa cctttcactt aggcatcgat 6540
   agcacccatc agatggccaa atcattattt actggaccag gccttttcaa aactatcaag 6600
   cagatagtca gggcctgtga agtgtgccag agaaataatc ccctgcctta tcgccaagct 6660
   ccttcaggag aacaaagaac aggccattac cctggagaag actggcaact gattttaccc 6720
   acaagcccaa acctcaggga tttcagtatc tactagtctg ggtagatact ttcacgggtt 6780
   gggcagaggc cttcccctgt aggacagaaa aggcccaaga ggtaataaag gcactagttc 6840
   atgaaataat tcccagattc ggacttcccc gaggcttaca gagtgacaat agccctgctt 6900
   tccaggccac agtaacccag ggagtatccc aggcgttagg tatacgatat cacttacact 6960
   gcgcctgaag gccacagtcc tcagggaagg tcgagaaaat gaatgaaaca ctcaaaggac 7020
   atctaaaaaa gcaaacccag gaaacccacc tcacatggcc tgctctgttg cctatagcct 7080
   taaaaagaat ctgcaacttt ccccaaaaag caggacttag cccatacgaa atgctgtatg 7140
   gaaggccctt cataaccaat gaccttgtgc ttgacccaag acagccaact tagttgcaga 7200
   catcacctcc ttagccaaat atcaacaagt tcttaaaaca ttacaaggaa cctatccctg 7260
   agaagaggga aaagaactat tccacccttg tgacatggta ttagtcaagt cccttccctc 7320
   taattcccca tccctagata catcctggga aggaccctac ccagtcattt tatctacccc 7380
   aactgcggtt aaagtggctg gagtggagtc ttggatacat cacacttgag tcaaatcctg 7440
   gatactgcca aaggaacctg aaaatccagg agacaacgct agctattcct gtgaacctct 7500
   agaggatttg cgcctgctct tcaaacaaca accaggagga aagtaactaa aatcataaat 7560
   ccccatggcc ctcccttatc atatttttct ctttactgtt cttttaccct ctttcactct 7620
   cactgcaccc cctccatgcc gctgtatgac cagtagctcc ccttaccaag agtttctatg 7680
   gagaatgcag cgtcccggaa atattgatgc cccatcgtat aggagtcttt ctaagggaac 7740
   ccccaccttc actgcccaca cccatatgcc ccgcaactgc tatcactctg ccactctttg 7800
   catgcatgca aatactcatt attggacagg aaaaatgatt aatcctagtt gtcctggagg 7860
   acttggagtc actgtctgtt ggacttactt cacccaaact ggtatgtctg atgggggtgg 7920
   agttcaagat caggcaagag aaaaacatgt aaaagaagta atctcccaac tcacccgggt 7980
   acatggcacc tctagcccct acaaaggact agatctctca aaactacatg aaaccctccg 8040
   tacccatact cgcctggtaa gcctatttaa taccaccctc actgggctcc atgaggtctc 8100
   ggcccaaaac cctactaact gttggatatg cctccccctg aacttcaggc catatgtttc 8160
   aatccctgta cctgaacaat ggaacaactt cagcacagaa ataaacacca cttccgtttt 8220
   agtaggacct cttgtttcca atctggaaat aacccatacc tcaaacctca cctgtgtaaa 8280
   atttagcaat actacataca caaccaactc ccaatgcatc aggtgggtaa ctcctcccac 8340
   acaaatagtc tgcctaccct caggaatatt ttttgtctgt ggtacctcag cctatcgttg 8400
   tttgaatggc tcttcagaat ctatgtgctt cctctcattc ttagtgcccc ctatgaccat 8460
   ctacactgaa caagatttat acagttatgt catatctaag ccccgcaaca aaagagtacc 8520
   cattcttcct tttgttatag gagcaggagt gctaggtgca ctaggtactg gcattggcgg 8580
   tatcacaacc tctactcagt tctactacaa actatctcaa gaactaaatg gggacatgga 8640
   acgggtcgcc gactccctgg tcaccttgca agatcaactt aactccctag cagcagtagt 8700
   ccttcaaaat cgaagagctt tagacttgct aaccgctgaa agagggggaa cctgtttatt 8760
   tttaggggaa gaatgctgtt attatgttaa tcaatccgga atcgtcactg agaaagttaa 8820
   agaaattcga gatcgaatac aacgtagagc agaggagctt cgaaacactg gaccctgggg 8880
   cctcctcagc caatggatgc cctggattct ccccttctta ggacctctag cagctataat 8940
   attgctactc ctctttggac cctgtatctt taacctcctt gttaactttg tctcttccag 9000
   aatcgaagct gtaaaactac aaatggagcc caagatgcag tccaagacta agatctaccg   9060
   cagacccctg gaccggcctg ctagcccacg atctgatgtt aatgacatca aaggcacccc   9120
   tcctgaggaa atctcagctg cacaacctct actacgcccc aattcagcag gaagcagtta   9180
   gagcggtcgt cggccaacct ccccaacagc acttaggttt tcctgttgag atgggggact   9240
   gagagacagg actagctgga tttcctaggc tgactaagaa tccctaagcc tagctgggaa   9300
   ggtgaccaca tccaccttta aacacggggc ttgcaactta gctcacacct gaccaatcag   9360
   agagctcact aaaatgctaa ttaggcaaag acaggaggta aagaaatagc caatcatcta   9420
   ttgcctgaga gcacagcagg agggacaatg atcgggatat aaacccaagt cttcgagccg   9480
   gcaacggcaa ccccctttgg gtcccctccc tttgtatggg agctctgttt tcatgctatt   9540
   tcactctatt aaatcttgca actgc                                         9565
<210> 13
   <211> 543
   <212> DNA
   <213> Homo sapiens
<400> 13
   atgacaacag aagaaagaaa acaattcccc acaggccagc aggcagttcc cagcgtagac     60
   cttcattggg acacagaatc agaacatgga gattggtgcc gcagacattt actaacttgc    120
   gcgctagaag cactaaggaa aactaggaag aagcctatga attattcaat gatgtccact    180
   ataacacagg gaaaggaaga aaatcctact gcctttctgg agagactaag ggaggcattg    240
   agaaagcata cctctctgtc acctgactct attgaaggcc aactaatctt aaaggataag    300
   ttttccactc agtcagctgc agacattaga aaaaaacttc aaaagtctgc gttaggccgg    360
   gagcaaaact tagaaaccct attgaacttg gcaacctcag ttttttatga tagagatcag    420
   gaggatcagg tggaatggac aaatgagatt ttaaaaaaag gccaccactt tagtcatggc    480
   cctcaggcaa gcagactttg gacactctgg aaaagggaaa agctgggcaa atcgaatgcc    540
   taa                                                                  543
<210> 14
   <211> 180
   <212> PRT
   <213> Homo sapiens
<400> 14
   Met Thr Thr Glu Glu Arg Lys Gln Phe Pro Thr Gly Gln Gln Ala Val
   1               5                   10                  15
   Pro Ser Val Asp Leu His Trp Asp Thr Glu Ser Glu His Gly Asp Trp
               20                  25                  30
   Cys Arg Arg His Leu Leu Thr Cys Ala Leu Glu Ala Leu Arg Lys Thr
           35                  40                  45
   Arg Lys Lys Pro Met Asn Tyr Ser Met Met Ser Thr Ile Thr Gln Gly
       50                  55                  60
   Lys Glu Glu Asn Pro Thr Ala Phe Leu Glu Arg Leu Arg Glu Ala Leu
   65                  70                  75                  80
   Arg Lys His Thr Ser Leu Ser Pro Asp Ser Ile Glu Gly Gln Leu Ile
                   85                  90                  95
   Leu Lys Asp Lys Phe Ser Thr Gln Ser Ala Ala Asp Ile Arg Lys Lys
               100                 105                 110
   Leu Gln Lys Ser Ala Leu Gly Arg Glu Gln Asn Leu Glu Thr Leu Leu
           115                 120                 125
   Asn Leu Ala Thr Ser Val Phe Tyr Asp Arg Asp Gln Glu Asp Gln Val
       130                 135                 140
   Glu Trp Thr Asn Glu Ile Leu Lys Lys Gly His His Phe Ser His Gly
   145                 150                 155                 160
   Pro Gln Ala Ser Arg Leu Trp Thr Leu Trp Lys Arg Glu Lys Leu Gly
                   165                 170                 175
   Lys Ser Asn Ala
               180
<210> 15
   <211> 435
   <212> DNA
   <213> Homo sapiens
<400> 15
   atgattcagc cccaggactc agggtgccca gggcaagcgc cagcctatgc catcaccctc      60
   acagagccct gggtatgctt gaccattgag ggtcaggagg ttaactatct cctggacact     120
   ggcgtggcct tctcagtctt actctcctgt cccggacaac tgtcctccag atctgtcact     180
   atccgagggt ttctacgaca gccagccact agatacttct cccagccact aagttgtgac     240
   tggggaactc tactcttttc acatgttttt ctaattatgc ctgaaagccc cactcctttg     300
   ttagggaaag acattctagc aaaagcaggg gccattatac acctgaacat aggagaagga     360
   acacctgttt gttgtcccct gcttgaagaa ggaattaatc ctgaagtctg gacaacagaa     420
   ggacaataca gatga                                                      435
<210> 16
   <211> 144
   <212> PRT
   <213> Homo sapiens
<400> 16
   Met Ile Gln Pro Gln Asp Ser Gly Cys Pro Gly Gln Ala Pro Ala Tyr
   1               5                   10                  15
   Ala Ile Thr Leu Thr Glu Pro Trp Val Cys Leu Thr Ile Glu Gly Gln
               20                  25                  30
   Glu Val Asn Tyr Leu Leu Asp Thr Gly Val Ala Phe Ser Val Leu Leu
           35                  40                  45
   Ser Cys Pro Gly Gln Leu Ser Ser Arg Ser Val Thr Ile Arg Gly Phe
       50                  55                  60
   Leu Arg Gln Pro Ala Thr Arg Tyr Phe Ser Gln Pro Leu Ser Cys Asp
   65                  70                  75                  80
   Trp Gly Thr Leu Leu Phe Ser His Val Phe Leu Ile Met Pro Glu Ser
                   85                  90                  95
   Pro Thr Pro Leu Leu Gly Lys Asp Ile Leu Ala Lys Ala Gly Ala Ile
               100                 105                 110
   Ile His Leu Asn Ile Gly Glu Gly Thr Pro Val Cys Cys Pro Leu Leu
           115                 120                 125
   Glu Glu Gly Ile Asn Pro Glu Val Trp Thr Thr Glu Gly Gln Tyr Arg
       130                 135                 140
<210> 17
   <211> 312
   <212> DNA
   <213> Homo sapiens
<400> 17
   atgccccctc caggcagtgg gaggaggaga attcggccca gccagagtgc atgtaccttt     60
   ttttttctct cagacttaaa gcaaattaaa atagacctag gtaaattctc agataaccct     120
   gatggctata ttgatgtttt acaagggtta ggacaatcct ttgctctgac atggagagat    180
   ataatgttac tgctaaatca gacactaacc ccaaatgaga gaagtgtcac catagctgca    240
   gcccaagagt ttggcaatct ctggtatctc agtcaggtca atgataggat gacaacagag    300
   gaaagggaat ga                                                         312
<210> 18
   <211> 103
   <212> PRT
   <213> Homo sapiens
<400> 18
<210> 19
   <211> 207
   <212> DNA
   <213> Homo sapiens
<400> 19
   atgggcagtt atgtaggacc cattccccac cacacttgcc agggccccaa gtttgtaatg      60
   gctaagagag agacacagag agagagagag agatggagag agagacaagg agggagtcaa     120
   agagaaaaag aaagaaaaag aaatagtaga aaaaaaagtg tgccctattc ctttaaaagc     180
   cagggtaaat ttaaaacctg taattga                                         207
<210> 20
   <211> 68
   <212> PRT
   <213> Homo sapiens
<400> 20
<210> 21
   <211> 306
   <212> DNA
   <213> Homo sapiens
<400> 21
   atgtcaagaa agaaacgact tatattcttc tgcagtactg cctggccacg atatcctctt      60
   caagggggag aaacctggcc tcctgaggga agtacaaatt ataacaccat cttacagcta     120
   gacctctttt gtagaaaaga aggcaaatgg agtgaagtgc catatgtgca aactttcttt     180
   tcattaagag acaactcaca attatgtaaa aagtgtggtt tatgtcttac aggaagccct     240
   cagagtctac ctccctatcc cagcattccc ccgactcctt ccccaactaa taagcaccac     300
   ccttga                                                                306
<210> 22
   <211> 101
   <212> PRT
   <213> Homo sapiens
<400> 22
   Met Ser Arg Lys Lys Arg Leu Ile Phe Phe Cys Ser Thr Ala Trp Pro
   1               5                   10                  15
   Arg Tyr Pro Leu Gln Gly Gly Glu Thr Trp Pro Pro Glu Gly Ser Thr
               20                  25                  30
   Asn Tyr Asn Thr Ile Leu Gln Leu Asp Leu Phe Cys Arg Lys Glu Gly
           35                  40                  45
   Lys Trp Ser Glu Val Pro Tyr Val Gln Thr Phe Phe Ser Leu Arg Asp
       50                  55                  60
   Asn Ser Gln Leu Cys Lys Lys Cys Gly Leu Cys Leu Thr Gly Ser Pro
   65                  70                  75                  80
   Gln Ser Leu Pro Pro Tyr Pro Ser Ile Pro Pro Thr Pro Ser Pro Thr
                   85                  90                  95
   Asn Lys His His Pro
               100
<210> 23
   <211> 603
   <212> DNA
   <213> Homo sapiens
<400> 23
   atgctagaag gactaaggaa aactaggaag aagcctacga attattcaat gatgtccact     60
   ataacacagg gaaaggaaga aaatcctact gcctttctgg agcgactaag ggaggcattg    120
   aggaagcata cttccctgtc acctgactct attgaaggcc aactaatctt aaaggataag    180
   tttatcactc agtcagctga agacattagg aaaaaacttc aaaagtctgc cttaggccca    240
   gagcaaaact tagaaacccc attgaacttg gcaacctcgg ttttttataa tagagatcag    300
   gaggagcagg cggaacagga caaacggggt aaaaaaaagg ccaccgcttt agttatggcc    360
   ctcaggcaag tggactttgg aggctctgga aaagggaaaa gctgggcaaa tcgaatgcct    420
   actagggctt gcttccagag tggtctacaa ggacactttg aaaaagattg tccaagtaga    480
   aataagtcgc cccttcgtcc atgcccctta tatcaaggga atcactggaa ggcccactat    540
   cccaggggac aaatgtcctc tgagtcagaa gccactaacc agatgatcca gcagcaggac    600
   tga                                                                  603
<210> 24
   <211> 200
   <212> PRT
   <213> Homo sapiens
<400> 24
   Met Leu Glu Gly Leu Arg Lys Thr Arg Lys Lys Pro Thr Asn Tyr Ser
   1               5                   10                  15
   Met Met Ser Thr Ile Thr Gln Gly Lys Glu Glu Asn Pro Thr Ala Phe
               20                  25                  30
   Leu Glu Arg Leu Arg Glu Ala Leu Arg Lys His Thr Ser Leu Ser Pro
           35                  40                  45
   Asp Ser Ile Glu Gly Gln Leu Ile Leu Lys Asp Lys Phe Ile Thr Gln
       50                  55                  60
   Ser Ala Glu Asp Ile Arg Lys Lys Leu Gln Lys Ser Ala Leu Gly Pro
   65                  70                  75                  80
   Glu Gln Asn Leu Glu Thr Pro Leu Asn Leu Ala Thr Ser Val Phe Tyr
                   85                  90                  95
   Asn Arg Asp Gln Glu Glu Gln Ala Glu Gln Asp Lys Arg Gly Lys Lys
               100                 105                 110
   Lys Ala Thr Ala Leu Val Met Ala Leu Arg Gln Val Asp Phe Gly Gly
           115                 120                 125
   Ser Gly Lys Gly Lys Ser Trp Ala Asn Arg Met Pro Thr Arg Ala Cys
       130                 135                 140
   Phe Gln Ser Gly Leu Gln Gly His Phe Glu Lys Asp Cys Pro Ser Arg
   145                 150                 155                 160
   Asn Lys Ser Pro Leu Arg Pro Cys Pro Leu Tyr Gln Gly Asn His Trp
                   165                 170                 175
   Lys Ala His Tyr Pro Arg Gly Gln Met Ser Ser Glu Ser Glu Ala Thr
               180                 185                 190
   Asn Gln Met Ile Gln Gln Gln Asp
           195                 200
<210> 25
   <211> 1617
   <212> DNA
   <213> Homo sapiens
<400> 25
   atggccctcc cttatcatat ttttctcttt actgttcttt taccctcttt cactctcact     60
   gcaccccctc catgccgctg tatgaccagt agctcccctt accaagagtt tctatggaga    120
   atgcagcgtc ccggaaatat tgatgcccca tcgtatagga gtctttctaa gggaaccccc    180
   accttcactg cccacaccca tatgccccgc aactgctatc actctgccac tctttgcatg    240
   catgcaaata ctcattattg gacaggaaaa atgattaatc ctagttgtcc tggaggactt    300
   ggagtcactg tctgttggac ttacttcacc caaactggta tgtctgatgg gggtggagtt    360
   caagatcagg caagagaaaa acatgtaaaa gaagtaatct cccaactcac ccgggtacat    420
   ggcacctcta gcccctacaa aggactagat ctctcaaaac tacatgaaac cctccgtacc    480
   catactcgcc tggtaagcct atttaatacc accctcactg ggctccatga ggtctcggcc    540
   caaaacccta ctaactgttg gatatgcctc cccctgaact tcaggccata tgtttcaatc    600
   cctgtacctg aacaatggaa caacttcagc acagaaataa acaccacttc cgttttagta    660
   ggacctcttg tttccaatct ggaaataacc catacctcaa acctcacctg tgtaaaattt    720
   agcaatacta catacacaac caactcccaa tgcatcaggt gggtaactcc tcccacacaa    780
   atagtctgcc taccctcagg aatatttttt gtctgtggta cctcagccta tcgttgtttg    840
   aatggctctt cagaatctat gtgcttcctc tcattcttag tgccccctat gaccatctac    900
   actgaacaag atttatacag ttatgtcata tctaagcccc gcaacaaaag agtacccatt    960
   cttccttttg ttataggagc aggagtgcta ggtgcactag gtactggcat tggcggtatc   1020
   acaacctcta ctcagttcta ctacaaacta tctcaagaac taaatgggga catggaacgg   1080
   gtcgccgact ccctggtcac cttgcaagat caacttaact ccctagcagc agtagtcctt   1140
   caaaatcgaa gagctttaga cttgctaacc gctgaaagag ggggaacctg tttattttta    1200
   ggggaagaat gctgttatta tgttaatcaa tccggaatcg tcactgagaa agttaaagaa   1260
   attcgagatc gaatacaacg tagagcagag gagcttcgaa acactggacc ctggggcctc   1320
   ctcagccaat ggatgccctg gattctcccc ttcttaggac ctctagcagc tataatattg   1380
   ctactcctct ttggaccctg tatctttaac ctccttgtta actttgtctc ttccagaatc   1440
   gaagctgtaa aactacaaat ggagcccaag atgcagtcca agactaagat ctaccgcaga   1500
   cccctggacc ggcctgctag cccacgatct gatgttaatg acatcaaagg cacccctcct   1560
   gaggaaatct cagctgcaca acctctacta cgccccaatt cagcaggaag cagttag      1617
<210> 26
   <211> 538
   <212> PRT
   <213> Homo sapiens
<400> 26
   Met Ala Leu Pro Tyr His Ile Phe Leu Phe Thr Val Leu Leu Pro Ser
   1               5                   10                  15
   Phe Thr Leu Thr Ala Pro Pro Pro Cys Arg Cys Met Thr Ser Ser Ser
               20                  25                  30
   Pro Tyr Gln Glu Phe Leu Trp Arg Met Gln Arg Pro Gly Asn Ile Asp
           35                  40                  45
   Ala Pro Ser Tyr Arg Ser Leu Ser Lys Gly Thr Pro Thr Phe Thr Ala
       50                  55                  60
   His Thr His Met Pro Arg Asn Cys Tyr His Ser Ala Thr Leu Cys Met
   65                  70                  75                  80
   His Ala Asn Thr His Tyr Trp Thr Gly Lys Met Ile Asn Pro Ser Cys
                   85                  90                  95
   Pro Gly Gly Leu Gly Val Thr Val Cys Trp Thr Tyr Phe Thr Gln Thr
               100                 105                 110
   Gly Met Ser Asp Gly Gly Gly Val Gln Asp Gln Ala Arg Glu Lys His
           115                 120                 125
   Val Lys Glu Val Ile Ser Gln Leu Thr Arg Val His Gly Thr Ser Ser
       130                 135                 140
   Pro Tyr Lys Gly Leu Asp Leu Ser Lys Leu His Glu Thr Leu Arg Thr
   145                 150                 155                 160
   His Thr Arg Leu Val Ser Leu Phe Asn Thr Thr Leu Thr Gly Leu His
                   165                 170                 175
   Glu Val Ser Ala Gln Asn Pro Thr Asn Cys Trp Ile Cys Leu Pro Leu
   180 185 190
   Asn Phe Arg Pro Tyr Val Ser Ile Pro Val Pro Glu Gln Trp Asn Asn
   195 200 205
   Phe Ser Thr Glu Ile Asn Thr Thr Ser Val Leu Val Gly Pro Leu Val
   210 215 220
   Ser Asn Leu Glu Ile Thr His Thr Ser Asn Leu Thr Cys Val Lys Phe
   225 230 235 240
   Ser Asn Thr Thr Tyr Thr Thr Asn Ser Gln Cys Ile Arg Trp Val Thr
   245 250 255
   Pro Pro Thr Gln Ile Val Cys Leu Pro Ser Gly Ile Phe Phe Val Cys
   260 265 270
   Gly Thr Ser Ala Tyr Arg Cys Leu Asn Gly Ser Ser Glu Ser Met Cys
   275 280 285
   Phe Leu Ser Phe Leu Val Pro Pro Met Thr Ile Tyr Thr Glu Gln Asp
   290 295 300
   Leu Tyr Ser Tyr Val Ile Ser Lys Pro Arg Asn Lys Arg Val Pro Ile
   305 310 315 320
   Leu Pro Phe Val Ile Gly Ala Gly Val Leu Gly Ala Leu Gly Thr Gly
   325 330 335
   Ile Gly Gly Ile Thr Thr Ser Thr Gln Phe Tyr Tyr Lys Leu Ser Gln
   340 345 350
   Glu Leu Asn Gly Asp Met Glu Arg Val Ala Asp Ser Leu Val Thr Leu
   355 360 365
   Gln Asp Gln Leu Asn Ser Leu Ala Ala Val Val Leu Gln Asn Arg Arg
   370 375 380
   Ala Leu Asp Leu Leu Thr Ala Glu Arg Gly Gly Thr Cys Leu Phe Leu
   385 390 395 400
   Gly Glu Glu Cys Cys Tyr Tyr Val Asn Gln Ser Gly Ile Val Thr Glu
   405 410 415
   Lys Val Lys Glu Ile Arg Asp Arg Ile Gln Arg Arg Ala Glu Glu Leu
   420 425 430
   Arg Asn Thr Gly Pro Trp Gly Leu Leu Ser Gln Trp Met Pro Trp Ile
           435                 440                 445
   Leu Pro Phe Leu Gly Pro Leu Ala Ala Ile Ile Leu Leu Leu Leu Phe
       450                 455                 460
   Gly Pro Cys Ile Phe Asn Leu Leu Val Asn Phe Val Ser Ser Arg Ile
   465                 470                 475                 480
   Glu Ala Val Lys Leu Gln Met Glu Pro Lys Met Gln Ser Lys Thr Lys
                   485                 490                 495
   Ile Tyr Arg Arg Pro Leu Asp Arg Pro Ala Ser Pro Arg Ser Asp Val
               500                 505                 510
   Asn Asp Ile Lys Gly Thr Pro Pro Glu Glu Ile Ser Ala Ala Gln Pro
           515                 520                 525
   Leu Leu Arg Pro Asn Ser Ala Gly Ser Ser
       530                 535
<210> 27
   <211> 18
   <212> DNA
   <213> Artificial
<220>
   <223> amorce d'amplification
<400> 27
   tgcagatgct gtgtctgg
<210> 28
   <211> 17
   <212> DNA
   <213> Artificial
<220>
   <223> amorce d'amplification
<400> 28
   cgtactggcc caggacc
<210> 29
   <211> 20
   <212> DNA
   <213> Artificial
<220>
   <223> amorce d'amplification
<400> 29
   ggttcgtgct aattgagctg
<210> 30
   <211> 20
   <212> DNA
   <213> Artificial
<220>
   <223> amorce d'amplification
<400> 30
   atggtggcaa gcttcttgtt 20
<210> 31
   <211> 20
   <212> DNA
   <213> Artificial
<220>
   <223> amorce d'amplification
<400> 31
   tgagctttcc ctcactgtcc 20
<210> 32
   <211> 20
   <212> DNA
   <213> Artificial
<220>
   <223> amorce d'amplification
<400> 32
   tgttcggctt gattaggatg 20
<210> 33
   <211> 20
   <212> DNA
   <213> Artificial
<220>
   <223> amorce d'amplification
<400> 33
   catggcccaa tattccattc 20
<210> 34
   <211> 21
   <212> DNA
   <213> Artificial
<220>
   <223> amorce d'amplification
<400> 34
   ggtccttgtt cacagaactc c 21
<210> 35
   <211> 20
   <212> DNA
   <213> Artificial
<220>
   <223> amorce d'amplification
<400> 35
   ccgctcctga ttggactaaa 20
<210> 36
   <211> 20
   <212> DNA
   <213> Artificial
<220>
   <223> amorce d'amplificaton
<400> 36
   cgtgggtcaa ggaagagaac 20
<210> 37
   <211> 21
   <212> DNA
   <213> Artificial
<220>
   <223> amorce d'amplification
<400> 37
   atgacccgca gcttctaaca g 21
<210> 38
   <211> 20
   <212> DNA
   <213> Artificial
<220>
   <223> amorce d'amplification
<400> 38
   ctccgctcac agagctccta 20
<210> 39
   <211> 21
   <212> DNA
   <213> Artificial
<220>
   <223> amorce d'amplification
<400> 39
   ccaacatcac taacacaacc t 21
<210> 40
   <211> 20
   <212> DNA
   <213> Artificial
<220>
   <223> amorce d'amplification
<400> 40
   gggagttagt aaggggtttg 20
<210> 41
   <211> 24
   <212> DNA
   <213> Artificial
<220>
   <223> amorce d'amplification
<400> 41
   caacctatta aacaaaacta aatt 24
<210> 42
   <211> 27
   <212> DNA
   <213> Artificial
<220>
   <223> amorce d'amplification
<400> 42
   agatttaata gagtgaaaat agagttt 27
<210> 43
   <211> 22
   <212> DNA
   <213> Artificial
<220>
   <223> amorce d'amplification
<400> 43
   ttattagttt aggggatagt tg 22
<210> 44
   <211> 23
   <212> DNA
   <213> Artificial
<220>
   <223> amorce d'amplification
<400> 44
   acacaataaa caacctacta aat 23
<210> 45
   <211> 19
   <212> DNA
   <213> Artificial
<220>
   <223> amorce d'amplification
<400> 45
   gagggtaagt ggtgataaa 19
<210> 46
   <211> 21
   <212> DNA
   <213> Artificial
<220>
   <223> amorce d'amplification
<400> 46
   aacctactaa atccaaaaaa a 21
<210> 47
   <211> 22
   <212> DNA
   <213> Artificial
<220>
   <223> amorce d'amplication
<400> 47
   taggatttta ggtttattgt ta 22
<210> 48
   <211> 19
   <212> DNA
   <213> Artificial
<220>
   <223> amorce d'amplification
<400> 48
   aaaaataaaa tattaaacc 19
<210> 49
   <211> 20
   <212> DNA
   <213> Artificial
<220>
   <223> amorce d'amplification
<400> 49
   atatgtggga gtgagagata 20
<210> 50
   <211> 22
   <212> DNA
   <213> Artificial
<220>
   <223> amorce d'amplification
<400> 50
   caacaacaaa caataataat aa 22
<210> 51
   <211> 22
   <212> DNA
   <213> Artificial
<220>
   <223> amorce d'amplification
<400> 51
   ttgagttttt ttattgatag tg 22
<210> 52
   <211> 21
   <212> DNA
   <213> Artificial
<220>
   <223> amorce d'amplification
<400> 52
   tctaaatcct attttcctac t 21
<210> 53
   <211> 24
   <212> DNA
   <213> Artificial
<220>
   <223> amorce d'amplification
<400> 53
   gtttttttat tgatagtgag agat 24
<210> 54
   <211> 20
   <212> DNA
   <213> Artificial
<220>
   <223> amorce d'amplification
<400> 54
   taacaaacct ttaatccaat 20
<210> 55
   <211> 22
   <212> DNA
   <213> Artificial
<220>
   <223> amorce d'amplification
<400> 55
   tttagtgagg atgatgtaat at 22
<210> 56
   <211> 22
   <212> DNA
   <213> Artificial
<220>
   <223> amorce d'amplification
<400> 56
   caacttaata aaaataaacc ca 22
<210> 57
   <211> 24
   <212> DNA
   <213> Artificial
<220>
   <223> amorce d'amplification
<400> 57
   ataatgtttt agtaagtgtt ggat 24
<210> 58
   <211> 20
   <212> DNA
   <213> Artificial
<220>
   <223> amorce d'amplification
<400> 58
   acaattacaa acctttaacc 20
<210> 59
   <211> 20
   <212> DNA
   <213> Artificial
<220>
   <223> amorce d'amplification
<400> 59
   aattcattca acatccattc 20
<210> 60
   <211> 26
   <212> DNA
   <213> Artificial
<220>
   <223> amorce d'amplification
<400> 60
   ggtttaatat tatttattat tttgga 26
<210> 61
   <211> 25
   <212> DNA
   <213> Artificial
<220>
   <223> amorce d'amplification
<400> 61
   ctcttacctt cctatactct ctaaa 25
<210> 62
   <211> 28
   <212> DNA
   <213> Artificial
<220>
   <223> amorce d'amplification
<400> 62
   agagtgtagt tgtaagattt aatagagt 28